(19) Europäisches Patentamt / European Patent Office / Office européen des brevets

(11) **EP 4 221 828 B1**

(12) **EUROPEAN PATENT SPECIFICATION**

(45) Date of publication and mention
of the grant of the patent:
**31.12.2025  Bulletin 2026/01**

(21) Application number: **21793860.4**

(22) Date of filing: **01.10.2021**

(51) International Patent Classification (IPC):
***A61N 5/06*** *(2006.01)*

(52) Cooperative Patent Classification (CPC):
**A61N 5/0616; A61N 5/0613;** A61B 5/01;
A61B 5/4866; A61N 2005/0626

(86) International application number:
**PCT/EP2021/077205**

(87) International publication number:
**WO 2022/069755 (07.04.2022 Gazette 2022/14)**

(54) **DEVICE FOR APPLYING PHOTOBIOMODULATION**

VORRICHTUNG ZUR ANWENDUNG VON PHOTOBIOMODULATION

DISPOSITIF  D'APPLICATION DE PHOTOBIOMODULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.10.2020  EP 20199710
15.04.2021  PCT/EP2021/059842**

(43) Date of publication of application:
**09.08.2023  Bulletin 2023/32**

(73) Proprietor: **G Life
01710 Thoiry (FR)**

(72) Inventors:
 • **GERELLI, Emmanuel
   1046 Rueyres (CH)**
 • **JONIOVA, Jaroslava
   1007 Lausanne (CH)**
 • **GERELLI, Sébastien
   01710 Thoiry (FR)**
 • **WAGNIÈRES, Georges
   1095 Lutry (CH)**
 • **BONNEAU, Michel
   24190 Saint Jean d'Ataux (FR)**

(74) Representative: **Perreaud, Jérémie
André Roland SA
IP Attorneys & Services
Avenue Collonges 21
1004 Lausanne (CH)**

(56) References cited:
**WO-A1-2016/040534      WO-A2-2008/016894
US-A1- 2007 219 604      US-A1- 2011 034 971
US-A1- 2011 251 659**

• **LEE GEON-HUI ET AL: "Multifunctional materials
for implantable and wearable photonic
healthcare devices", NATURE REVIEWS
MATERIALS, NATURE PUBLISHING GROUP UK,
LONDON, vol. 5, no. 2, 7 January 2020
(2020-01-07), pages 149 - 165, XP037012936, DOI:
10.1038/S41578-019-0167-3**
• **ADDISON P S ET AL: "Time–frequency analysis
of biosignals", IEEE ENGINEERING IN MEDICINE
AND BIOLOGY MAGAZINE, IEEE SERVICE
CENTER, PISACATAWAY, NJ, US, vol. 28, no. 5, 1
September 2009 (2009-09-01), pages 14 - 29,
XP011295822, ISSN: 0739-5175**

**Description**

**Field of invention**

The present disclosure generally relates to photobiomodulation (PBM) and more precisely to devices and methods for applying photobiomodulation therapy (PBMT).

**State of the art**

<u>Definitions</u>

The following definitions apply to the present document.

- Light: Electromagnetic radiations with wavelengths ranging between 250 nm and 3 $\mu$m.
- Irradiance or primary incidence **E** [W/m$^2$]: The irradiance describes the power per unit surface directly received from a source.
- Radiance **L** [W/(m$^2$.sr)]: The radiance is the power of light that passes through or is emitted from a unit surface area and propagates within a unit solid angle in a specified direction.
- Fluence rate **F** [W/m$^2$]: The fluence rate is the power entering a sphere presenting a unit cross-section. It takes into account diffusion and/or scattering effects in the target environment. The fluence rate is measured with an isotropic power meter. It takes into account the direct flux (the irradiance) as well as the scattering and diffusion contributions. Like the Fluence (see below) this term is of fundamental importance in dosimetry where multiple scattering and diffusion in the target tissue are of great importance.
- Fluence or light dose $\Psi$ [J/m$^2$]: Is the time integral of the fluence rate. Therefore, the fluence is the energy entering a sphere presenting a unit cross-section.
- Absorption coefficient $\mu_a$ [m$^{-1}$]: Inverse of the mean free path before photon absorption
- Scattering coefficient $\mu_s$ [m$^{-1}$]: Inverse of the mean free path between photon scattering
- Reduced scattering coefficient $\mu_s'$ [m$^{-1}$]: $\mu_s' = \mu_s(1-g)$
- Anisotropy factor **g** [--]: g is equal to the mean value of cos "theta", where "theta" is the deflection angle of a photon scattered by a particle.
- Effective attenuation coefficient $\mu_{eff}$ [m$^{-1}$]: $\mu_{eff} = (3\,\mu_a(\mu_a+\mu_s'))^{1/2}$

Photobiomodulation, named PBM in the present document, refers to the treatment of biological objects, such as a tissue or an organ, with certain wavelength(s) of light. This treatment may facilitate tissue or nerve regeneration and remodeling, resolve inflammation, reduce edema, relieve pain, modulate the immune system and the metabolism. It positively acts on age related macular degeneration, blood treatment, wound healing, immunomodulation, and possibly even viral and bacterial infections.

Many conditions are associated with perturbations of the metabolism, including deficiencies of the mitochondrial respiration. These conditions include neurodegenerative diseases (Parkinson's, Alzheimer's and Huntington's diseases), atherosclerosis, certain forms of diabetes, autoimmune diseases, cancer, chronic wounds, damages resulting from ischemia-reperfusions and chronic or acute inflammation like the acute respiratory distress syndrome (ARDS). It is also well known that the metabolism is significantly altered in the cases of stroke, heart attack, grafts or ischemic wounds, among other. As an example, it has been shown that the mitochondrial respiration plays an important role in the heart remodeling [Kindo, 2016], and that the cardiac metabolism reacts to a parietal stress by a mitochondrial dysfunction [Kindo, 2012].

Therefore, strategies to normalize, restore and/or increase the metabolism are of high interest to treat and characterize numerous conditions. PBM therapy is one of these strategies [Hamblin 2017; Hamblin 2018].

PBM therapy is based on the administration of light at low (sub-thermal) irradiance, mostly at wavelengths ranging between 600 and 900 nm, a spectral window corresponding to the maximal light penetration depth in most soft tissues. PBM has a broad range of molecular, cellular, and tissular effects [Hamblin 2017; Hamblin 2018].

However, its mechanisms are not yet fully understood. Moreover, PBM treatment parameters are very rarely optimized and/or mastered. Based on the studies conducted by several groups [Hamblin 2017; Hamblin 2018] and, most importantly, in vitro and in vivo observations carried out by the inventors, one can conclude that PBM generates several positive effects, in particular:

a) An increase of the tissue oxygen ($O_2$) consumption following or during hypoxia,
b) A stimulation of angiogenesis,
c) A stimulation of regeneration processes at the cellular level,

d) An increase, following an application of 5-aminolevulinic acid (ALA), of the endogenous production of protoporphyrin IX (PpIX) [Sachar,2016], which can be used as an $O_2$ sensor. It should be noted that several formulations of ALA to induced PpIX as photosensitizer and fluorescing markers are approved for cancer therapy and detection, respectively.

e) An increase of the ATP production, indicating an improved metabolic activity,

f) A modulation of reactive oxygen species (ROS)

g) A modulation of reactive nitrogen species (RNS)

h) A rescuing of cells subject to an intoxication.

i) An increase of the survival rate of embryos subject to anoxia/reoxygenation events.

j) An increase of circulating nitric oxide (NO) during long hypoxia or hypoxemia event.

k) A sustained homeostasis (based on hemodynamics variables, blood gas measurement as glycemia) during long hypoxia or hypoxemia event.

These observations probably result from a stimulation of the metabolic activities and are of high interest for numerous medical applications, including those mentioned above [Hamblin 2017; Hamblin 2018].

PBM is, in particular, of interest for the treatment of myocardial infarction (MI) [Liebert, 2017], which is one the most common acute pathologies. It represents a major cause of death worldwide. At present, the treatments of choice for patients suffering from MI to limit its size and reduce acute myocardial ischemic injury are time consuming, have side effects and limited efficacies. They consist of either primary percutaneous coronary intervention or thrombolytic therapy. Moreover, the treatment itself (process of reperfusion) can be the cause of death of cardiomyocytes until days after the treatment, a process also known as myocardial reperfusion injury, for which, up to this date, there is still no effective treatment [Chouchani, 2016; Ferrari, 2017; Kalogeris, 2017]. PBMT is also of interest for the treatment of systemic inflammation as it is the case for fibromyalgia, rheumatology-related arthritis or auto immune disease, in particular when the circulating blood is directly illuminated. PBMT can also help to avoid consequences of SARS-Cov2 in acute phase where a strong immune response through a cytokinic storm induces acute respiratory distress syndrome (ARDS) or during chronic phases resulting from long SARS-Cov2 effect.

US 2007/219604 A1 discloses a method for applying PBM on a biological object wherein light is delivered with an adequate temporal evolution of the optical power, the power being determined on the basis of the biological object optical coefficients and the light delivery geometry on/in the biological object. In this patent application US 2007/219604 A1, the PBM effects are induced by the generation of a fluence rate, successively in each parts of the volume of the biological object. It should be noted that specific values of the fluence rates and illumination times are not mentioned in this application.

Existing methods for applying PBM are however not efficient enough, in particular because of the bimodal effects of PBM, as explained below.

The limited use of PBMT can also be explained by the absence of methods to monitor the metabolic activity of biological tissues. This statement is supported by another discovery of the inventors demonstrating that the importance of the PBM effects depends on the time at which light is applied relative the metabolic activity, as determined, for example, by the oxygen consumption. There is therefore a need to improve the use of PBM for the treatment of biological objects.

## List of figures

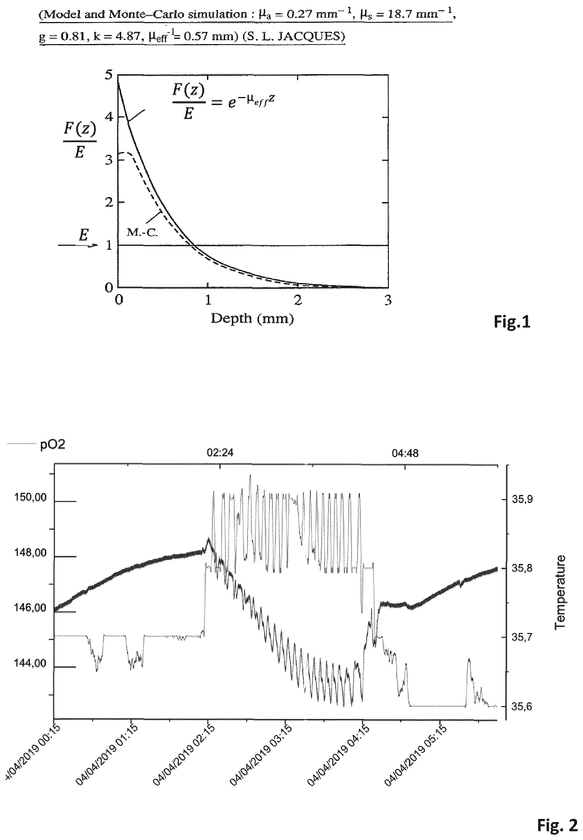

**Figure 1:** Evolution of the fluence rate/irradiance ratio versus the depth in a semi-infinite tissue for a "broad", collimated and perpendicular illumination of the air-tissue interface. The continuous curve is the solution of the diffusion approximation, whereas the dashed curve is the solution of a Monte-Carlo computer-based simulation, where $\mu_a$ and $\mu_s$ are the absorption and scattering coefficients, respectively. $\mu_{eff}$ is the effective attenuation coefficient, g is the anisotropy factor, whereas k is the pre-exponential factor resulting from the backscattering of light. Derived from [Jacques, 2010].

**Figure 2:** Temporal evolution of the $pO_2$ (black curve; mmHg) and temperature (grey curve; °C) measured above a monolayer of HCM cells subject to metabolic oscillations.

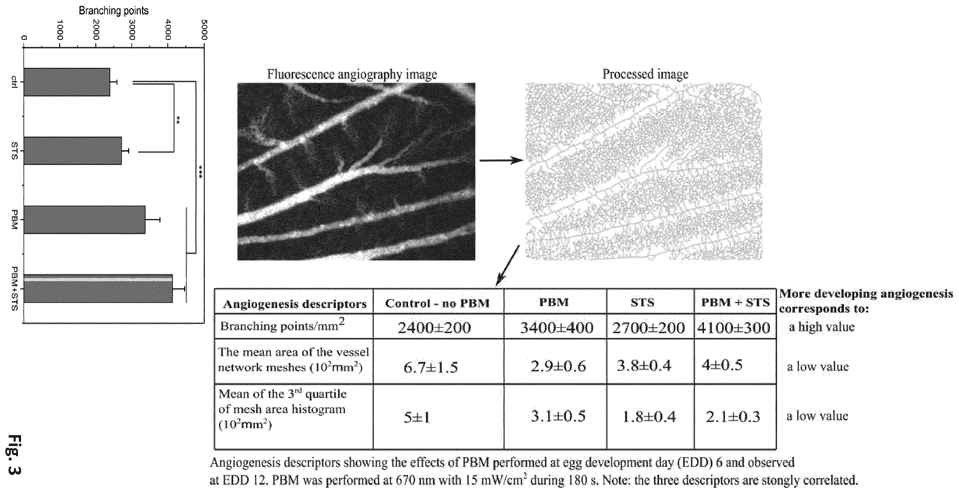

**Figure 3:** Synergic effect of STS and PBM on angiogenesis.

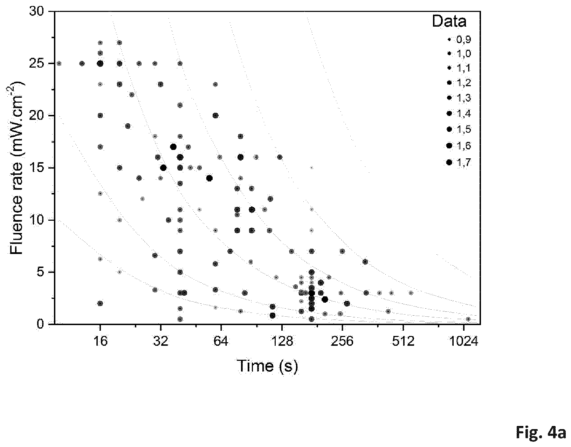

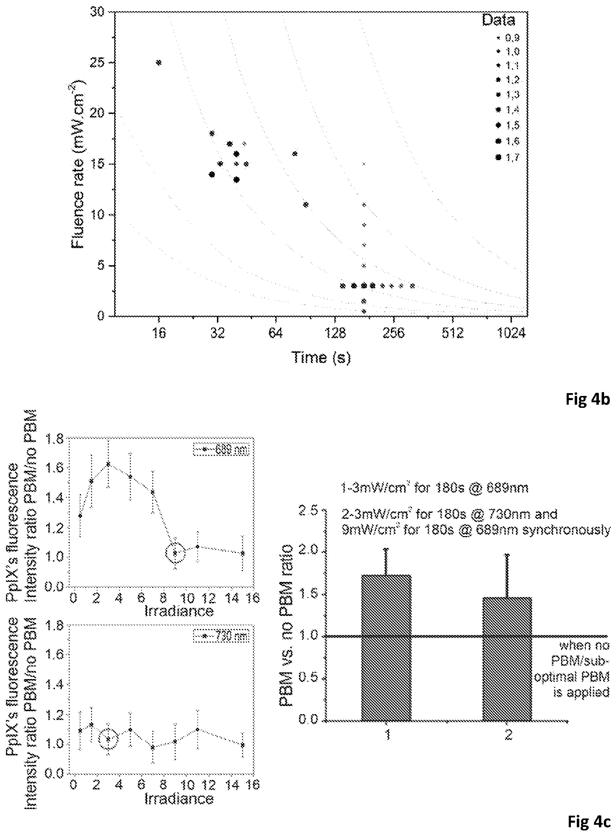

**Figures 4a, 4b,:** Various PBM conditions presented as a ratios of the PpIX fluorescence intensity of PBM/no PBM reflecting in particular the metabolic activity, observed in human cardiomyocytes (HCM) at 689nm (Figure 4a) and 652nm (Figure 4b). The values of the ratio " PBM / no PBM" are given by the monochrome bar.

**Figure 4c** : Left The fluence rate dependence effect (fluence rate ranging from 0.5 - 15 mW/cm$^2$) at 689nm ( a potent wavelength) and 730nm (a non-potent wavelength) . Right : The combination of the potent wavelength using a not effective fluence rate (9mW/cm$^2$) with a nonpotent wavelength inducing a significant effect in the relative increase of the PpIX fluorescence.

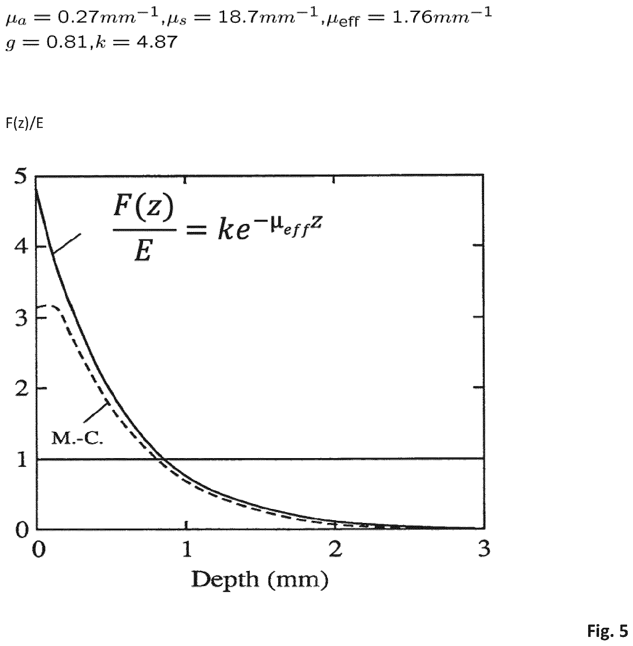

**Figure 5:** Evolution of the fluence rate/irradiance ratio (F/E) versus the depth in a semi-infinite tissue for a "broad", collimated and perpendicular illumination of the air-tissue interface. The continuous curve is the solution of the diffusion approximation, whereas the dashed curve is the solution of a Monte-Carlo computer-based simulation, where $\mu_a$ and $\mu_s$ are the absorption and scattering coefficients, respectively. $\mu_{eff}$ is the effective attenuation coefficient, g is the anisotropy factor, whereas k, which depends on the refractive index matching conditions ($n_{tissue}/n_{air}$ = 1.37) as well as the optical coefficients, is the pre-exponential factor resulting from the backscattering of light. Derived from [Jacques, 2010].

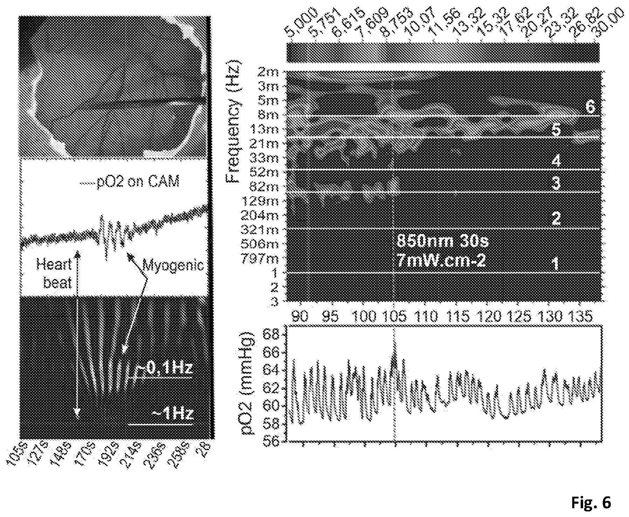

**Figure 6:** Frequency analysis of the $pO_2$ in the CAM at EDD 7. Left up: Image of the experiment showing the metallic Clark's probe applied against a blood vessel. Left middle: $pO_2$ signal (acquired at 50 Hz). Left down: the associated spectrum based on a wavelet analysis (the vertical scale is the frequency, and the monochrome level represents the oscillation amplitude, the horizontal scale is the time in seconds). Right bottom: $pO_2$ signal (acquired at 1 Hz) and right up: the monochrome level represents the oscillation amplitude) associated spectrum resulting from the wavelets analysis. The horizontal axis is the time given in minutes. A strong activation of the $pO_2$ tone is observed 25 minutes (time out of the scale) after a topical application of NaHS (10 $\mu$l - 1$\mu$M), which is deactivated by PBM (850 nm, 7 mW.cm$^2$, 30 s) at time 105 min (see myogenic signal). The horizontal lines reported on the spectrum indicate specific frequencies. 1 - Cardiac, 2 - Respiratory, 3 - Myogenic, 4 - Neurogenic, 5 - eNOS dept, 6 - eNOS indept (probably prostaglandin [Shiogai and al.]). Cardiac frequency cannot be resolved within the sampling. Respiratory and neurogenic bands do not exist at this EDD.

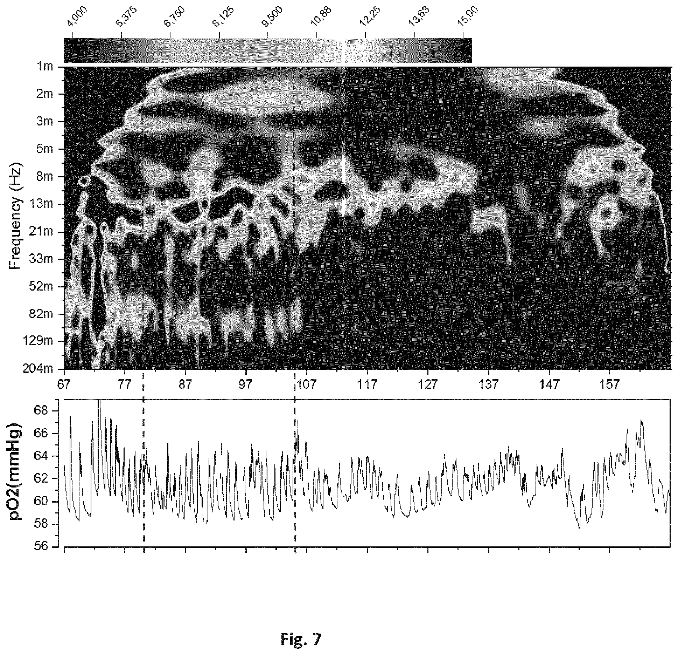

**Figure 7:** Enlargement of the experiment presented in figure 6. The horizontal axis gives the time in minutes.

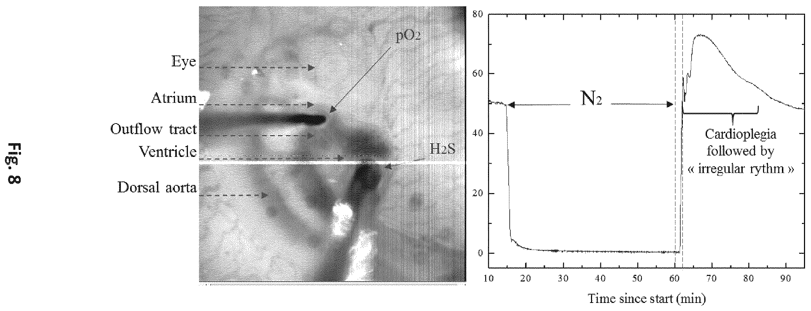

**Figure 8:** Left: Image of the *in ovo* anoxia reoxygenation experiment. Right: $pO_2$ signal (OX-100 $\mu$m Unisense®) recorded during the whole duration of the experiment (Vertical axis: mmHg). The hypoxia due to the flushing of $N_2$ (up to 60 minutes) is followed by a reoxygenation.

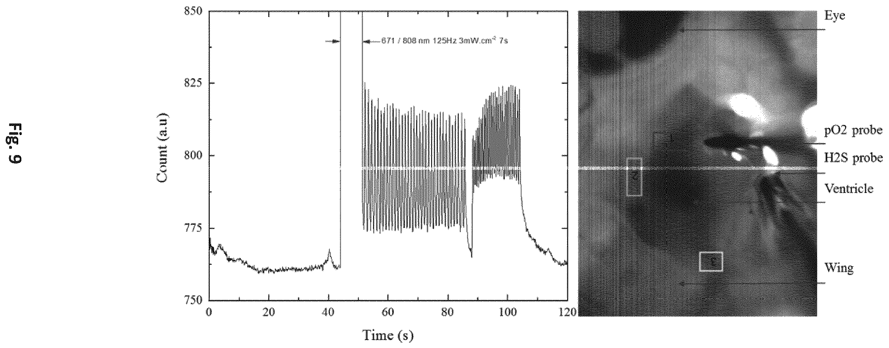

**Figure 9:** Stimulation of a chicken embryo's heart at EDD 5 by PBM during an anoxic cardioplegia. Left: Time course measurement of the heartbeat (assessed by the change of the heart reflectivity in the region of interest defined by the rectangle presented on the image showing the embryo (right). The heartbeat is stopped between 0 and 45 s. Then, a PBM illumination during 7 seconds re-activated the heart beat for more than 1 min.

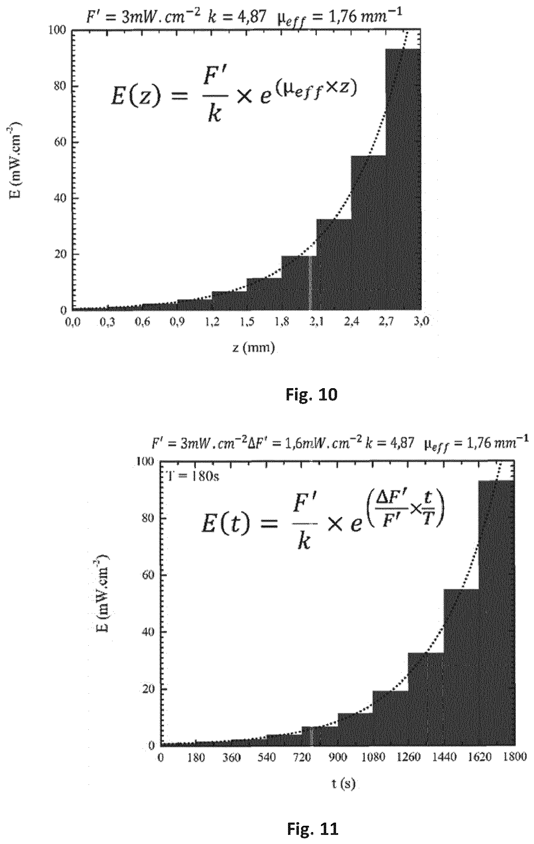

**Figure 10:** Spatial evolution of the fluence rate (E) in a semi-infinite tissue for a "broad", collimated and perpendicular illumination of the air-tissue interface. $\Delta z$=0,3 mm; n=10; $n_{tissue}/n_{air}$ = 1.37; T=180 s; $\Delta F'$= 1.6 mW/cm$^2$; The dotted curve corresponds to the continuous fit of the "step-based" evolution of the fluence rate. The analytical expression of this fit is presented as an insert in this figure. This illustrates that E may be changed continuously instead of incrementally.

**Figure 11:** Temporal evolution of the fluence rate (E) in a semi-infinite tissue for a "broad", collimated and perpendicular illumination of the air-tissue interface. $\Delta z$=0,3 mm; n=10; $n_{tissue}/n_{air}$ = 1.37; T=180 s; $\Delta F'$= 1.6 mW/cm$^2$; The dotted curve corresponds to the continuous fit of the "step-based" evolution of the fluence rate. The analytical expression of this fit is presented as an insert in this figure. This illustrates that E may be changed continuously instead of incrementally.

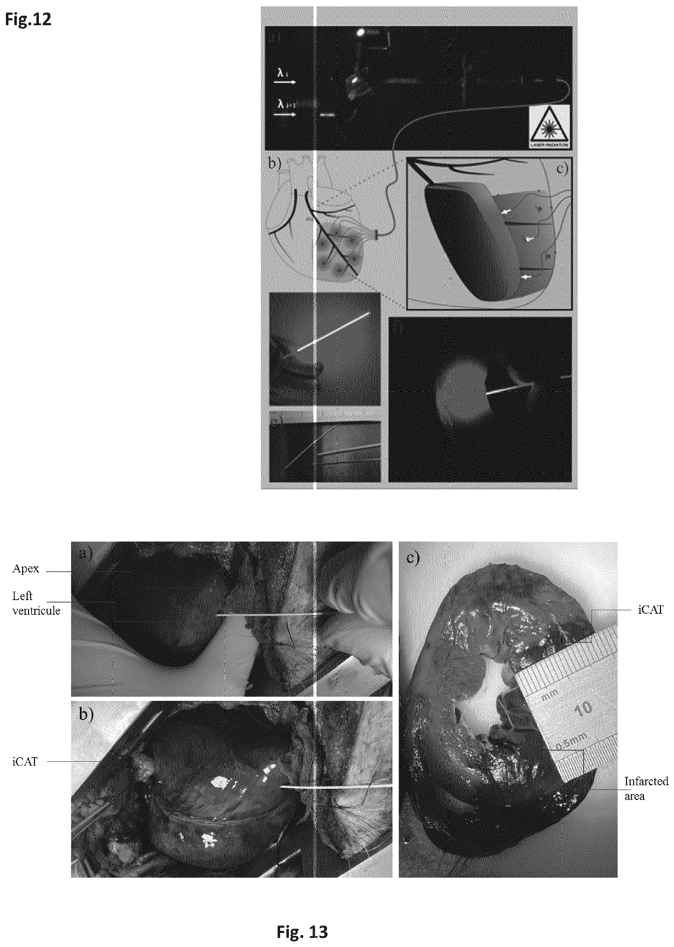

**Figure 12:** Overview of a trans-myocardial implantation at 90° during coronary thrombosis (b) of cylindrical distributors (d) connected to an optical source (a) that are placed trans-myocardially (c) with a predefined pattern and spacing through a mask. (e) the light distributor, the iCAT® catheter and the guide to be inserted into the catheter for transfixion. (f) image illustrating the propagation of light from an isotropic distributor (sphere of 0.8 mm diameter) implanted in the center of an *ex vivo* swine left ventricular myocardium (652 nm - 100mW).

**Figure 13:** Implantation of an iCAT® catheter all along the damage left ventricle of the swine heart in situ after a sternotomy. A) Catheter was introducing from the apex side until it come out of the top of left ventricle B). C) Heart cross section after the heart excision without removing the catheter. The catheter, here is well placed close to the middle of the myocardium thickness.

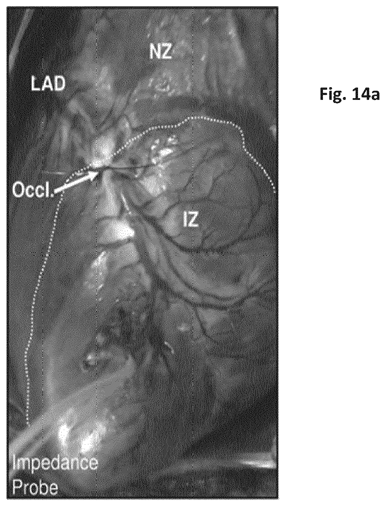

**Figure 14a:** Visual localization of the ischemic area (IZ) after the occlusion of the descending left anterior coronary artery (LAD) partially perfusing the left ventricle during an open-heart surgery on pig. The IZ is easily differentiated from non-ischemic area (NZ). An electrical impedance sensor can also be used to characterize the ischemic area. AJP- Heart Circ Physiol

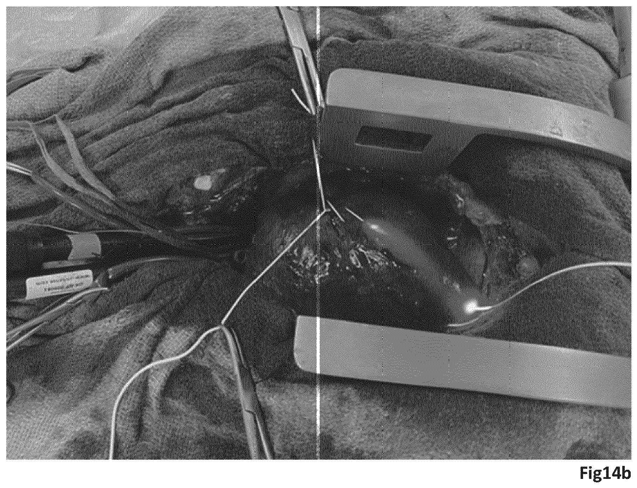

**Figure 14b:** Visual representation of the transmyocardial photoconditioning described in example 1, after the occlusion of the LAD during an open-heart surgery on pig. During the ischemic phase, interstitial catheters (iCAT® - 0.89mm) were inserted into the left myocardium from the apex to left atrium with an optimal distance between them in order to maximize the treatment area. Cylindrical distributors (RD250® - stick length 7cm) were then placed in each iCAT®. PBM treatment was launched few seconds to few minutes depending on the illumination time before the reperfusion. A 670nm and 808nm illumination were used from two distributors few seconds before the reperfusion of the ventricle. Catheters were then removed just after the reperfusion. Eventually, it is possible for them to be to let in place after the surgery for further regenerative illuminations.

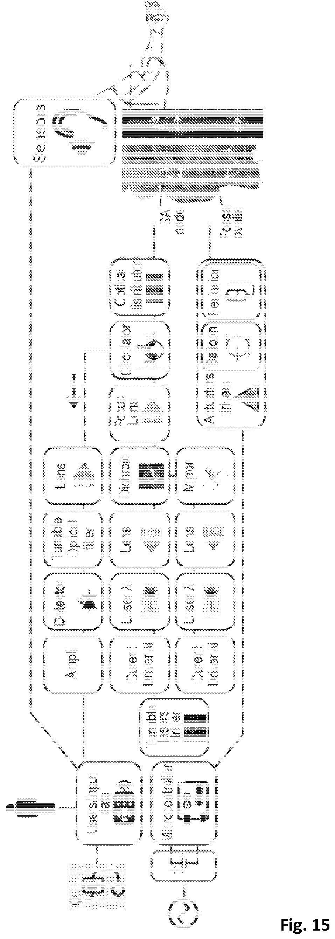

**Figure 15:** Simplified diagram illustrating an example of a part of the device supplying a treatment. Depending on the disease and the treatment method, interstitial or systemic for instance, the device can be modular with combination of different elementary communicated blocks. Some blocks can be a sensor based on observables described in the present document, or an interface to acquire data from usual clinical system. Some blocks can characterize the spectroscopy of the illumination whereas others can be dedicated to control exogenous agent perfusion and perfusion temperature into a catheter. Others actuators can also be added to combined exogenous stimulus like mechanical pressure or temperature change with PBM. In case of the use of multi-lumens balloon catheter, as shown here, some blocks can control the time and/or the period and/ or the level of inflation /deflation with or without taking into account the monitoring of the change. Part of the device also integrates specific balloon size and shape to optimally treat the biological objects. For instance, as represented in the figure, a balloon centered into the right atrium which present two opposites conic shapes can optimally treat circulating objects coming from the inferior and the superior part of the vena cava. Moreover, since PBM strongly influences the biological rhythms, particularly during the hypoxia or hypoxemia, balloon can be shaped in order to be in contact to the sino-atrial node located on the top of the right atrium or to be in contact of the atrial - ventricle node. Moreover, using a multi-lumens catheter, exogenous agents can be injected through the balloon upstream or downstream the illumination function of the agent. Obviously the device can also be used to photo-activate the photo-sensitive agent. Blocks of these device can also be implemented directly through various implants to minimize platelet aggregate and coagulation on heart valve. It can also be implemented on artificial heart or pancreatic chambers for instance, to increase the biocompatibility / biostability, and/or can be implemented to reduce inflammation and immune response of implant chamber used in chemotherapy as well as activating endothelialization of hip prosthesis or vascular stent as other examples.

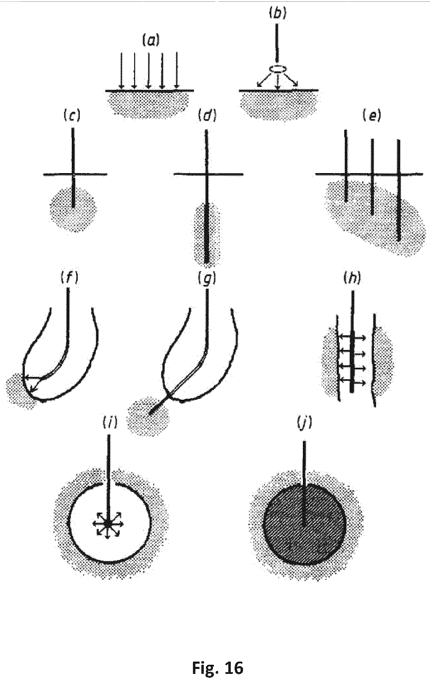

**Figure 16:** Examples of the irradiation geometries used in PBM. The stippled areas represent schematically the pattern of fluence rate in tissue. (a), (b) Surface irradiation from broad beam or lens-tipped fiber. (c)-(e) Interstitial irradiation with cut-end or cylindrical fibers. (f)-(h) Intracavitary and intralumenal irradiation. (i), (j) Intracavitary whole-surface irradiation using an isotropically-tipped fiber or a light-diffusing liquid (shaded). Source: Wilson, 1986.

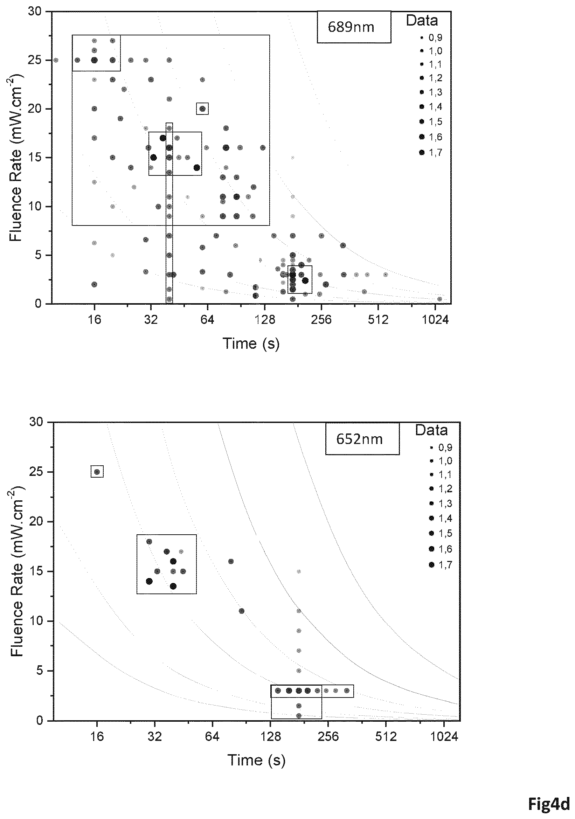

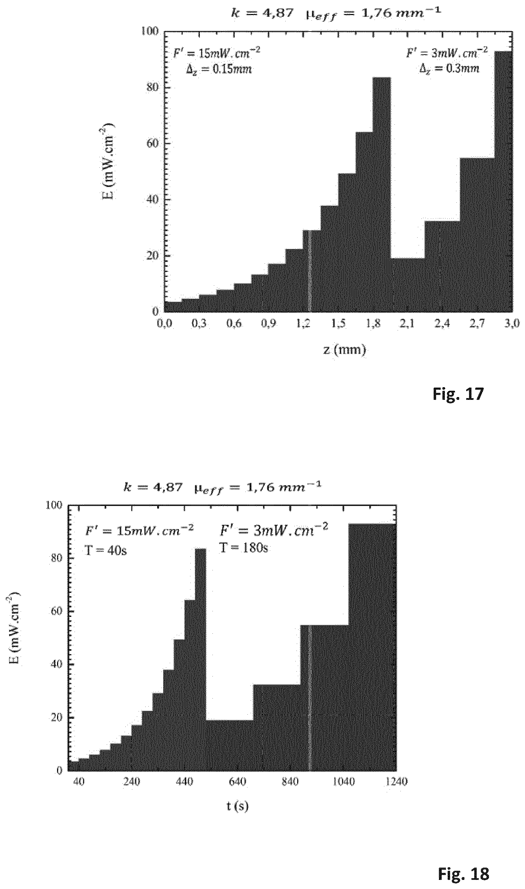

**Figure 17:** Spatial evolution of the fluence rate (E) in a semi-infinite tissue for a "broad", collimated and perpendicular illumination of the air-tissue interface. The spatial evolution of E is optimized in such a way that its value never exceeds 100 mW/cm$^2$, while minimizing the total illumination time. The idea is to illuminate the sample exploiting two PBM hot spots visible in figure 4, i.e. generating fluence rates of 15 and 3 mW/cm$^2$ during 40 and 180 s (values of T), respectively. The corresponding values for $\Delta$F', $\Delta$z and n (the number of steps) are respectively:

- 4 mW/cm$^2$, 0,15 mm and 13 for T = 40 s
- 1.6 mW/cm$^2$, 0.3 mm and 4 for T = 180 s.

$n_{tissue}/n_{air}$ = 1.37.

**Figure 18:** Temporal evolution of the fluence rate (E) in a semi-infinite tissue for a "broad", collimated and perpendicular illumination of the air-tissue interface. The spatial evolution of E is optimized in such a way that its value never exceeds 100 mW/cm$^2$, while minimizing the total illumination time. The idea is to illuminate the sample exploiting two PBM hot spots visible in figure 4, i.e. generating fluence rates of 15 and 3 mW/cm$^2$ during 40 and 180 s (values of T), respectively. The corresponding values for $\Delta$F', $\Delta$z and n (the number of steps) are respectively:

- 4 mW/cm$^2$, 0,15 mm and 13 for T = 40 s
- 1.6 mW/cm$^2$, 0.3 mm and 4 for T = 180 s.

$n_{tissue}/n_{air}$ = 1.37.

The "step-based" evolution of the fluence rate can be fitted by the analytical expression presented as insert in figure 11.

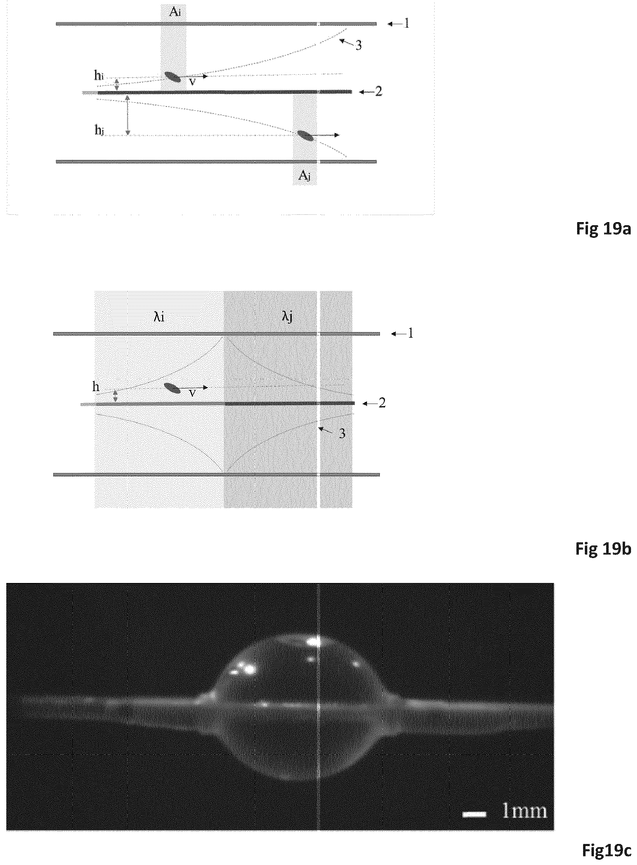

**Figure 19a:** A non-uniform light emittance illustrated with a non-uniform longitudinal emittance from a cylindrical distributor. The distributor (2) is placed into a vessel delimited by the wall vessel (1). The emittance of the distributor is shaped in order to create a light gradient all along the stick (part of the distributor which emits the light), represented here by iso-curves of the fluence rate which are not parallel with the stick. Considering the two identical circulating objects passing all along the distributor at a certain distance $h_i$ and $h_j$ within a certain speed $\vec{v}$ will find at particular place, $A_i(h_i)$ and $A_j(h_j)$ respectively, the optimal fluence rate in respect to the illumination time defining by $\vec{v}$ in order to address a particular hot spot $\Omega_{i,\lambda}$. Obviously, the light gradient is adapted on the basis of optical coefficient of the circulating medium, the geometry of the vessel as well as the level of speed and the nature of the flow (laminar, turbulent, pulsatile).

**Figure 19b:** Wavelength combination of non-uniform emittance illustrated with a sequential non-uniform longitudinal emittance from a cylindrical distributor. In certain scenario where particular hot spots cannot be selected, due to for instance, a mismatch between the constraint illumination time and accessible fluence rate, illumination combination of a potent ($\lambda_i$) and a non-potent ($\lambda_j$) wavelengths can be used to overpass the issue. In the scheme, where any hot spot for any kind of circulating can be selected, the iso-dose can be in parallel for both wavelengths, then, a sequential or simultaneous uniform illumination can be used to obtain a potent PBM effect. In contrary, if hot spots for certain kind of circulating object can be selected, a non-uniform illumination must be considered to optimally treat all kind of circulating object.

**Figure19c:** Modulation of the emittance of a uniform longitudinal distributor within a balloon. Encapsulation of a RD® (stick of 2cm delimited by the radiomarkers) into a balloon based catheter. The shape and the size of the balloon is defined by the shape of the emittance of the RD. In this case, circulating object passing in the vicinity of the balloon will be exposed to different fluence rate since the distance within the distributor is modulated by the modulation of the diameter of the balloon.

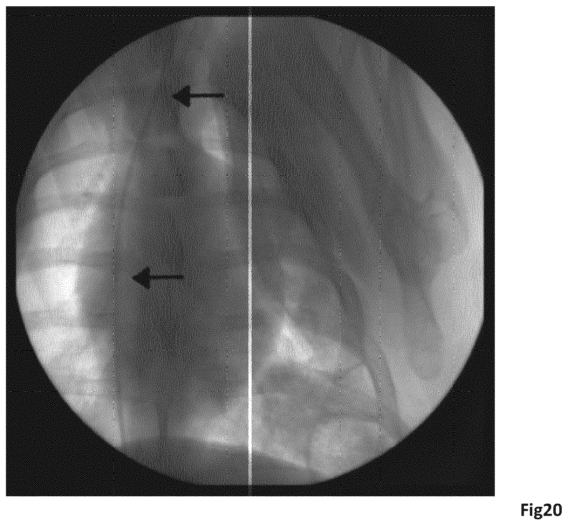

**Figure20:** Fluoroscopy of a pig chest showing a cylindrical distributor (RD®70) where arrows locate radiomarkers which delimit the stick (7cm). The stick starts from the superior vena cava then passes through the right atrium, and finishes in the inferior vena cava as shown in figure 15. The distributor is placed under the procedure described in example 13 with the difference that the used catheter is a peelable one enabling to remove it and only let the optical distributor in the central venous line. The optical distributor can be let during days, for chronic illumination.

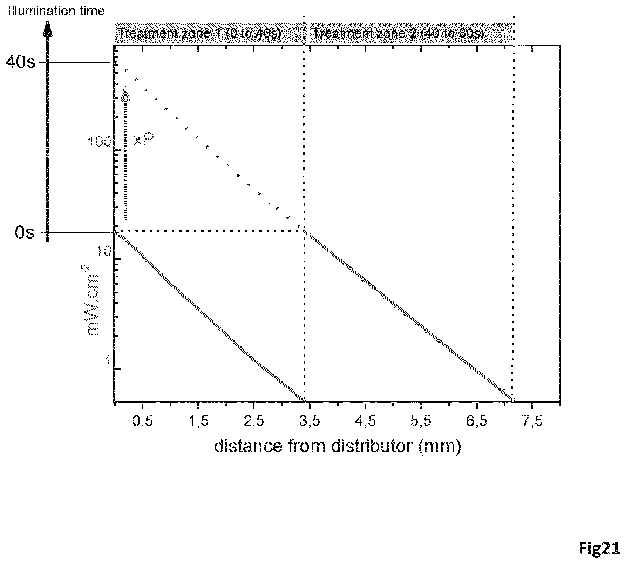

**Figure21:** Illumination scheme protocol exploiting a hot spot "line" at 689nm. In figure 4, it appears a relative potent line at 40s for fluence rate comprising between 0.5 to 20mW.cm$^{-2}$. This could be used to optimize the illumination treatment time by an increase of the treated depth per illumination time. It is known that the fluence rate emitted from an interstitial longitudinal uniform distributor placed into a semi-infinite medium can be approximated by an analytical expression based on Bessel function of second kind. Figure 21 shows the treatment protocol on the basis of the evolution of fluence rate perpendicular to the distributor axis defined within optical coefficient (ua = 0.17mm$^{-1}$: ueff = 0.88mm$^{-1}$). Between 0 to 40s, an optical power of 2.8 mW.cm$^{-1}$ is coupled to the distributor which induces a fluence rate of 20m W.cm$^{-2}$ in the vicinity of the distributor surface to a fluence rate of approximately 0.5mW.cm$^{-2}$ at ~3.5 mm. Between 40s to 80s, the optical power is adjusted (multiplication factor P) at 100 mW.cm$^{-1}$ in order to obtain a fluence rate of ~20 mW.cm$^{-2}$ at 3.5 mm whereas the fluence rate reach 0.5 mW.cm$^{-2}$ at ~7 mm. Therefore, using this hot spot line, in this case, the depth of treatment is 7 mm within a treatment time of 80 s.

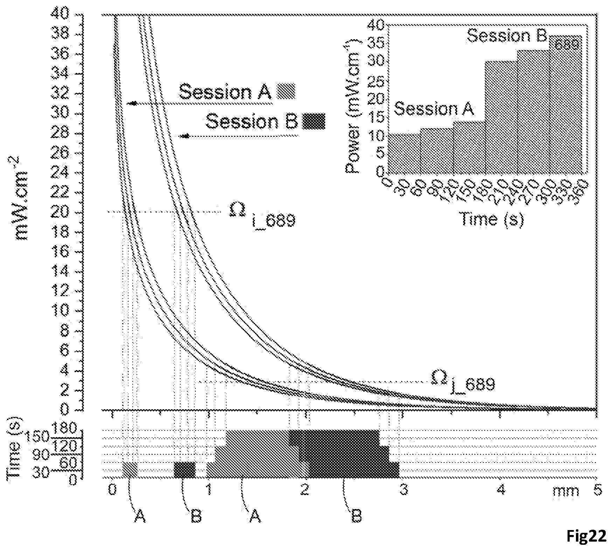

**Figure22:** Graphic illustration of the use of selection of combined hot spots of the same wavelength to treat simultaneously different parts of the biological object, within its specific illumination time. Based on the same simulation described in figure 21, where longitudinal distributor is placed into the myocardium, the figure shows the evolution of the fluence rate in the depth of the tissue for successive optical power applied within the time presented in inset. Using a particular combination of an hotspot spot $\Omega_{i,689}$ = (20 ±1 mW.cm$^{-2}$ ; 60 ±1s ) within another one which presents a lower fluence rate but a multiple of illumination time $\Omega_{j,689}$ = ( 3 ±1.6 mW.cm$^{-2}$ ; 180 ±1s ) for instance, to successfully treat conjoint superficial layers (using $\Omega_{i,689}$ ) each 60 s, whereas in parallel a deeply second zone is treating cumulatively (part of the fluence rate which is underlined in black). During the session A, three superficial layers (by increasing the optical power every 60 s in respect to $\Omega_{i,689}$ ) is treated (illumination time is projected on a lower graph) whereas a deeply zone will receive a succession of 3 x 60s in the range of 3 ± 1.6 mW.cm$^{-2}$. Since a part of the deep area have then received 180s, the optical power of the session is defined to avoid to illuminate parts which have already received 180s. Then as it is shown the starting optical power of session B is defined to continue the $\Omega_{j,689}$ which induces another part of the object will be subject to $\Omega_{i,689}$.

**Figure20:** Fluoroscopy of a pig chest showing a cylindrical distributeur (RD®70) where arrows locate radiomarkers which delimite the stick (7cm). The stick starts from the superior vena cava then passes through the right atrium, and finishes in the inferior vena cava as shown in figure 15. The distributor is placed under the procedure described in exemple 13 with the difference that the used catheter is a peelable one enabling to remove it and only let the optical distributor in the central venous line. The optical distributor can be let during days, for chronic illumination.

**Figure21:** Illumination scheme protocol illustrating how the "line" hot spot visible in Figure 4a can be exploited to minimize the illumination time with a "long" cylindrical light distributor inserted in a "large" biological object. In this geometry the evolution of the fluence rate as a function of the distance from the surface of the light distributor can be modeled by an analytical expression containing Bessel functions of the second kind. The evolution of the fluence rate as a function of the distance mentioned above is shown for the following optical coefficients of the biological object ($\mu_a$ = 0.17 mm$^{-1}$: $\mu_{eff}$ = 0.88 mm$^{-1}$) for two different linear power densities expressed in mW/cm of the light distributor length. The first one (2.8 mW/cm), applied for 40 s, induces the fluence rate of 20 mW/cm$^2$ at the light distributor surface, whereas this fluence rate is about 0.5 mW/cm$^2$ at a distance of 3.5 mm. The second linear power density (100 mW/cm) is applied between 40 and 80 s, thus resulting in a fluence rate of 20 mW/cm$^2$ at 3.5 mm whereas its value is 0.5 mW/cm$^2$ at 7 mm. Therefore, using this hot spot line the depth of treatment is 7 mm for a treatment lasting 80 s.

**Figure22:** Graphic illustration of the combined use of two hot spots, corresponding to the surfaces $\Omega_{i,689}$ and $\Omega_{j,689}$, to treat simultaneously different depths in the biological object, with one wavelength. Considering the geometric and optical conditions corresponding to Figure 21, the evolution of the fluence rate with depth is shown for different linear power densities that are applied sequentially. Using a particular combination of hot spots ($\Omega_{i,689}$: 20 $\pm$1 mW.cm$^{-2}$; 60 $\pm$1s), ($\Omega_{j,689}$: 3 $\pm$1.6 mW.cm$^{-2}$; 180 $\pm$1s) four layers are treated in two illumination sessions (A and B). During the session A, two layers are treated by increasing the linear power density by steps of 60 s until 180 s in order to use the hot spot $\Omega_{i,689}$, whereas the remaining two layers are treated at the while using the hot spot $\Omega_{j,689}$. As depicted in the insert located in the upper right corner of Figure 22, the total treatment time is 360 s. It should be noted, that the linear power density used for the session B is defined in such a way that the two treated layers located between 1 and about 3 mm are contiguous.

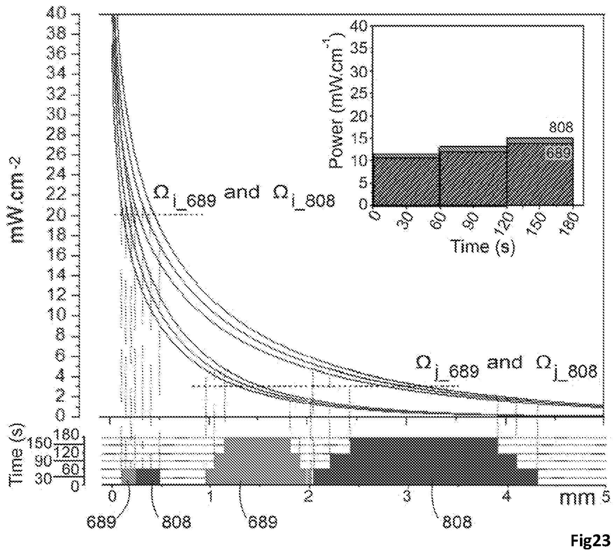

**Figure 23:** This figure is a generalization of Figure 22, when two wavelengths, presenting different penetration depths in the tissue, are applied synchronously. The combined use of these two wavelengths enables, as a consequence, to reduce the treatment time mentioned in Figure 22 by factor of 2.

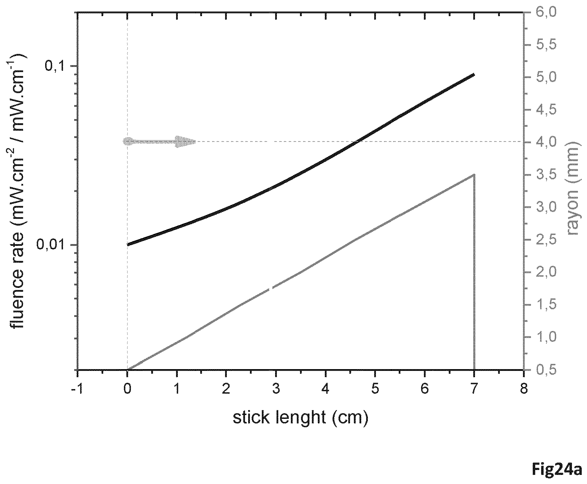

**Figure 24a** This figure presents the normalized fluence rate, expressed in (mW/cm$^2$)/(mW/cm), around a conical light distributor presenting a length of 7 cm, surrounded by a fluid with optical properties corresponding to the blood ($\mu_a$ = 0.25 mm$^{-1}$, $\mu_{eff}$ =1.07 mm$^{-1}$). The arrow represents a blood volume element propagating according to a trajectory that is parallel to the light distributor axis at a distance of 4 mm from this axis. This figure illustrates that the blood volume element will be exposed to the desired normalized fluence rate independently of the position of the blood volume element.

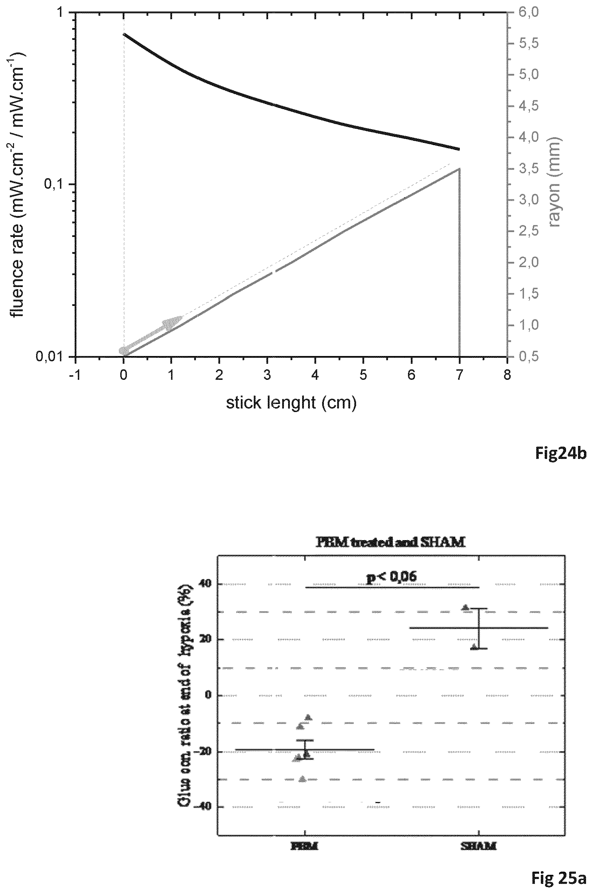

**Figure 24b:** This figure represents the same situation as Figure 24a but the blood volume element propagates at the surface of the conical light distributor.

**Figure 25a** Glycaemia ration between the beginning and the end of hypoxemia event. PBM illumination in deoxygenated blood during hypoxemia significantly reduces the level of glycaemia in blood.

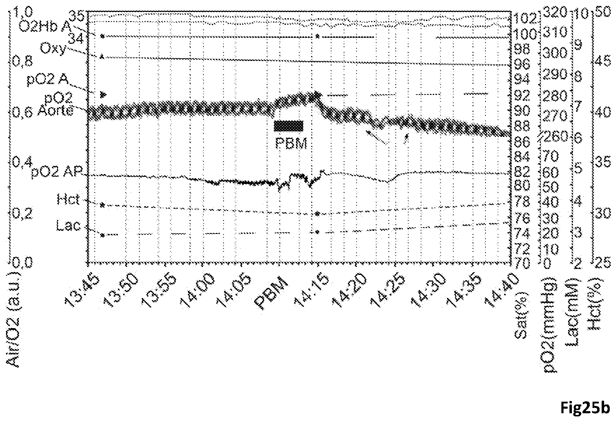

**Figure 25b:** Monitoring of the Clark probe in the aorta of the arterial partial pressure during the PBM illumination in the lung arteries in normoxia.

## General description of the invention

The inventors have shown that the control of the light dosimetry (fluence rate [mW/cm$^2$]; light dose [J/cm$^2$]) and spectroscopy (wavelength(s)) as well as the illumination duration and the time of illumination are crucial to induce optimal PBM effects. This observation is very important since the PBM effects are known to be bimodal (sometimes qualified as biphasic), i.e. too high or too low fluence rates and/or light doses significantly reduce the PBM effects and are therefore frequently associated to the Arndt-Schultz rule observed in pharmacology. This bimodal response has been reported by numerous groups looking at various "standard" effects (mitochondria membrane potential; ATP production; etc) [Huang 2009; Hamblin 2017; Hamblin 2018]. Looking at the PBM effects on the endogenous production of PpIX in different cell lines, including glioma cells and human cardiomyocytes (HCM), the inventors found that both the fluence rate and the illumination time must be applied in a controlled manner. These two parameters must be applied with specific values, for a given illumination in each parts of the volume of the biological object to optimize PBM effects. In contradiction to what is reported in this field, the inventors have discovered that the bimodal effects of PBM are only observed for a specific set of these parameters. These sets of parameters are defined as "hot spots" (figure 4a and 4b) in this document. Moreover, the inventors have shown that some of these hot spots are wavelength independent.

It is also established that the optical properties of biological tissues, described mostly by their absorption and scattering coefficients, have an important impact on the propagation of the light around a light source [Tuchin, 2015; Hamblin 2017; Hamblin 2018]. In general, the fluence rate (and the light dose) decreases with the distance from the light source due to the absorption and scattering of the light in the tissue (see Figure 1). Therefore, the fluence rate and/or the light dose in the tissues are, in most situations, never optimal at the same time in different locations in the tissues treated by PBM. The existence of the parameters hot spots mentioned above combined with: i) the heterogeneous distribution of the light in the tissues treated by PBM, and ii) the very limited control of the light delivery and dosimetry by the vast majority of the research or clinical groups active in this field explain the limited and contradictory outcomes reported in the literature [Chung 2012]. This situation also explains why PBMT is poorly used at present.

An object of the present invention is to provide an improved PBM for the treatment of biological objects, such as tissues, circulating blood and/or the lymph.

Another object of the present invention is to provide an efficient treatment of ischemia reperfusion injuries, such as myocardial infarction (MI), by PBM applied with the conditions and methods mentioned above and below.

Another object of the present invention is to provide an efficient treatment of fibrillations, including atrial fibrillations, by PBM applied with the conditions and methods mentioned above and below.

Another object of the present invention is to provide an efficient PBM-based treatment of metabolic disorders such as type 2 diabetes, hepatic diseases or hormones secretion with the conditions and methods mentioned above and below.

Another object of the present invention is to provide an efficient treatment of systemic inflammation or exacerbated systemic immune response by PBM applied with the conditions and methods mentioned above and below.

Another object of the present invention is to provide an efficient PBM-based treatment to maintain systemic homeostasis during hypoxemia and or hypoxia with the conditions and methods mentioned above and below.

Another object of the present invention is to provide efficient methods in cells-based therapy notably to increase the proliferation rate of stem cells as well as to trig cells differentiation.

Another object of the present invention is to provide an efficient treatment/diagnosis of PpIX-based methods, for instance in photodynamic therapy or in cancer detection by imaging the PpIX fluorescence. Embodiments of this invention involves: the use of a helmet, integrating light emitting diodes, which induce a PBM illumination through the skull on a specific area of the brain before the PhotoDynamic Detection (PDD) or PhotoDynamic Therapy (PDT)procedures used to manage cancers.

Another object of the present invention is to increasing and homogenizing the endogenous production of PpIX in plants and larvae. One embodiment of this approach is to increase the efficacy of the phototoxic effects induced in weed/larvae.

Another object of the present invention is to provide an efficient treatment of conditions by PBMT based on the monitoring of the metabolic activity. This monitoring, based on a frequency analysis of parameters reflecting the metabolic activity, enables to adjust the radiometric (fluence rate, illumination time, light dose, ...) and spectral (wavelength(s)) parameters in such a way that the PBM effects are maximized. This monitoring can also be used to assess the status of the metabolic activity to determine the optimal light application moment. Embodiments of the present invention involve the use of standard probes to measure physiological of biochemical parameters reflecting the metabolic activity. As mentioned below, such probes include, thermocouple, Clark's pO2 probes or optical fiber-based probes to measure these parameters. The signals delivered by these probes are then processed by a dedicated unit to perform the frequency analysis enabling to extract parameters providing information on the PBM effects and metabolic activity.

The above objects are achieved with the device and methods of the invention as defined in the claims.

Advantageously the device and method according to the invention are characterized by the fact that the PBM effects are induced by the generation of a specific fluence rate during a specific time corresponding to specific "hot spots" as selection conditions (see below), successively in each parts of the volume of the biological object.

An illustrative embodiment of the present invention consists to use one or several light source(s) coupled to one or several light distributor(s) applying a specific fluence rate during a specific time corresponding to one or several "hot spots" presented in figure 4a and 4b to increase the metabolism in the tissues/conditions of interest mentioned below.

The invention optionally also encompasses devices and methods predicting the time for applying PBM on a biological object, based on a frequency analysis (Differential analysis of temporal signal (integration of the past or derivation of the present)) of fluctuations of parameters reflecting the metabolic activity or predictive methods based on artificial intelligence of one or several parameters reflecting the PBM effects or the metabolic activity of the biological object. Optionally, the light power delivered by the device, the illumination time and the moment of PBM application relative to variations of the metabolic activity is adapted on the basis of feedback observables (see the list given below) to optimize the PBM effects.

**Observable feedbacks:**

a) The temperature.

Figure 2 presents the evolution of the temperature measured with a thermocouple and a probe used to measure the oxygen consumption rate (OCR), which reflects the metabolic activity, in an experimental setup developed by the inventors. This setup consists in a monolayer of Human CardioMiocytes (HCM) positioned at the bottom of a petri dish which was covered with a 15 mm thick layer of physiologic water. The thermocouple and the probe to measure the OCR as well as the partial pressure of oxygen ($pO_2$) were both positioned 1 mm above the cell monolayer.

As can be seen on figure 2, significant and easily measurable changes of the temperature are observed while the $pO_2$, and hence the OCR, are changing. The temperature increases with an increasing activity of the OCR. Interestingly, the high-frequency oscillations observed for the OCR and the temperature after a maximal value of the $pO_2$ are in phase and synchronous.

Therefore, measuring the temperature provides an observable feedback to monitor and/or adapt the light dose used for PBM. Measuring the temperature also enables to determine the optimal PBM illumination time relative to variations of the metabolisms.

b) The tissue autofluorescence reflecting the redox state of enzymes involved in the metabolism.

Oxidation is the main process producing the necessary energy in cells. It can occur in aerobic or anaerobic conditions. In many situations, biological oxidation starts with substrate dehydrogenation, i.e. the displacement of two hydrogen atoms, whereas coenzymes such as NAD+, NADP+ and FAD serve as acceptors of these atoms. Since the cellular concentration of these coenzymes is low, they must be recycled by re-oxidization. Therefore, these coenzymes serve as primary donor and acceptor in the process of oxidative phosphorylation (OXPHOS) [Ferraresi, 2012]. Since NADH and FAD are bound to many enzymes involved in metabolic pathways [Alberts, 2002], the relative ratio between the NADH and FAD binding sites changes as well when the cells are switching their metabolism [Banerjee, 1989]. Hence, cell responses to changes of the $O_2$ level (change of metabolic activity) resulting from PBM can be monitored looking at their effects on FAD and NADH.

These coenzymes can be studied non-destructively looking at their autofluorescence, i.e. without the addition of exogenous probes [Ramanujam, 2001]. One of the most common optical techniques giving information about the metabolic state of cells is based on the determination of the redox ratio of FAD and NADH by fluorescence spectroscopy [Chance, 1979; Walsh, 2012; Blacker, 2016], in particular time-resolved fluorescence spectroscopy [Skala, 2007; Skala, 2010; Kalinina, 2016; Walsh, 2013], a field corresponding to the expertise of the inventors since more than two decades [Wagnières, 1998]. For instance, in cancer cells, an increase of cellular metabolism is usually indicated by a decrease of the redox ratio [Chance, 1989].

Therefore, steady-state and/or time-resolved fluorescence spectroscopy (or imaging) of the tissue autofluorescence is an interesting feedback observable to monitor or adapt the light dose used for PBM. Interestingly, the combined use of this approach with direct $O_2$ sensing based on the time-resolved luminescence spectroscopy of molecular probes (PPIX or exogenous $pO_2$ probes as proposed by Kalinina et al. [Kalinina, 2016]) or interstitial Clark's probes, provide unique information on the PBM effects. Monitoring these parameters is minimally invasive and fast.

c) The assessment of the hemoglobin saturation.

In normal conditions, the body maintains a stable level of oxygen saturation for the most part by chemical processes of aerobic metabolism associated with breathing. However, it is well known that the hemoglobin saturation can change for different metabolic activities.

Since many methods are well established to measure the hemoglobin saturation, notably the peripheral or central venous saturation which is known to reflect the cardiac output excepting in sepsis shocks, applying this approach to monitor the changes of metabolic activities induced by PBM is of high interest.

In addition, since several gazes can be endogenously produced and diffused within the tissue and the circulating blood, and can bind to various metalloproteins, which present strong optical absorption bands, for instance, NO or $H_2S$ can bind deoxyhemoglobin to create nitrosyl hemoglobin or sulfhemoglobin or carboxyhemoglobin which decrease the level of available deoxyhemoglobin, and since PBM can induce photodissociation (Photolysis) of metalloproteins as hemoglobin, especially nitrosyl [Lohr, 2009] with a simultaneous formation of methemoglobin, and since the changes of these different forms of "hemoglobin" can be measured (via optical absorption measurement [Van leeuwen, 2017] ), monitoring the changes of the metabolic activities induced by PBM through the assessment of these various metalloproteins complexes is of high interest.

d) The pH and or the level of bicarbonate ($HCO_3^-$).

It is well known that glycolysis, which is the metabolic pathway that converts glucose into pyruvate, leads to an acidification of the extracellular surrounding media. This acidification frequently results from the excretion of lactic acid after its conversion from pyruvate [Wu, 2007]. Since many methods are well established to measure the pH or the level of bicarbonate in tissues or in the circulating blood, applying this approach to monitor the changes of metabolic activities induced by PBM is of high interest.

As tissular (or saliva) lactate concentration can be constantly assess with minimally invasive device such subcutaneous microneedle [Tsurukoa, 2016] or in mouth, this approach can be used to monitor the changes of metabolic activities induced by PBM.

e) The concentration of ROS.

Reactive oxygen species (ROS) are chemically reactive chemical species containing oxygen. Examples include peroxides, superoxide, hydroxyl radical, singlet oxygen, [Hayyan, 2016] and alpha-oxygen. In a biological context, ROS are formed as a natural byproduct of the normal metabolism of oxygen and have important roles in cell signaling and homeostasis [Devasagayam, 2004]. ROS are produced during a variety of biochemical reactions within the cell and within organelles such as mitochondria, peroxisomes, and endoplasmic reticulum.

Effects of ROS on cell metabolism are well documented in a variety of species [Nachiappan, 2010]. These include not only roles in apoptosis (programmed cell death) but also positive effects such as the induction of host defense genes and mobilization of ion transport systems. This implicates them in control of cellular function. In particular, platelets involved in wound repair and blood homeostasis release ROS to recruit additional platelets to sites of injury. These also provide a link to the adaptive immune system via the recruitment of leukocytes.

Abnormal levels of ROS are implicated in numerous pathologies through a strong modulation of various biological cascades [Sies, 2020]. Interestingly ROS level are also primordial for cell reprograming [Bigarella, 2014; Zhou, 2016] and tissular remodeling. For instance, their production kinetics depend on a broad spectrum of extrinsic or intrinsic repetitive stimulus such as hormones secretion or mechanical forces (like vascular shear stress), which influence directly the tissular behavior and properties [Hwang, 2003; Brandes, 2014], and finally phenotypic aspects.

Since many methods are well established to measure the ROS in tissues, applying this approach to monitor the changes of metabolic activities induced by PBM is of high interest.

As ROS and reactive nitrogen species (RNS) are intricate by nature [Moldogazieva, 2018] and since many methods to assess to RNS are well established [Griendling, 2016], the termed of reactive oxygen and nitrogen species RONS should be used here. Actually, in a full extends, the term should be reactive oxygen and nitrogen and sulfur species (RONSS).

f) The level of $H_2S$.

Hydrogen sulfide ($H_2S$) exert a wide range of actions on the whole organism. It is an epigenetic modulator inducing histone modification particularly via DNA demethylation, a process which permit cell differentiation [Yang, 2015]. It is fundamental in aging process of aerobic living organism by maintaining a high level of copy number of mitochondrial DNA [Li, 2015], as well in senescence process through sirtuin 1 activation. Interestingly, $H_2S$ is the only species which is both substrate and inhibitor of the OXPHOS inside the mitochondria depending of its concentration [Szabo, 2014], which may to be in parallel to the famous observation that exogenous $H_2S$ inhalation induce a suspended animation-like state in small mammals, known as artificial hibernation, or hypometabolism [Blackstone, 2005]. $H_2S$ is known to protects against many cardiac conditions, including pressure overload-induced heart failure [Snijder, 2015]. This supports the hypothesis that endogenous $H_2S$ is a regulator of energy production in mammalian cells particularly during stress conditions, which enables cells to cope with energy demand when oxygen supply is insufficient [Fu, 2012].

Moreover, we observed in fertilized chick's eggs an in-ovo anoxia reoxygenation. This study was performed by gently placing an $H_2S$ microprobe above the ventricle of the chicken or the dorsal aorta. We observed that the $H_2S$ level increased significantly within the anoxic (transient) cardioplegia (or when the heart beat extremely slowly) and decrease when it beat again. Therefore, it appears that the blood flow plays a role by removing endogenous production of $H_2S$ which could bind deoxyhemoglobin to form sulfhemoglobin and propagate it.

Since many methods are well established to measure H2S in tissues [Olson, 2012], applying this approach to monitor the changes of metabolic activities induced by PBM is of high interest.

g) The level of hydrogen selenide ($H_2Se$).

Alongside oxygen and sulfur, selenium, the constitutive element of $H_2Se$ belongs to the chalcogens group and have similar excretory and metabolic pathways. Analog to $H_2S$, $H_2Se$, is an endogenous small gaseous molecule which can induce a suspended animation like state and show reperfusion injury protection [Iwata, 2015]. It reversibly binds COX, which inhibits the mitochondrial respiration and argued to be the fourth gasotransmitors with $H_2S$, NO and carbon monoxide (CO) [Kuganesan, 2019]. Moreover, incorporated into numerous selenoprotein oxidoreductase enzymes as glutathione peroxidase, it is essential for maintaining redox-status homeostasis in health and diseases, and its deficiency induces a substantial increase of ROS, which is suspected to be one important cause of cancer and CVD [Bleys, 2008].

Since many methods are well established to measure H2Se in tissues or Selenium in serum, applying this approach to monitor the changes of metabolic activities induced by PBM is of high interest.

h) The concentration of ions.

Ions play an important role in the metabolism of all organisms as reflected by the wide variety of chemical reactions in which they take part [van Vliet, 2001]. Ions are cofactors of enzymes, catalyzing basic functions such as electron transport, redox reactions, and energy metabolism; and they also are essential for maintaining the osmotic pressure of cells. Because both ions limitation and ions overload delay growth and can cause cell death, ion homeostasis is of critical importance to all living organisms.

Since many methods are well established to measure ions in tissues (in particular calcium, potassium, chloric and/or hydrosulfide ions), applying this approach to monitor the changes of metabolic activities induced by PBM is of high interest.

i) The level of cytochrome, including cytochrome c oxidase, by NIRS.

It is well established that broadband Near InfraRed Spectroscopy (NIRS) can be used to monitor concentration changes of the oxidation state of cytochromes as cytochrome-c-oxidase ($\Delta$oxCCO) which plays a key role in the mitochondrial respiration [Roever, 2017].
Since different methods are well established to measure $\Delta$oxCCO in tissues (or other cytochromes involved in the metabolism), applying these methods, including NIRS to monitor the changes of metabolic activities induced by PBM is of high interest.

j) The use of medical (functional) imaging techniques (MRS "Magnetic Resonance Spectroscopy"; MRI "Magnetic Resonance Imaging"; NMR "Nuclear Magnetic Resonance"; PET "positron emission tomography"; EPR "Electron Paramagnetic Resonance"; SPECT "single photon emission computed tomography" BOLD "Blood oxygenation level dependent" NIRS "Near infrared Spectroscopy").

Metabolic imaging focuses and targets changes in metabolic pathways for the characterization of various clinical conditions. Most molecular imaging techniques are based on PET and MRS, including conventional $^1$H and $^{13}$C MRS at thermal equilibrium and hyperpolarized magnetic resonance imaging (HP MRI). The metabolic pathways that are altered in many pathological conditions and the corresponding probes and techniques used to study those alterations have been reviewed by Di Gialleonardo et al. [Di Gialleonardo, 2016]. In addition, Fuss et al. [Fuss, 2016] described the use of medical imaging to address various conditions in humans.
Since many metabolic imaging-based methods are well established to assess the metabolism, applying these methods, including functional metabolic imaging, to monitor the changes of metabolic activities induced by PBM is of high interest.

k) The vascular tone and the vasomotion.

Vascular tone refers to the degree of constriction experienced by a blood vessel relative to its maximally dilated state. All arterial and venous vessels under basal conditions present some degree of smooth muscle contraction between balance of constrictor and dilatator influences that determines the diameter of the vessel, e g the vascular resistance to adapt / regulate blood flow and pressure. Basal vascular tone differs among macro and micro-circulation and organs. Certain organs have a large vasodilatory capacity (e.g., myocardium, skeletal muscle, skin, splanchnic circulation) hence a high vascular tone, whereas others organs have relatively low vasodilatory capacity (e.g., cerebral and renal circulations), hence a low vascular tone.
The vascular tone regulation differs among the macro (arteries, veins) and the micro (arterioles, venules, capillaries). Notably, even if the tone can be modulated via extrinsic factor (nerves, circulating metabolites), blood vessel can exhibit spontaneous oscillations (vasomotion) which give rise to flow motion [Aalkaejer, 2011]. Therefore, through the dependence of the vascular tone in a multiplicity of actuator from local to systemic, the analysis of this tone, give insight on the metabolic activity, and can reflect the degree of aging [Bentov, 2015] and many pathophysiological conditions, as ulcer risk, type 2 diabetes [Smirnova,2013], endothelial dysfunction or hypertension [Ticcinelli, 2017], renal diseases [Loutzenhiser, 2002] [Carlstrom, 2015] or metabolic syndrome [Walther, 2015]. Moreover, the assessment of the skin microvascular endothelial function is used as diagnosis as well as prognostic of CVD [Hellman, 2015].
Since many methods are well established to assess to the vascular tone and the vasomotion, such as videocapillaroscopy, plethysmography [Tamura, 2019], laser doppler flowmetry, pressures measurement via cutaneous vascular conductance (CVC) or time frequency analysis as example, and since all the cardiovascular system is argued to be a single entity of coupled oscillators in a dynamic point of view [Shiogai, 2010], any methods which enable to assess to the change of metabolic activities induced by PBM (including heart rate variability (HRV) which give information on the autonomic nervous system via ECG or heart sound measurement [Alvarez, 2018] via phonocardiogram (PCG) [Patidar, 2014] are of high interest. As observe by the inventors along surgical procedure respiratory frequency variability (RFI) [stevanovska,2007] or ballistocardiography to monitor the changes of metabolic activities induced by PBM is of high interest.

1) The use of electromagnetic endogenous signals.

Electrocardiogram (ECG), electroencephalogram (EEG) and electromyogram (EMG) are standard measurements of electrical activity of the metabolism of the heart, the brain and the muscle respectively. Novel ECG analysis based on signal computational classification [Patidar, 2015] are promised tools in heart diagnosis notably giving insight in coronary artery disease [Kumar, 2017] [Acharia, 2017], arrhythmia and ischemia disorders [Bhoi, 2017]. Same kind of analysis have been performed on EEG which show interesting outcomes in epileptic seizures, the most common brain disorders [Bhattacharyya, 2018].

Since many methods are well established to monitor these electromagnetic endogenous signals, methods which enable to assess to the change of metabolic activities induced by PBM are of high interest.

m) The use of bio impedance signals.

It can be used in the clinic for measuring various physiologic parameters [Petterson, 2016]. This approach is used for pacemakers as Ensite from St Jude Medical, OptiVol from Medtronic and closed loop stimulation from biotronik.

Since many methods are well established to measure electrical bioimpedance in tissue or directly on the skin, any these methods which enable to assess to the change of metabolic activities induced by PBM is oh high interest.

n) The presence of markers in the circulating blood.

A long list of circulating markers of interest to monitor the light dose during PBM includes metabolites (succinate, pyruvate, etc.), coagulation factors, apoptotic factors, (pro and anti) inflammatory factors, as well as hepatic factors, mitokines, or level of isolated mitochondria for instance. It should be noted that only a few of them is enumerated here.

Glucose level: Many pathologies are associated with a dysregulation of circulating glucose level, which directly induces systemic metabolic disorders, as it is the case for diabetes. Hence, monitoring the change of the metabolic activities through the assessment of the glycaemia is of high interest.

Succinate: Succinate is a key intermediate of the tricarboxylic acid cycle (TCA) cycle which plays an essential role in anabolic and catabolic pathways. Moreover, it is notably associated with reperfusion injuries [Chouchani 2014]. Mitochondria are the physiological source of succinate, however accumulated succinate can be transported into the cytosol and then in the circulating blood. This TCA cycle intermediate connects intracellular metabolic status and intercellular signaling [Tretter, 2016]. Level of succinate in blood can vary from 2 to 20 $\mu$M, where this concentration can increase, with hypoxic stress, pro-inflammatory stimuli, exercise, or with pathological conditions such as type 2 diabetes, obesity or ischemia reperfusion injury [Grimolizzi, 2018]. Since circulating level of succinate can be monitored via bioluminescent assay, or Raman spectroscopy the assessment of the change of metabolic activities induced by PBM via the circulating level of succinate is of high interest.

Lactate and lactate dehydrogenase (LDH): LDH is a common marker of cell damage and cell death. In addition, LDH produced during anaerobic exercise can be reduced by PBM [Park, 2017].

Hence, using these markers to assess the change of metabolic activities induced by PBM is of high interest. Moreover, combination of LDH level with aspartate aminotransferase (AST) level serves as a potent indicator of the damage to the body's tissues. It should be noted that lactate levels can be assessed with new aerometric method directly on the saliva [Tamura, 2018].

Since the high level of metabolism activites in inflammatory or immune response, notably by the capacity of immune cells to change their phenotype, serum/plasma level of immune/inflammatory markers, such as: mtDNA copies, number of leucocytes, total antioxidant capacity, bicarbonate, malondialdehyde (MDA), uric acid, bilirubin, level of cytokines or chemokine markers such as interleukins IL2, IL6, IL7, IL10, IL18 or TNF$\alpha$ for instance, as well as macrophage inflammatory protein 1-$\alpha$, IP10, MCP1, as well as activation of lymphocytes T and / or monocytes M2 through flux cytometryare are also of high interest.

Serum/plasma levels of thioredoxin: The level of this enzyme is elevated in infection, ischemia-reperfusion, and other oxidative stresses. Therefore, they are good markers for monitoring of the oxidative stresses. Plasma levels of thioredoxin are also elevated in patients with coronary spastic angina and other cardiovascular diseases [Nakamura, 2004].

Cardiac markers: Several established markers (myoglobin, creatine kinase isoenzyme, troponin I and T, B-type natriuretic peptide, transaminase) are clinically used for cardiac infarction diagnosis and also for other organs injuries. To a lesser extent, LDH, glycogen phosphorylase and recently ischemia-modified albumin can be used in diagnosis within 30-minute assay [Dasgupta, 2014]. This is also the case for thioredoxin level [Jekell, 2004].

Level of circulating eNOS as well NO or nitrite or nitrate: The levels of these compounds are essential, notably for the regulation of systemic blood pressure and systemic homeostasis [Wood, 2013]. By extension, also levels of circulating $H_2S$ or sulfite or sulfate level can be assessed to monitor the change of the metabolic activities induced by PBM.

Circulating mitochondria: It has been recently shown that cell free functional mitochondria are present in the circulating blood. Moreover, mitokynes are important in the metabolic remodeling, especially in the heart failure [Duan, 2019]. Therefore, monitoring of the change of the metabolic activities through the assessment of the circulating mitochondria level or mitokynes level is of high interest.

o) The use of voltage-sensitive fluorescent dyes for the optical mapping of cardiac electrical signals.

Although, the optical imaging of cardiac electrical signals using voltage-sensitive fluorochromes (VSF) has only been performed in experimental studies because these VSFs are not yet approved for clinical use, FDA approved dyes, such as Indo Cyanine Green (ICG) [Martisiene, 2016], exhibits voltage sensitivity in various tissues, thus raising hopes that electrical activity of cardiac tissues could be optically mapped in the clinic. Therefore, methods based on the use of voltage-sensitive dyes to map (or to assess locally with a "point measurement" system) the cardiac electrical signal to monitor/adapt the light dose during PBM is of high interest.

p) The use of redox sensors to assess of the status of various tissular redox states. Since metabolic and redox reactions are intricated and since many methods are based on the measurement of redox sensors proteins to asses to metabolic activities, monitoring changes of metabolic activities induced by PBM with redox indicators probes is of high interest.

q) The use of ultrasounds to assess the status of various tissues, including cardiac tissues.

Ultrasonography is a well-established method to investigate cardiac tissue. Many parameters characterizing the heart tissues as well as the blood flow are routinely obtained during ultrasonography.

Therefore, methods based on the use of ultrasonography to monitor the light dose during PBM are of high interest.

r) The use of the $pO_2$ (and / or the OCR) to reflect the metabolic activity of various tissue and / or of the whole body through the measurement of the partial pressure of arterial oxygen ($PaO_2$) and the fraction inspired oxygen ($FiO_2$).

$pO_2$ can be easily measured within exogenous or endogenous probes in different compartment (tissular or organ) or different organelles within the cell. For instance, such probes can be optically detected. Other techniques, such as EPR oximetry, polarographic electrodes or BOLD imaging are of high interest to assess changes of metabolic activities induced by PBM.

s) The use of hemodynamic variables.

In the clinic, real time assessment of these variables is a must to monitor the metabolic activity, especially in case of cardiovascular injuries. Such measurements notably involve, arterial and venous gases pressures, cardiac output, stroke volumes, capillary pressure, as well as systemic and pulmonary resistance. Therefore, the use of these methods to assess changes of metabolic activities induced by PBM are of high interest.

t) The use of Krebs cycle enzymes kinetics.

It is well known that Krebs cycle enzymes kinetics are good markers of metabolism notably to assess to the level of mitochondrial proteins. Since, for instance, acotinase or succinate dehydrogenase activities are commonly measured in clinics, the use of these methods to assess changes of metabolic activities induced by PBM are of high interest.

w) The level of PpIX

Protoporphyrin IX, is a precursor of numerous organometallic proteins, such as hemoglobin and chlorophyll. The inventors have shown that cells treated by PBM tend to increase their endogenous production of PpIX. Therefore, the Δuse of methods based on the detection of the PpIX level to assess changes of metabolic activities induced by PBM are of high interest. Since the heme concentration is a feedback parameter in the PpIX endogenous production pathway, by extension, measuring the level of circulating hematocrits to assess changes of the metabolic activities induced by PBM is of high of interest.

x) Monitoring of the metabolomics and lipidomics , in particular oxylipines.

Oxylipines are bioactive metabolites derived from the oxygenation of polyunsaturated fatty acids. Furthermore, they play a key role in the progression of cardiovascular disease thrombosis and risk factors. Hence, their monitoring is of high interest.

y) The level of glycoproteins

It is well known that glycoproteins, comprising of protein and carbohydrate chains, are involved in many physiological functions, including immunity. They possess receptors signaling domains that recruit signaling molecules.

z) Level of glycerol

Glycerol can serve as a marker of apoptosis. One of the function of glycerol is that is serves as a chemical chaperone. In particular, it possesses an ability to enhance the expression of apoptotic regulators (*bax*).

aa) The assessment of the immunomodulatory effects induced by PBM can be monitored by pro-inflammatory circulating monocytes like CD14, CD16 which can differentiate to the dendritic cells. It can be also assessed by the cytokines profiles of macrophages.

ab) Monitoring of the basis of the level of oxytocin. For the latter, it has been shown that monitoring of the oxytocin levels in the intensive care unit in the premature infants serves as a relevant marker of pain.

Optionally, the light power the illumination duration and the application time of PBM define by this device, is to be combined with the administration of exogenous stimulus wherein the stimulus could be an agents (see the list given below) to increase the PBM effects. It should be note that the time between the administration of exogenous agents and the PBM illumination may take into account the assimilation duration as well as activation kinetics of the agent.

The inventors demonstrated a synergy resulting from the combined administration of PBM with notably exogenous agents (sulfur donors) and nitric oxide donors.

As already shown by the inventors, an administration of ALA combined with PBM increases the PpIX build up and, consequently, the level of endogenous PpIX. Therefore, the coadministration of ALA and light is of high interest to increase PBM effects. It should be noted that other exogenous agents can be combined within PBM to increase its effects, as indicated below.

As presented in figure 3, the inventors recently observed that when PBM is combined with an FDA approved exogenous agent, sodium thiosulfate (STS), which are sulfur donors, the angiogenesis observed on the chick's Embryo Chorioallantoic Membrane (CAM) is even more stimulated. This strongly suggests that the combination of PBM with exogenous such agents stimulates even more angiogenesis, or the metabolic activity, than PBM or such agents applied separately.

The assessments of these PBM effects on angiogenesis were performed using an approach based on fluorescence angiographies performed on the CAM several days after PBM. Fig. 3 presents a typical CAM fluorescence angiogram (left image) characterized quantitatively with an image processing and analysis software developed in our laboratory [Nowak-Sliwinska, 2010]. The main objective of this development was to characterize dynamic changes taking place in the capillary/vessels network of the CAM between embryo development day (EDD) 6 and 12, when the CAM vessels are monitored using an epi-fluorescence microscope equipped with a scientific camera following the intravenous (iv) injection of a fluorescent agent [Nowak-Sliwinska, 2010]. From the resulting angiogram, 3 descriptors are extracted: the number of branching points/mm$^2$, the mean area of the vessels network meshes, and the mean of the 3$^{rd}$ quartile of the mesh area histogram. As presented in Figure 3, our proof-of-concept results demonstrates that PBM significantly stimulates the CAM angiogenesis. This effect is even more pronounced if PBM is combined with an exogenous application of 175 mM STS.

Interestingly, in parallel, the inventors observed in-ovo, through the monitoring of the $H_2S$ and NO level on the chicken embryo, that a topical application of STS induced a significant increase of NO after a long time (6 at 12 hours), whereas, when performing a PBM irradiation 1 - 2 hours after the STS application, the time when NO was produced was significantly reduces, typically down to one hour.

Since STS is a clinically approved $H_2S$ donor [Snijder, 2015] to protects against many cardiac conditions, as also already reported for $H_2S$ [Yu, 2014], including pressure overload-induced heart failure via upregulation of endothelial nitric oxide (NO) synthase [Kondo, 2013] as well as renal ischemia / reperfusion injury [Bos, 2009], the combined use of PBM, applied with the device/protocol mentioned above, with the administration of $H_2S$ donors (such as STS or methylsulfonylmethane (MSM), or dithiolthiones for instance, or other donors presenting different $H_2S$ kinetics release) and/or NO donor substances, as for instance arginine, including NO itself, is of high interest.

Since Cysteine is an important source of sulfide in the human metabolism, combining the administration of this proteinogenic amino acid, or derivatives thereof, such as selenocysteine, or synthetic form as N-acetylcysteine is of high interest to potentiate the effects of PBM.

MSM, a naturally occurring organosulfur compound, is utilized as an alternative source of biologically active sulfur. It is mostly used for anti-inflammatory treatments. It has been investigated in animal models, as well as in many human clinical trials [Butawan, 2017]. MSM is also recognized for its antioxidant capacity and it was proposed that the antioxidant mechanism acts indirectly via the mitochondria rather than directly at the chemical level [Beilke, 1987]. As an FDA approved substance, MSM, is well-tolerated by most individuals at dosages of up to four grams daily, with very few side effects [Butawan, 2017]. Results from in vivo and in vitro studies indicate that MSM actions are at the crosstalk of oxidative stress and inflammation at the transcription and sub-cellular levels [Butawan, 2017]. Interestingly, Kim et al. [Kim, 2009] demonstrated that MSM can also diminish the expression of inducible nitric oxide (NO) synthase (iNOS) and cyclooxygenase-2 (COX-2) through suppression of the nuclear factor-kappa B (NF-κB), a transcription factor involved in the immune and cellular responses to stress. This observation is highly interesting since NO is a powerful vasodilator involved in many metabolic functions. As some other gas transmitters, called gasotransmitors [Donald, 2016], NO can have differential effects depending on its local concentration and microenvironment [Thomas, 2015] which can impact many different processes [Rapozzi, 2013; Reeves, 2009]. It has also been suggested that PBM causes NO photodissociation from COX [Karu, 2005; Lane, 2006]. Concomitantly, NO photodissociation from other intracellular "reservoirs" such as nitrosylated forms of myoglobin and hemoglobin have also been hypothesized [Lohr, 2009]. It is well established that cell respiration is down regulated by the NO production by mitochondrial NO synthase. The $O_2$ displacement from COX by NO inhibits cellular respiration, and ATP production [Antunes, 2004; Cooper, 2008]. Therefore, it is believed that PBM increases ATP production. An alternative and, possibly, parallel mechanism to explain the release and/or increase of NO bioavailability by PBM could be linked to an action of COX as a nitrite reductase enzyme (a one-electron reduction of nitrite gives NO), in particular when the $O_2$ partial pressure is low [Ball, 2011].

All together, these observations indicate that MSM has an indirect effect on the mitochondrial electron transport chain (ETC) through its NO modulation. In addition, this analysis of the literature indicates that the combined use of PBM with NO donors, such as S-Nitrosothiols or alkyl nitrites, including NO itself, induces a potent synergetic effect.

Moreover, since interaction between $H_2S$ and NO can produce nitroxyl (HNO), which plays an effective role within the cardiovascular system about oxidative stress and cardioprotection, heart contractility, vascular tone as well as angiogenesis [Nagpure, 2016; Wu 2018], the coadministration of (H)NO as well as nitroxyl donor, cimlanod or 1-Nitrosocyclo Hexyl Acetate for instance, is of high interest to increase PBM effects.

Ebselen, an FDA approved $H_2Se$ donor is of high interest to increases PBM effects as already discuss by the inventor (page 13, point g).

As already mention, NAD+ is required for redox reactions and control hundreds of key process of energy metabolism to cell survival, rising and falling depending on food uptake, exercise, and time of the days. Therefore, administration of NAD+ donor, as vitamin B3 within PBM is of high of interest to increase PBM effect.

Other exogenous agents of interest for their combined use with PBM are:

- Curcumin, a major active component of turmeric (Curcuma longa, L.), is known to have various effects on both healthy and cancerous tissues. Notably, curcumin induces ER stress, thereby causing an unfolded protein response, the major retrograde signaling, and calcium release, which destabilizes the mitochondrial compartment and induce apoptosis.
- Dexmedetomidine, a well-known α2 agonist agent used in anesthesia, has gained of interest recently since it is suggested that dexmedetomidine preconditioning mitigates myocardial ischemia/reperfusion injury *via* inhibition of mast cell degranulation. Similarly, EPO have shown positive effect in ischemia reperfusion injuries into the kidney as well as lipopolysaccharide in arterial vascular damages.
- Ivermectin are approved by the FDA to treat people with intestinal strongyloidiasis and onchocerciasis, two conditions caused by parasitic worms. Moreover, clinical evidence supports the use of ivermectin in decreasing mortality in patient with SARS-CoV2 infection. The combination of ivermectin and PBM can reduce the dose of ivermectin, in particular to reduce side effects.
- Viagra as a source of nitrite in various forms (as alkyl nitrites)

By extension, any exogenous agents which are known to modulate the metabolism, especially within the mitochondria through the modulation of the ETC or ROS modulator for instance, are of high interest to increase PBM effects. It is the case for adenosine diphosphate (ADP) which is known to increase the OCR, or for vitamin K, ketamine suxamethonium, acetylcholine and atropine, as well as bradykinin. Additional agents include, catecholamines like adrenaline noradrenaline or dopamine, opioids which activate various G proteins, or various kinases modulator or various anti-oxidant and/or anti-inflammatory donor such as resveratrol. Finally, targets of the rapamycin or sirtuin modulators are of high of interest to increase PBM effects.

By extension, since temperature, exogenous or endogenous mechanical pressure [Li, 2005; Hwang 2003], physical exercise as well as electrostimulation, hyperoxia, hemostasis (remote preconditioning) are known to modulate the metabolism, any exogenous stimulus, or combination of, like environmental/physical /electrical or electromagnetic stimulus applied on the biological object is of high interest to increase PBM effect. For instance, it is known that the level of endogenous $H_2S$ is inversely correlated within the temperature. An increase of the temperature in situ can be viewed as an indirect endogenous $H_2S$ donor and, reciprocally, an increase of the in situ temperature can be viewed as an endogenous

$H_2S$ inhibitor.

As observed by the inventors, the potency of PBM not only depends on the light dose [$J/m^2$] and spectroscopy (wavelengths) but, surprisingly, also on the fluence rate for specific illumination times. For example, the inventors have observed for a specific case, as indicated in figure 4a and 4b, that cells must be illuminated during 3 minutes with an irradiance (which is equal to the fluence rate in this specific setting consisting of cell cultures) of 3 mW/$cm^2$ (i.e. a dose of 0.54 J/$cm^2$). Different illumination times and/or irradiances do not induce any PBM effects, excepting "hot spots" observed at, for instance, an irradiance of 15 mW/$cm^2$ applied for 40 seconds, or 25 mW/$cm^2$ applied during 22 seconds. Interestingly some of these hot spots are wavelength independent, as one can conclude comparing figures 4a and 4b where hot spots are present in the same place in terms of irradiance and illumination time.

This is an illustration of a more complex PBM response compare to the well-known bimodal effects of PBM, i.e. too high or too low fluence rates and/or light doses significantly reduce the PBM effects. The inventors have also demonstrated, in certain conditions, the absence of "neutralization" of the PBM effects by an over-dose/irradiance before and/or after PBM applied with optimal conditions.

The inventors have also observed surprising results when performing PBM with a combination of wavelengths, one of them being ineffective when used alone. This is the case for 730 nm which is not potent when used alone, as it can be seen in the figure 4c. It can be seen from this figure that the wavelength of 689 nm, which is effective for an illumination time of 180 s when applied with an irradiance of 3 mW/$cm^2$ is ineffective if it is applied with 9 mW/$cm^2$. Surprisingly, combining synchronously or sequentially illuminations at 730 nm (with an irradiance of 3 mW/$cm^2$ for 180 s) and 689 nm (with an irradiance of 9 mW/$cm^2$ for 180 s), resulted in a marked PBM effect, evidenced by the PpIX fluorescence intensity ratio (PBM/no PBM). These effects were comparable to those corresponding to the "hot spots" presented in figure 4a and 4b. Therefore, the synchronous or sequential combination of wavelengths in such a way that at least one of them is not (or poorly) potent is of high of interest to increase PBM effect, in particular when specific hot spots of the potent wavelength are difficult to reach due to optical or geometric constraints.

Indeed, it is well established that the optical properties of biological tissues, described mostly by their absorption $\mu_a$ and reduced scattering $\mu_s$' coefficients, have an important impact on the propagation of the light around a light distributor. In general, the fluence rate (and the light dose) decreases with the distance from the light source due to the absorption and scattering of the light in the tissue for a given power (and illumination time). Figure 5 illustrates how the fluence rate F (normalized by the irradiance E) decreases with the distance from the surface on the specific case of a "broad" (much larger than $\mu_{eff}^{-1}$), collimated illumination of a semi-infinite sample. Therefore, the fluence rate and the light dose in the tissues are never optimal at the same time in different locations of the tissues treated by PBM.

It should be noted that the geometry of the light distributor (illumination geometry) is adapted to the specific organs to be treated. For example, frontal (broad field), balloon-based, or interstitial illuminations, with one or several fibers, are considered, in particular (see the products commercialized by Medlight SA "http://www.medlight.com/#" as illustrative examples).

The inventors have also established an innovation to adjust the radiometric and spectral conditions used in PBMT based on frequency analysis (in particular using the wavelet theory) of parameters reflecting the metabolic activity. More precisely, they have conducted a time frequency analysis of the partial pressure of oxygen (p$O_2$) of the chick's embryo chorioallantoic membrane (CAM) during PBM.

It is well known that arterioles, particularly in the peripheral microcirculation, strongly respond to the surrounding tissue p$O_2$ [Jackson, 2016] through complex metabolic regulation mechanisms [Reglin, 2014] where low frequency oscillations of the blood perfusion exist [Kvandal, 2006]. Based on local measurements of the p$O_2$ performed in the CAM during a "long" (several hours) time using commercially available Clark's probes (Unisense®, OX-needle, OX100-Fast) we calculated frequency spectra resulting from a wavelet-based analysis of the p$O_2$. Wavelet analysis is a well-known mathematical transform which enables to characterize nonstationary frequencies during the measurement time. Figure 6 (middle left) shows a typical measurement of the p$O_2$ close to a new CAM arteriole at embryo development day (EDD) 7. Here, the time signal (measurement time: ~180 s) mostly results from the superposition of 2 frequencies (see figure 6, lower left; the vertical axis is the frequency): the heartbeat (~1 Hz) and the myogenic tone (~0,1 Hz) that represents the intrinsic activity of the vascular smooth muscle.

$H_2S$ is a potent regulator of the vascular tone [Köhn, 2012] which can be induced by the administration of NaSH. We measured with our Clark's probe that a $H_2S$ stimulation of a CAM arteriole induced by the topical application of NaSH (10 μl, 1 μM in physiologic serum) generates a strong modulation of the p$O_2$ around 60 mmHg. This modulation is observed at least for the myogenic (0,05Hz - 0,15Hz), the endothelial nitric oxide synthase dependent (0,01 Hz - 0,02 Hz) and the endothelial nitric oxide synthase independent (0,005 Hz - 0,01 Hz) bands. Other lower frequencies bands are also activated where it was notably suggesting that some of them are correlated within prostaglandin or prostacyclin release from the endothelium.

Our innovation results from PBM irradiations of the CAM we have performed with a frontal light distributor (850 nm, 7 mW.$cm^{-2}$, 30 s) at t=80 min and t = 105 min (see figures 7). A modulation ("dimming") of these frequencies (figure 6 (top right) and figure 7) is clearly induced by PBM.

It appears, in particular, that the bands 3, 5 and 6, as well as those corresponding to undefined lower frequencies, are fully or partially inhibited by PBM (see figure 6). Inhibition of band 3 (the myogenic one) indicates that, since this band is due to arteriolar smooth muscle cells via activation of NADPH oxidase (NOX) and subsequent ROS generation [Nowicki, 2001; Li, 2017], PBM inhibits the NOX activity. Since the NOX superfamily plays a fundamental role in inflammatory and immune responses where notably NOX are implicated in the metabolic switch of leucocytes activation our observations reveal an important pathway of PBM to modulate inflammations and be potent as an important immunomodulator. Moreover, modulate the myogenic tone of the biological object by PBM can prevent numerous hypertension injuries as it is the case for instance in renal pathologies [Loutzenhiser, 2002; Moss, 2016]. Altogether, our results indicate that a frequency analysis of a parameter reflecting the metabolic activity, such as the $pO_2$, enables to monitor the light dose and/or the fluence rate used for PBM. It should be noted that the oscillations observed in figures 6 and 7 were induced by $H_2S$ for illustrative purpose, i.e. to increase the signal to noise ratio. Indeed, such oscillations are present even in the absence of $H_2S$.

Therefore, adapting the radiometric and spectral conditions used in PBM therapy based on the frequency analysis of parameters reflecting the metabolic activity is of high interest.

This specific type of monitoring can be performed for two main purposes: i) to apply the PBM light at an optimal time relative to the metabolic "oscillations" or, ii) to assess the level of change of the metabolism induced by PBM in such a way that it is optimal (to adapt the radiometry). The $pO_2$, as presented just above, is not the only parameter to be analyzed using the wavelets, or frequency-based analysis to monitor the light dosimetry during PBM. The list presented above (List of feedback observables) describes other parameters of interest:

The inventors have also shown that application(s) time of light irradiation within the biological object is crucial to induce significant PBM effects. These observations are very important since biological objects are dynamic within a wide frequency scale of metabolic activity triggered from transient or regular endogenous or exogenous factors. Notably, the inventors have shown that, when light is applied at a specific time during the metabolic activity of glioma cells or HCM, the metabolic response of cells is significatively modulate differently which modulate accordingly phenotypical long-time response. Inventors have also shown, using the in-ovo chicken embryo heart models during anoxia / reoxygenation studies, that the survival rate is significantly higher when the PBM irradiations start just before the reoxygenation, compare to when PBM is performed during ischemia long time before or long time after the reoxygenation. Interestingly, it is observed in this condition, that reoxygenation induces an arrest of the heart beating during a time ranging between one second and several minutes. PBM conditioning prior to reoxygenation significatively avoid this arrest. Therefore, the inventors have shown that PBM restarts or modulates the heart beat following an anoxic cardioplegia or presenting bradychardia or tachycardia, whereas no influence is observed on healthy beating hearts. These *in ovo* observations have been confirmed in *vivo* by the inventors during ischemia/reperfusion events induced by the ligation of swine hearts coronary.

The inventors have also shown that PBM can be used for the conditioning of the heart chicken embryo during hypoxia reoxygenation events.

One aspect of the present invention is the application of the device or method to treat ischemia reperfusion injury, in particular those affecting the myocardium to reduce the infarction size following acute myocardium infarction (MI).

Based on the chicken embryo heart the inventors developed an anoxia reoxygenation experiment in ovo where eggs were placed in a thermoregulated gas chamber with a continuous monitoring of the environmental and embryonic temperature and $pO_2$. For some experiments, small $H_2S$, NO and pH probes were also positioned around the embryonic heart or at different location of the embryonic tissue. This chamber was placed under a microscope for image recording. After a stabilization time (temperature stabilization), an anoxic environment was created by flushing nitrogen all around the egg during tens of minutes without any change of the environmental temperature, followed by a reoxygenation of the egg as depicted on the figure 8.

At Embryonic Development Day (EDD) 3, flushing pure $N_2$ during 45 min before a reoxygenating of the embryos induced a mortality rate larger than 50 % 48h after the end of the experiment. This experiment supports one aspect of the "reperfusion injury" mentioned as the "oxygen paradox" in the article published by Latham et al. (Latham, 1951), i.e. that reperfusions could be, in certain cases, lethal (Piper, 2000). In our case, as well for isolated chicken heart embryo (Raddatz, 2010), reoxygenation induce a burst of Reactive Oxygen Species (ROS) and a permanent or transient cardioplegia followed by irregular heartbeat (bradycardia, tachycardia). In our experiment, when embryo at EDD3 undergo a 45 min anoxia, we observed that a photoconditioning (671 or 808 nm, 5 mW.cm$^2$, 30 s) of the embryo just before the reperfusion significantly avoid cardioplegia and, importantly, increase the survival rate at 48 h. Interestingly, this positive effect of PBM is not observed if light is applied too early or too late after the reoxygenation. This last observation clearly suggests that the time at which the light is applied relative to the reoxygenation is critical to produce a beneficial outcome.

Therefore, one application of high interest for the invention consists to use PBM delivered by our original medical device and method to treat damages resulting from hypoxia reoxygenation events and by extension for ischemia reperfusion events.

The inventors have also shown that the heart beat can be stimulated by PBM after an anoxic non-permanent cardioplegia of the chicken embryo heart.

Before Embryonic Development Day (EDD) 7, oxygen supply of the chick's embryo was mainly performed by diffusion across the shell, then through the embryo. Heart beat and blood flow, which are observable from EDD 2, mainly act as stimulus for cardiovascular development. Embryo, up to day 5 are flattened on the "surface" located just below an albumin layer. Therefore, it is easily accessible after removing a part of the shell. This is why, in parallel to the chicken embryo ontogeny, embryo from EDD 2 up to EDD 5 are used since decades as excellent models for developmental biology and, in particular, in cardiogenesis and rythmogenesis. This model is also used for anoxia-reoxygenation studies [Sedmera, 2002] where their behaviors are studied during and following hypoxia or anoxia, but also during hypoxic induced tachycardia, bradycardia or for fibrillation studies.

One interesting PBM effects observed by the inventors during an anoxic in ovo experiment is in relation with the positive effect(s) of light which enable to restart the heard after a cardioplegia. Indeed, a prolonged anoxia leads to a stop of the heart beat which, sometimes, restarts to beat again transiently until an irreversible and total cardioplegia takes place. The inventors observed, in most cases, that a PBM irradiation often restart the beating heart (Figure 9) after such a cardioplegia in parallel to the resuscitation observations of ischemic swine. Moreover, based on frequency and phase shift analysis of sub-compartments of embryonic heart (Atrium, ventricle, outflow tract) beats, PBM illumination stimulated the recovery of this frequency (especially harmonics) as well as the phase shift of contraction between sub-compartments after hypoxia. This is of particular interest since this phase shift must be preserved to maintain the efficacy of the pump functionality (cardiac output). Therefore, through the monitoring of the frequency and the phase shift of the heart sub-compartments movements, a local illumination of the sub-compartment, notably atrium, which is known, as shown by the inventor, to be very sensitive to hypoxia, is of high of interest to maintain the cardiac output.

This surprising positive effect of PBM strongly suggests that it triggers the metabolic activities involved in the heart beat, including after an anoxic cardioplegia. Since PBM is known to reduce inflammations and to boost the metabolic activity, PBM is of high interest to treat conditions such as fibrillations, including atrial fibrillations, for instance

By extension, since the metabolism is subject to autonomous (i.e. independent of the cell cycle [Papagiannakis, 2017]) and non-autonomous rhythms of various frequencies, as it is the case, for instance, for the circadian rhythm [Bailey, 2014], applying PBM light at specific times and/or frequencies to lock, trig and/or (re)synchronize metabolic oscillations is of high of interest, in particular to treat various metabolic disorders, such as type 2 diabetes [Petrenko, 2020], metabolic switch as aerobic glycolysis (Warbugg effect) in cancer cells [Gatenby, 2018], or within hepatic disorders and diseases [Zhong, 2018].

The inventors have also shown that PBM light delivered directly in the blood perfusing large vessels (pulmonary artery, vena cava of pigs), or in the right atrium, which contain deoxygenated blood, can be used to modulate systemic hemodynamics and oxygen tension, generate anti-inflammatory, immunomodulatory, anti-aggregation, endothelial and epithelial cells protection. Surprisingly, such illuminations of deoxygenated blood during long hypoxia event in swine maintain homeostasis according to gas measurements performed in arterial and venous blood (using cobas b 123 POC System Roche diagnostics®). In addition, these illuminations maintained and stabilized functional hemodynamic variables, such as the cardiac output, concomitantly to an increase and a stabilization of the systemic labile NO level, as measured within a heparinated NO probe (NO-NP Unisense® placed into pulmonary arteries or the atrium during tens of minutes after the PBM Illumination. This effect is unexpected since it is usually well accepted that the lifetime of labile NO in blood is ten to hundred times shorter. Interestingly, without a concomitant increase of methhemoglobin assess in venous or arterial gas during the experiment. In addition, the inventors have shown that PBM light delivered directly in the blood perfusing large vessels (pulmonary artery), or in the right atrium, which contain deoxygenated blood, can be used to control hypoxemia, hemoglobin saturation, arterial and venous oxygen partial pressure associated to a hypoxia. In addition, this approach can be used to maintain the glycaemia level (figure 25a), in order, notably, to reduce the probability to induce systemic tissular damage as it is the case for multiple organ failure (MOF) resulting from glycaemia deregulation.

**Detailed description of the invention:**

According to the results presented in Figure 4a and 4b that shows PBM effects on the ability of HCM cells to produce PpIX, a fluence rate (or intensity) of 3 mW/cm² must be applied during 3 minutes to maximize this effect. Since the fluence rate is not uniform in bulk (or 3D) tissues for a fixed irradiance, as illustrated in figure 5 for a particular geometry and specific optical coefficients, the medical device according to the invention preferably delivers an irradiance which changes with time in such a way that all cells receive the optimal fluence rate during 3 minutes. In most situations, the illumination geometry is not changed during the illumination. Therefore, the irradiance is simply given by the power [W] illuminating the tissues divided by the surface [m²] of the illumination spot. Although Yaroslavsky et al. [reference] already described similar concepts, one finding of the inventors mentioned in the present document is to identify specific values of the fluence rate and illumination times which must be both applied at the same time.

Let's consider the specific situation corresponding to the geometry and the optical coefficients mentioned in figure 5. Since the fluence rate F can be determined by the solution of the diffusion approximation (n.b.: the hypothesis that are at the basis of this diffusion approximation must be fulfilled, i.e.: i) $\mu_a \ll \mu_s'$; and ii) we are looking at the fluence rate at locations "z" which are "far" (i.e. $z > 1/\mu_s'$) from the light source(s) and boundaries; "k" is a factor resulting from the light backscattered by the tissue which increases the fluence rate under the interface, as described by Jacques [Jacques, 2010])

$$F = k.E.e^{-\mu_{eff} z} \qquad \text{Equation 1)}$$

, the only way to maintain F constant, called F' thereafter, for increasing values of z is to increase E. This statement derives from the inversion of the previous expression which writes:

$$E = (F'/k). e^{\mu_{eff} z} \qquad \text{Equation 2)}$$

Since each HCM cell must be illuminated during 3 minutes with F' = 3 mW/cm$^2$, another concept of important has to do with the tolerance affecting this fluence rate. If cells must be irradiated with exactly 3 mW/cm$^2$ the total treatment of the whole sample would take an infinite time since the volume corresponding to these cells is equal to 0 mm$^3$ (they are confined in a plane located at depth "z" which has a volume equal to 0 mm$^3$). However, looking at figure 4a indicates that the irradiance full width half maximum (FWHM) of the "peak" located at 3 mW/cm$^2$ and 180 s is about 3.2 mW/cm$^2$, i.e. the fluence rate F' must range within $3 \pm 1.6$ mW/cm$^2$ (written F' $\pm \Delta$F' thereafter) to induce optimal PBM effects after 180 s. It means that the HCM cells in which an optimal PBM effect is induced are not in a plane but in a slice of thickness $\Delta z$. The thickness $\Delta z$ of this slice is given by:

$$\Delta z = \Delta F'/(dF/dz) = \Delta F'/ k.E. \mu_{eff} .e^{-\mu_{eff} z}. \qquad \text{Equation 3)}$$

Since $E = (F'/k). e^{\mu_{eff} z}$, we have

$$\Delta z = \Delta F'/\mu_{eff}.F'. \qquad \text{Equation 4)}$$

Therefore, $\Delta z$ only depends on F', $\Delta$F' and $\mu_{eff}$.

If the tissue volume to be treated ranges between $z_1$(proximal position) and $z_2$ (distal position), the number "n" of different irradiances to apply during 180 s (thereafter called T) is equal to: $z_2 - z_1$ /$\Delta z$.

Consequently, the spatial evolution of the irradiance E is as presented in figure 10.

The temporal evolution of the irradiance E(t) is (see figure 11):

$$E(t) = (F'/k). e^{\mu_{eff}. \Delta z.t/T} = (F'/k). e^{\Delta F'.t/F'.T} \qquad \text{Equation 5)}$$

where E will be equal (providing that the diffusion approximation equation is valid) to F'/k.e$^{\Delta F'/2F'}$ when 0<t<T, F'/k.e$^{3\Delta F'/2F'}$ when T<t<2T, F'/k.e$^{5\Delta F'/2F'}$ when 2T<t<3T, F'/k.e$^{(2n+1)\Delta F'/2F'}$ when nT<t<(n+1)T, with n = $\mu_{eff}$.F'.($z_2 - z_1$)/ $\Delta$F' (see equation 6 below).

Therefore, the total time "$t_{tot}$" it takes to treat a volume of tissue ranging between $z_1$ and $z_2$ is: T.($z_2 - z_1$)/ $\Delta z$. With the explicit expression of $\Delta z$ (Equation 4) we have:

$$t_{tot} = T(z_2 - z_1)/ (\Delta F'/\mu_{eff}.F') = T.\mu_{eff}.F'.(z_2 - z_1)/ \Delta F'. \qquad \text{Equation 6)}$$

Finally, it should be noted that, similarly to F', T can be applied with a certain tolerance due to the FWHM of the PBM peak along the illumination time axis (see figure 4a).

In summary, in this example the device according to the invention applies an irradiance E(t), during a time which ranges between 0 and $t_{tot}$, given by: $E(t) = (F'/k). e^{\Delta F'.t/F'.T}$.

Since the device illuminates a certain area of surface S [m$^2$] with a certain power P [W], we have that E(t) = P(t)/S.

Therefore, the device delivers an optical power P(t), during a time which ranges between 0 and $t_{tot}$, given by:

$$P(t) = (S.F'/k). e^{\Delta F'.t/F'.T}. \qquad \text{Equation 7)}$$

All parameters involved in the expressions of E(t) and $t_{tot}$ are determined for a specific organ (of known thickness $z_2 - z_1$) and illumination geometry (of surface S): Indeed, F', ΔF' and T are derived from the figure 4a (for HCM), whereas k is derived from the tissue optical coefficients (which are known for the tissue of interest).

This detailed description, for an optimal PBM effect on the whole volume addressing a specific hot spot of figures 4a and 4b can be generalized to "hot spot line" or "hot spot surfaces" $\Omega_{i\lambda}$, where i represents a specific hot surface on the map presented in figure 4d at the wavelength λ. Since, the points presented in figures 4a and 4b represent specific coordinates (fluence rate; illumination time) and since points around the maximum of hot spots can have a reasonable efficacy, hot spots can be described by an area defined by a rectangle $\Omega_{i\lambda}$ ($\Delta F'_{i\lambda}$; $\Delta T_{i\lambda}$): the dimensions of the hot spot surface $\Omega_{i\lambda}$), with a typical potency equal to the mean or the barycenter of the points inside $\Omega_{i\lambda}$, as represented in figure 4d. The selection of illumination conditions corresponding to specific $\Omega_{i\lambda}$, which have their specific dimensions $\Delta F'_{i\lambda}$ and $\Delta T_{i\lambda}$, can be defined in order to minimize the total treatment time as described in more details below, within a concomitant reduction of the expected PBM effect. Then function of the treatment case, acute, chronic, severe, moderate, and the geometry of the treated area, a minimization cost algorithm can define the best hot spots (point, line or surface) one the basis of parameter define by the users notably by defining the maximum treatment time value as well as the minimal level of efficacy expected. For instance, in order to treat a superficial wound, optimally, a hot spot point can be used, where in this case, time of treatment will not be to long since the small depth of the wound. However, in case of acute infarction, time is critical and the treated volume is relatively important, then the hot spot point can be translated to an hot spot surface with relatively higher $\Delta F'_{i\lambda}$ and or $\Delta T_{i\lambda}$, in order to treat as much as possible the total volume in a minimum time. It should to be noted that most of the hot spots are located in the dose range of $0.2 J.cm^{-2}$ to $1 J.cm^{-2}$ but others hotspots may exist in the illumination time range of seconds within corresponding fluence rates of hundreds of mW per $cm^{-2}$.

In the description given above, the tissue is considered to be static while the power of the light source is changed with time to generate optimal fluence rate during an optimal time in the targeted tissues. However, there are situations, for example in fluids, including the blood, where the geometry is dynamic, due to the blood flow for example. In such situations, the power delivered by the light distributor can be stable (no time evolution), but the light pattern produced by the light distributor, combined with the fluid optical properties, can be such that the fluence rate is optimal in some volume elements due to the fluid flow. Therefore, longitudinal variation of the emittance in such a way that the light dose and/or fluence rate is optimal to induce PBM effects at different locations of a moving fluid, such as blood must be introducing. An illustrative example is presented in figure 19a where a non-uniform longitudinal light distributor is positioned at the center of a blood vessel. Since the light distributor emittance ($W/cm^2$) increases along its illuminating window, blood volume elements located close to the surface of the distributor will get an appropriated irradiance on the left of the image, whereas blood volume elements located far from this surface will get an appropriated irradiance on the right of the image. Therefore, the whole blood volume will receive an optimal dosimetry since it is moving along the cylindrical light distributor.

Based on the surprising results obtained by the inventors indicating that the effects resulting from the use of PBM-potent wavelengths applied in sub-optimal radiometric conditions can be optimize by the combined application of a non-potent wavelength, another PBMT protocol can be defined. An illustrative example (Figure 19b) involves PBMT illuminations where both potent and not potent wavelengths are delivered successively. It also can be delivered at the same location of the distributor. The optimal optical longitudinal profile depends on many parameters, including the vessel diameter (geometry), the blood flow, its regime (laminal, turbulent, pulsatile aspect, ...), and the blood optical properties. The device can easily be produced, with minor changes of the processes used to realize uniform cylindrical light distributor, or by designing specific balloon catheter shape/size, as presented in figure 19c. The use (inflation, illumination, deflation) of the balloon can be static or dynamic, as it is the case for counter pulsatile balloons used in the aorta, which are inflated or deflated synchronously with the heart beat during weeks. The time of flight (time during which the object is around the stick) can also be modulated to optimize the PBM treatment. This could be done on the basis of the modulation of the cardiac output (with NG-monomethyl-L-arginine (L-NMMA) for instance, or by the level of inflation or rhythm of inflation/deflation of a balloon-based catheter surrounding the light distributor. Obviously, this concept can also be applied on extracorporeal circulation or circulatory assistance devices.

Example 1: Treatment of ischemia-reperfusion of heart muscle

A detailed example of the device according to the invention is presented in this example.

This treatment of ischemia-reperfusion of heart muscle can be performed:

1. During acute or chronic myocardial infarction (MI).
2. During aortic clamping and cardioplegia induced by post extracorporeal circulation.
3. During organ transplants (heart, lung or other).

The optical distribution routes considered are:

1. Interstitial (trans-myocardial)
2. Endocavitary (endoventricular)
3. Endovascular (endocoronary)

**Trans-myocardial medical device**

This involves implanting light distributors, preferably cylindrical and based on one or more optical fibers, through the heart (Figure 12 and 13 and 14b) by a cardiac surgeon during myocardial revascularization consecutive to the acute phase of a MI or following an aortic clamping of more than 120 minutes under extracorporeal circulation, or during a heart transplant.

These light distributors are placed in the suffering cardiac area (ischemic area for example) at the end of the surgical procedure before or during reperfusing the coronary arteries.

These light distributors are placed according to the procedure described below:

1) Localization and estimation of the area to be treated by:

a. Macroscopic assessment of the area suffering (Figure 14a) (visual indicators: edema, akinesia, dyskinesia, vermilion color chromatic appearance and / or use of characterization apparatus)

b. Correlation to the coronarography and ultrasound data corresponding to the ischemic myocardial anatomical area (for example for the left ventricle):

i. Anterior territory
ii. Lateral territory
iii. Inferior territory

2) Determination of the number of light distributors and their relative location necessary for optical delivery on the basis of the extension and accessibility of the ischemic area:

a. In situ consideration of the distribution of the coronary arteries to avoid transfixing them

b. The light distributors are transfixing and implanted at angles ranging between horizontal to normal to the surface, in the thickness of the myocardium (according to ultrasound data in time-movement mode) both to maximize the volume to be treated by distributors and allow their fixation as close as possible to the epicardium

c. The light distributors have a length (5cm) greater than their maximum length in the myocardium to distribute the light throughout the myocardium thickness.

3) Placement and fixation of a semi-rigid and transparent silicone-type or biodegradable mask by 4 points (single-strand 5.0 wire) on the epicardial surface of the heart to be treated. This mask serves both as a guide / template for the transfixion (pre-drilled at the correct implantation angles and whose holes follow geometric patterns and predefined spacings taking into account the propagation of light in the tissue depending on the light wavelength(s) used). This mask is also used to maintain the transfixion catheters into which the light distributors are inserted.

4) Transfixion of the myocardial wall through the mask with, for instance, a iCAT® type catheters whose characteristics are:

a. less than 20 gauges (to minimize hemorrhages and tissue damage),
b. hollow,
c. transparent at the used wavelengths,
d. closed end cap,
e. visual markings on the catheter indicating the length of catheter placement.

5) Connection to the light distributor device followed by an optical calibration step

6) Introduction of a light distributor into each catheter (similar to the interstitial procedure used in TOOKAD®-type photodynamic therapy). Attaching the distributors to the catheter via a "luer lock". Attachment of all light distributors to the mask and / or to the patient's skin.

7) Determination of the temporal evolution of the light power emitted by the light distributors so that the treated cells receive, for an optimal time, a spatial irradiance ("Fluence rate") which is also optimal. This determination is made on the basis of the number and positioning of the light distributors determined by the user. It consists, for example, in deriving the temporal evolution of the light power emitted by the light distributors from the preset weighting matrix. These matrices are generated on the basis of a Monte-Carlo type simulation of the propagation of light in the tissues of interest and cost reduction algorithms taking into account the specificities (optical and biological) of each wavelength used to optimize processing time.

Optical delivery in such a way that the time evolution of the light power of the light source is done according to the determination described in the previous step. It can be modulated by monitoring methods based on various instrumental or biological data previously described. A simplified diagram illustrating an example of a part of the device supplying an optical distributor is presented in figure 15.

In the case of a single irradiation:

    i. Surgical reperfusion procedure
    ii. Removal of catheters and mask
    iii. Hemostasis if necessary, at transfixion points
    iv. Validation of the calibration of light distributors
    v. Continuation of the surgical intervention as usual.
    vi. Normal closing procedure as for any sternotomy.

In the case of multiple irradiations:

    i. Surgical reperfusion procedure
    ii. The catheters and light distributors are let in place and attached to the biodegradable mask. The light distributors can be tunneled to the skin, fixed and let in place for 8 to 10 days in order to repeat the procedure remotely.
    iii. Continuation of the surgical intervention as usual.
    iv. Normal closing procedure as for any sternotomy.
    v. The removal of the light distributors is done with chest drain in place, by a simple manual removal with an ultrasound check at the second hour.

**Endoventricular medical device**

This involves the placement of one or more light distributors percutaneously into the left ventricle by an interventional cardiologist under fluoroscopy during myocardial revascularization in the acute phase of a MI in pre, per or post conditioning.

These light distributors are placed at the start of the procedure before revascularizing the occluded coronary artery(ies).

These light distributors are implanted according to the procedure described below:

    1) Access to the radial or femoral artery by ultrasound puncture.
    2) Introduction of a Radiofocus-type guiding sheath (5F-6F), using the Seldinger method.
    3) Under fluoroscopy, navigation with a Radiofocus-type diagnostic guide catheter to the left ventricle after crossing the aortic valve. Removal of the guide and placement, via the diagnostic catheter of the light distributor with or without a self-inflating systolo-diastolic balloon in the left ventricle, or directly fixed in the wall of the ischemic left ventricle.
    4) Optical delivery. The determination of the temporal evolution of the light power emitted by the light distributors and the optical delivery is performed as described above.
    5) Removal of the catheter, light distributors and sheath; possibility to let the device in place for 8 to 10 days.
    6) Usual procedure for reperfusion by interventional radiology of the occluded coronary arteries.

**Intravascular medical device**

This involves the placement of one or more light distributors percutaneously in the arteries, whatever they may be, under fluoroscopy during a revascularization process after an ischemic phenomenon in interventional radiology, in pre, per or post conditioning, during a MI, a lung or other organ transplantations submitted to ischemia reperfusion phenomena.

These light distributors are placed at the beginning or at the end of the procedure before reperfusion.

These light distributors, in the case of the coronary arteries, are implanted according to the procedure described below:

    1) Radial or femoral arterial access with I.V catheter (Surflo® type).
    2) Introduction of a Radiofocus-type sheath (5F-6F), using the Seldinger method.
    3) Under fluoroscopy, navigation with a diagnostic catheter (4F, type JR4,) and a 0.035' guide (Radiofocus type), up to the coronary artery.

4) Removal of the guide and placement, via the diagnostic catheter, of the light distributor.
5) Optical delivery. The determination of the temporal evolution of the light power emitted by the light distributors and the optical delivery are performed as described above.
6) Removal of the catheter, light distributors and sheath.
7) Procedure for reperfusion of the coronary artery as usual.

Translation of these procedures can be performed to heart transplant since it is known that ischemic reperfusion injuries is a major issue during organ transplant, this issue being the main cause of graft rejections.

By extension, the procedures described above can also be applied to other organs subject to ischemia reperfusion injury, as it is the case for kidney, liver, spleen, or brain for instance. These procedures can be combined with other procedures in order to illuminate simultaneously different part of the body, the thyroid for instance, to control a possible negative systemic response induce by the organ subject to I/R.

Example 2 : continuous temporal change of the irradiance (or power) emitted by the light source.

Since $\Delta F'$, defined in the detailed description, is larger than zero, the temporal evolution of the irradiance (or power) delivered by the light source can be continuous instead of incremental (as presented by the dotted lines fitting the histograms in figure 10 and 11). In this case, the temporal evolutions of the irradiance or the power delivered by the light distributor(s) is given by equations 5 and 7, respectively.

Example 3: temporal decrease, instead of an increase, of the irradiance (or power) emitted by the light source.

The different tissue layers of thickness $\Delta z$ can be illuminated with the appropriated fluence rate while increasing or decreasing (incrementally and/or continuously) the irradiance (or the power).

In this case, $t_{tot}$ is the same, but the temporal evolution of the irradiance (or the power), are given by the expression $E(t) = (F'/k).e^{\Delta F'.(t_{tot}-t)/F'.T}$ or $P(t) = (S.F'/k).e^{\Delta F'.(t_{tot}-t)/F'.T}$, respectively ($0 < t < t_{tot}$).

Example 4: different illumination (light delivery) geometries.

Figure 5 shows the spatial distribution of the fluence rate for a specific geometry, namely in a semi-infinite tissue illuminated with a broad, collimated and perpendicular light beam at the air-tissue interface. It is well known in the field of photomedicine, in particular in photobiomodulation or LLLT, that different illumination geometries are considered depending on the tissue/organ structure(s) and access. Figure 6 illustrates some of the most common illumination geometries. Solutions of the diffusion approximation exist for many of these geometries to determine the fluence rate. Therefore, a general expression of equations 5 for other illuminations, organs and/or light delivery geometries can be written as: $E(t) = \mathbf{F}_E$ ($\mu_a$, $\mu_s$, g, $n_{ext}$, $n_{tissue}$, F', $\Delta F'$, S, T, t), where $\mathbf{F}_E$ is a function which depends on the tissue optical parameters, the organ and illumination geometries, the fluence rate, as well as its FWHM, and the illumination time(s) generating local maxima of the PBM effects. Numerous different approaches are known to determine $\mathbf{F}_E$, as described below in example 6.

Similarly, a general expression of equations 7 for other illuminations, organs and/or light delivery geometries can be written as: $P(t) = \mathbf{F}_P$ ($\mu_a$, $\mu_s$, g, $n_{ext}$, $n_{tissue}$, F', $\Delta F'$, S, T, t), where $\mathbf{F}_P$ is a function which depends on the tissue optical parameters, the organ and illumination geometries, the fluence rate, as well as its FWHM, and the illumination time(s) generating local maxima of the PBM effects. Numerous different approaches are known to determine $\mathbf{F}_P$, as described below in example 6.

A combination of the light delivery geometries presented in figure 16 may also be used. Obviously, use of optical sources, as LED or VCSEL *ex situ* or *in situ* for instance, in direct contact or in quasi contact must also be envisaged.

Finally, heterogeneous tissues, in particular layered tissues structures, must also be envisaged.

Example 5: different tissue optical properties.

Since different types of tissues have different optical properties, the formalism described above is valid for different values of $\mu_a$, $\mu_s$, g, $n_{ext}$, $n_{tissue}$, in particular if these optical properties are subject to changes for a given tissue during the illumination.

Example 6: Assessment of the fluence rate using other approaches but those based on the diffusion approximation.

Different approaches are well established to model the propagation of light in biological tissues [Martelli, 2009]. Therefore, these approaches can be used instead of, or in combination with, the formalism presented above, which is based on the diffusion approximation of the light transport equation, to determine the temporal evolution of the light source to generate optimal PBM effects. These approaches, which have been mostly developed in photomedicine to master the dosimetry of light in tissues, are classified in two categories:

1) The analytical approaches: Excepting the well-known diffusion approximation of the light transport theory which was used to establish the formalism presented above in the detailed description of the invention, other analytical approaches are well established in this field, including but not limited to: The Kubelka-Munk theory, delta-Eddington radiative transfer equation, ...

2) The computer-based approaches: numerous computer-based approaches have been proposed since decades to simulate the propagation of light in biological tissues. These approaches include, but are not limited to: Monte-Carlo simulations, Finite elements simulations, ...

Example 7: The taking into account of the different tissue responses to PBM depending on the cell types, wavelengths (spectral design) and metabolic activities.

The specific values given above for F'(3 mW/cm$^2$), $\Delta$F'(1.6 mW/cm$^2$) and T (180 s) result from our experimental observations obtained in specific conditions in terms of sample (human cardiomyocytes: HCM), environment (medium, temperatures, pO$_2$, ...) and spectral design (only one illumination performed at 689 nm). However, changing one, or a combination, of these conditions would lead to different values for F', $\Delta$F' and T, in particular. This is, in particular, the case if the chronogram of the application(s) of light is changed.

Therefore, the concepts presented above may be generalized for different conditions and cell types.

Example 8: Illumination of the tissues with radiometric conditions (irradiance/fluence rate, duration/dose) corresponding to multiple "hot spots" in figure 4a and 4b.

It should be underlined that figure 4a and 4b presents several "hot spots". Two "hot spots", namely the one, mentioned above, which corresponds to an irradiance of 3 mW/cm$^2$ and an illumination time of 180 s (hot spot 1), and a second one at 15 mW/cm$^2$ and 40 s for the irradiance and the illumination time, respectively (hot spot 2).

This is important, in particular to minimize the total treatment time.

Indeed, since "high" irradiances cannot be applied without damaging the tissues, only tissues located "close" to the illumination surface can be treated with the "high" irradiance of 15 mW/cm$^2$. Otherwise, cells close to the light source would experience thermal damages when distant cells receive a relatively high fluence rate.

It is well accepted by the scientific community of this field that thermal effects start to be significant if an irradiance of several hundreds of mW/cm$^2$ of red (or NIR) light is applied during more than several seconds over a broad (diameter larger than $\mu_{eff}^{-1}$) spot.

Figure 17 and 18 below illustrate how to take profit of the presence of the "hot spot 2" to minimize the treatment time if the irradiance must be less than 100 mW/cm$^2$ (the other conditions are identical to those considered for figures 10 and 11).

In this case, the treatment algorithm looks as follows:
As long as E < 100 mW/cm$^2$, the temporal evolution of this irradiance (or power) is given by the equations 5 (or 7) with: F' = 15 mW/cm$^2$; $\Delta$F' = 4 mW/cm$^2$ and T = 40 s. Otherwise, the values of F' = 3 mW/cm$^2$; $\Delta$F' = 1.6 mW/cm$^2$ and T = 180 s must be used.

This example can be enlarged by the use of the combination of wavelength(s) within their own hot spot(s).

Example 9: Use of a passive attenuator to generate a temporal evolution of the irradiance (or power) with continuous wave (CW) light sources according to equations 5 and 7.

Numerous light sources are commercially available nowadays to treat tissues by PBM. Needless to mention that none of them generate an irradiance (or power) according to equations 5 (or 7). However, since many of these commercially available light sources emit CW light and generate an irradiance larger than 0.62 mW/cm$^2$ (3 mW/cm$^2$ divided by k = 4.87 in our specific conditions), they may be combined with an attenuator which would change its transmission with time in such a way that the irradiance would correspond to the value given by equation 5.

More precisely, if E' is the irradiance produced at 689 nm by such a source without attenuator, the temporal evolution of the attenuator transmission (T$_r$) would be given by: T$_r$(t) = E(t)/E', where E(t) would be given by equation 5.

In summary, a particular design of the device according to the invention must integrate CW light sources combined with one or several attenuators to end up with an irradiance corresponding to that given in equation 5.

The generalizations mentioned above in Examples 1 to 8 also apply to this example.

Example 10: Use of high frequency modulation of light superposed to the temporal evolution of the irradiance or power given in example 4.

Since biological objects have dynamic optical absorption and responses to light, in part due to dynamic changes of their redox states, wavelength or multiplexed wavelengths used for PBM can be synchronized/modulated at higher frequencies than the temporal variation defining in equation 5 taking into account the kinetics/dynamic of the oxidative metabolic redox states.

Example 11: Use of pulse duration changes to modify the irradiance (or power) with pulsed light sources, according to equations 5 and 7.

As already mention in example 10, light can be modulated at higher frequency than that used for the variations of the irradiance (or power or fluence rate) according to equations 5 and 7. Since the average power P(t) is the time average of pulsed optical power p(t):

$$P(t) = \int p(t) dt$$

The temporal evolution of P(t) can be changed by the modulation of the duty cycle of p(t), for a given frequency and peak power.

Example 12: Induction of a bystander or abscopal effect.

As observed by the inventors under total blood volume illumination performed in the central venous line, transient or middle but significative modulations of the paO2 and other arterial gas such as chloric ion, took place in arterial blood only. Indeed, these modulations were not observed in the central venous blood (See figure 25 b and c). Since it is known that PBM induces, in certain conditions, bystander or abscopal effects, illuminating simultaneously or sequentially different parts of the biological object, including circulating objects such as the blood, is of high interest.

Example 13: Treatment by PBM of circulating biological object into blood or lymphatic vessels.

1) A temporal variation of the light power or irradiance can be performed to illuminate, within a range of fluence rates, circulating biologic object passing in the vicinity of the light distributor. The expressions of the irradiance and power given in example 4 can be adapted to take into account the different speeds of the biologic object into blood or lymphatic vessels targeted by PBM.
2) The power of light is synchronized with hemodynamic variables, such as the changes of flow due to the heart beat and the vasomotion, to irradiate optimally the targeted biologic object within the blood flow.

For instance, in order to overpass negative outcomes of the SARS-CoV-2 and, more generally, in the case of ARDS, optimizing the immune response, the hemoglobin oxygen affinity, the thrombogenesis processes and to promote the tissue regeneration using notably the bystander effects of PBM by inserting one or several light distributors in blood vessels, such as lung arteries, has shown impressive positive effects, as demonstrated by the inventors.

In particular, an example of a clinical procedure ("Seldinger method") to position light distributors in the right and left pulmonary arteries is described below:

1. Venous access by puncture of the right jugular vein under ultrasound imaging using an I.V catheter (Surflo, Terumo).
2. Introduction of a 7 Fr sheath (Radiofocus, Terumo) using the Seldinger method.
3. Under fluoroscopic guidance, use a 4 Fr JR 4 guiding catheter (Cordis) in order to engage the ostium of the right pulmonary artery, using a non-hydrophilic 0.035-inch guidewire (Radiofocus, Terumo).
4. Removal of the 0.035-inch guidewire and connection of a hemostasis valve Y connector to the 4 Fr JR 4 guiding catheter.
5. Placement of the optical distributor through the 4 Fr JR 4 guiding catheter between the entry of the upper and lower right lobar pulmonary artery.
6. Total removal of the 4 Fr JR 4 guiding catheter from the 7 Fr sheath.
7. Flush the 4 Fr JR 4 guiding catheter with saline through the hemostasis valve Y connector.

8. Use the same procedure as described in 3, 4, 5 and 6 with a second 4 Fr JR 4 guiding catheter in order to place a second optical distributor between the entry of the upper and lower left lobar pulmonary artery.

9. Attachment of the 2 optical distributors (intubated in the 4 Fr JR 4 guiding catheter) to the skin with an adhesive system (Grip Lock, Vygon).

This protocol can be adapted to performed a PBM illumination with one optical distributor place into the atrium as well as in the inferior and superior vena cava, as shown in figure 20. The optical distributor can be placed during days or weeks to repeat the treatment periodically.

Example 14): Combination of different illumination scheme.

The PBM effects result from the absorption of light by different primary photoacceptors leading, in particular, to changes of numerous signaling and transcription factors. PBM light is also known to photodissociate NO from nitroso-hemoglobin and to influence the nitrate reductase activity (NRA) involving certain metalloproteins, which also release labile NO at low oxygen tension and the presence of nitrite. Since NO is preconize within mild or deep hypoxemia, therefore different illuminations scheme must be considered to activate different mechanisms For instance, in circulating blood, the first illumination scheme can consist in the delivery of a constant or pulsed irradiance/fluence rate (higher power as possible while avoiding thermal effects, i.e. typically hundreds of $mW.cm^{-2}$) applied during an optimal time, ranging between seconds and minutes, at an appropriate wavelength to target the photodissociation of, for instance, nitrosyl-hemoglobin or sulfhemoglobin. This first illumination scheme must be combined with a second scheme based on the concept of hot spots mentioned above.

Example 15: The illumination of the biological object can be performed by a particular selection of hot spots, notably to optimize the treatment in term of time of duration. Figure 21, show the utilization of an hot spot line ($10\pm9,5$ $mW.cm^{-2}$; 40 s). The figure depicts the fluence rate from a cylindrical distributor into the myocardium illuminate at 689 nm; using a Bessel function of the second kind. Using this hot spot line, a first session of 40 s using a power into the cylindrical distributor of 2,8 $mW.cm^{-1}$ (which correspond to an fluence rate at the surface of the distributor equal to 20 $mW.cm^{-2}$ which enable to treat the first 3,5 mm. Then a second session of 40 s using a power of 100 $mW.cm^{-1}$ is used to treat tissue between 3,5 to 7 mm.

Example 16: The illumination of the biologic object can be performed synchronously with parameters corresponding to several hot spots. As shown in figure 22 since the fluence rate decreases with the distance from the light distributor, distant or deep-seated tissues are exposed to a low fluence rate (for example 3 $mW/cm^2$) when tissues close to the light distributor(s) are treated with a high fluence rate (for example 15 or 25 $mW/cm^2$)). This can be used to optimize the treatment time as well to target specific part of the biological object.

Example 17: A treatment protocol based on the synchronous or sequential use of several PBM-potent wavelengths presenting different penetration depths in tissues. As shown in figure 23 since the fluence rate decreases with the distance from the light distributor, distant or deep-seated tissues are treated with a penetrating wavelength with a fluence rate and duration corresponding to a "hot spot" presented in figure 4a and b, whereas tissues closer to the surface are treated with conditions corresponding to the same "hot spot" but with less penetrating wavelength(s). This can be used to optimize the treatment time as well to target specific part of the biological object.

Example 18: is a medical device for treating bone marrow, and inducing cell lines, designed to be introduced percutaneously into the bone marrow, femur, tibia, iliac crest or other medullary areas. It consists of one or more optical fibers. These fibers are put in a catheter sheath that can be attached to the skin. The distal ends of the fibers are hermetically connected to the catheter via an SMA connector which will be connected to the source. The proximal end is adjustable (between 2 and 8 cm) by retracting the catheter sheath, allowing the optical fibers to deploy, which are reinforced by a rigid material introduced into the spinal cord.

Example 19 Increasing and homogenizing the endogenous production of PpIX to improve the performances of PhotoDynamic Detection (PDD) and PDT. Embodiments of this surprising effect consists to:

1) Use of a helmet, integrating light emitting diodes, which induce a PBM illumination through the skull on a specific area of the brain between 6 and 72 hours before the PDD or PDT procedures to manage brain cancers, including glioblastoma.

2) Increasing and homogenizing the endogenous production of PpIX in plants and larvae. One embodiment of this approach is to increase the efficacy of the phototoxic effects induced in weed/larvae in the agriculture field. Which can be adapt to many agriculture engine.

Exemple20: Triggering or spatial resynchronization of metabolic activity of a biological object.

Spatial synchronization of metabolic activities is a must to sustains local or systemic homeostasis as well as to enable blood flow in arterial or venous capillaries. Notably, synchronized local contraction of a vessel from place to place induce vasomotion. These contractions can be seen as a spatial wavefront which move all along the vessel. A disruption of these synchronized contractions from a injured myogenic conduction for instance can be the cause of many vascular pathologies. Since it has been shown that different parts of the biological object can be targeted sequentially or successively by the selection of particular hotspots and since PBM can modulate or trig specific metabolic activities notably in the myogenic frequency range, illumination of a injured vessel at specific distance for instance, equal to the length define by the spatial period of the contraction wave can sustain the synchronization of the contraction from place to place in case of myogenic desynchronization or trig contractions from place to place to restore the blood flow.

The invention is as defined in the appended claims.

**References**

Aalkjær, C., Boedtkjer, D. & Matchkov, V., 2011. Vasomotion - what is currently thought? Acta Physiologica, 202(3), pp.253-269.

Acharya, U.R. et al., 2017. Application of higher-order spectra for the characterization of Coronary artery disease using electrocardiogram signals. Biomedical Signal Processing and Control, 31, pp.31-43. Available at: http://dx.doi.org/10.1016/j.bspc.2016.07.003

B. Alberts, A. Johnson, J. Lewis, M. Raff, K. Roberts, and P. Walter, Molecular Biology of the Cell, 4th ed. Garland Science, 2002.

Álvarez, R.C. et al., 2018. Cardiac Hemodynamic Monitoring in the Subcutaneous Space: A pre-clinical proof-of-concept. MeMeA 2018 - 2018 IEEE International Symposium on Medical Measurements and Applications, Proceedings.

F. Antunes, A. Boveris, and E. Cadenas, "On the mechanism and biology of cytochrome oxidase inhibition by nitric oxide," Proc. Natl. Acad. Sci. U. S. A., vol. 101, no. 48, pp. 16774-16779, Nov. 2004.

Bailey, S.M., Udoh, U.S. & Young, M.E., 2014. Circadian regulation of metabolism. Journal of Endocrinology, 222(2). DOI: https://doi.org/10.1530/JOE-14-0200.

K. A. Ball, P. R. Castello, and R. O. Poyton, "Low intensity light stimulates nitrite-dependent nitric oxide synthesis but not oxygen consumption by cytochrome c oxidase: Implications for phototherapy," J. Photochem. Photobiol. B, vol. 102, no. 3, pp. 182-191, Mar. 2011.

S. Banerjee and D. K. Bhatt, "Histochemical studies on the distribution of certain dehydrogenases in squamous cell carcinoma of cheek," Indian J. Cancer, vol. 26, no. 1, pp. 21-30, Mar. 1989.

M. A. Beilke, C. Collins-Lech, and P. G. Sohnle, "Effects of dimethyl sulfoxide on the oxidative function of human neutrophils," J. Lab. Clin. Med., vol. 110, no. 1, pp. 91-96, Jul. 1987.

I. Bentov and M. J. Reed, "The effect of aging on the cutaneous microvasculature," Microvasc. Res., vol. 100, pp. 25-31, 2015

Bhattacharyya, A. et al., 2018. A novel approach for automated detection of focal EEG signals using empirical wavelet transform. Neural Computing and Applications, 29(8), pp.47-57.

Bhoi, A.K., Sherpa, K.S. & Khandelwal, B., 2017. Arrhythmia and ischemia classification and clustering using QRS-ST-T (QT) analysis of electrocardiogram. Cluster Computing, 21(1), pp.1033-1044.

Bigarella, C.L., Liang, R. & Ghaffari, S., 2014. Stem cells and the impact of ROS signaling. Development (Cambridge), 141(22), pp.4206-4218.

T. S. Blacker and M. R. Duchen, "Investigating mitochondrial redox state using NADH and NADPH autofluorescence," Free Radic. Biol. Med., vol. 100, no. Supplement C, pp. 53-65, Nov. 2016.

Blackstone, E., Morrison, M. & Roth, M.B., 2005. H2S induces a suspended animation-like state in mice. Science, 308(5721), p.518.

Bleys, J., Navas-Acien, A. & Guallar, E., 2008. Serum selenium levels and all-cause, cancer, and cardiovascular mortality among US adults. Archives of Internal Medicine, 168(4), pp.404-410.

Brandes, R.P., Weissmann, N. & Schröder, K., 2014. Nox family NADPH oxidases in mechano-transduction: Mechanisms and consequences. Antioxidants and Redox Signaling, 20(6), pp.887-898.

M. Butawan, R. L. Benjamin, and R. J. Bloomer, "Methylsulfonylmethane: Applications and Safety of a Novel Dietary Supplement," Nutrients, vol. 9, no. 3, Mar. 2017.

Bos, E.M. et al., 2009. Hydrogen sulfide-induced hypometabolism prevents renal ischemia/reperfusion injury. Journal of the American Society of Nephrology, 20(9), pp.1901-1905.

Carlström, M., Wilcox, C.S. & Arendshorst, W.J., 2015. Renal autoregulation in health and disease. Physiological Reviews, 95(2), pp.405-511.

B. Chance, B. Schoener, R. Oshino, F. Itshak, and Y. Nakase, "Oxidation-reduction ratio studies of mitochondria in freeze-trapped samples. NADH and flavoprotein fluorescence signals," J. Biol. Chem., vol. 254, no. 11, pp. 4764-4771, Jun. 1979.

B. Chance, "Metabolic Heterogeneities in Rapidly Metabolizing Tissues," J. Appl. Cardiol., vol. 4, no. 4, pp. 207-221, 1989.

Chouchani E. T.,1,2 Victoria R. Pell,3 Andrew M. James,4 Lorraine M. Work,5 Kourosh Saeb-Parsy,6 Christian Frezza,7 Thomas Krieg,3 and Michael P. Murphy4, "A Unifying Mechanism for Mitochondrial Superoxide Production during Ischemia-Reperfusion Injury", Cell Metabolism 23, February 9, 2016.

Chouchani, E.T. et al., 2014. Ischaemic accumulation of succinate controls reperfusion injury through mitochondrial ROS. Nature, 515(7527), pp.431-435.

Chung H, Dai T, Sharma S, Huang Y, Carroll J, Hamblin M. The Nuts and Bolts of Low-level Laser (Light) Therapy. Ann Biomed Eng. 2012;40(2):516-533. doi:10.1007/s10439-011-0454-7.

C. E. Cooper and G. C. Brown, "The inhibition of mitochondrial cytochrome oxidase by the gases carbon monoxide, nitric oxide, hydrogen cyanide and hydrogen sulfide: chemical mechanism and physiological significance," J. Bioenerg. Biomembr., vol. 40, no. 5, pp. 533-539, Oct. 2008.

Dasgupta, A., 2014. Cardiac Markers. Clinical Chemistry, Immunology and Laboratory Quality Control, pp.127-144. https://doi.org/10.1016/B978-0-12-407821-5.00008-5.

Devasagayam TP, Tilak JC, Boloor KK, Sane KS, Ghaskadbi SS, Lele RD, "Free radicals and antioxidants in human health: current status and future prospects". The Journal of the Association of Physicians of India. 52: 794-804, 2004.

Di Gialleonardo V., David M. Wilson, and Kayvan R. Keshari, "The Potential of Metabolic Imaging", Semin. Nucl. Med., 46(1): 28-39, 2016.

Donald JA. Gasotransmitter Family. In: Handbook of Hormones. Elsevier; 2016:601. doi:doi.org/10.1016/B978-0-12-801028-0.00103-3.

Duan, J. et al.. Metabolic remodeling induced by mitokines in heart failure. Aging (Albany NY). 2019 Sep 15; 11(17): 7307-7327.

Ferraresi C., M. R. Hamblin, and N. A. Parizotto, "Low-level laser (light) therapy (LLLT) on muscle tissue: performance, fatigue and repair benefited by the power of light," Photonics Lasers Med., vol. 1, no. 4, pp. 267-286, Nov. 2012.

Ferrari R., MD, PhD; Cristina Balla, MD; Michele Malagù, MD; Gabriele Guardigli, MD; Giampaolo Morciano, MD, PhD; Matteo Bertini, MD; Simone Biscaglia, MD; Gianluca Campo, MD, "Reperfusion Damage - A Story of Success, Failure, and Hope", Circ J, 81: 131-141, 2017.

Fu M, Zhang W, Wu L, Yang G, Li H, Wang R., "Hydrogen sulfide (H2S) metabolism in mitochondria and its regulatory role in energy production", Proc Natl Acad Sci U S A., 109(8):2943-8, 2012.

Fuss T. L., B.A. and Leo L. Cheng, "Metabolic Imaging in Humans", Top Magn Reson Imaging, 25(5): 223-235, 2016.

R. A. Gatenby and R. J. Gillies, "Why do cancers have high aerobic glycolysis?," Nat. Rev. Cancer, vol. 4, no. 11, pp. 891-899, Nov. 2004.

Grimolizzi, F. & Arranz, L., 2018. Multiple faces of succinate beyond metabolism in blood. Haematologica, 103(10), pp.1586-1592.

Griendling, K.K. et al., 2016. Measurement of Reactive Oxygen Species, Reactive Nitrogen Species, and Redox-Dependent Signaling in the Cardiovascular System: A Scientific Statement from the American Heart Association, Circulation journal, 119,5,39-65.

Hamblin M., M. Victor Pires de Sousa and T. Agrawal, "Handbook of Low-Level Laser Therapy", Pan Stanford Publishing Pte. Ltd., Singapore, 2017.

Hamblin M., C. Ferraresi, Y.-Y. Huang, L. F. de Freitas, and J. Carroll, "Low-level light therapy: photobiomodulation", Editor: SPIE Press, Bellingham, Washington, USA, 2018.

Hayyan M, Hashim MA, AlNashef IM (March 2016). "Superoxide Ion: Generation and Chemical Implications". Chemical Reviews. 116 (5): 3029-85.

Hellmann, M., Roustit, M. & Cracowski, J.L., 2015. Skin microvascular endothelial function as a biomarker in cardiovascular diseases? Pharmacological Reports, 67(4), pp.803-810.

Huang, Ying-Ying, Carroll JD, H.M., 2009. Biphasic dose response. Dose response, pp.358-383. http://dx.doi.org/10.2203/dose-response.09-027.Hamblin

Hwang, J. et al., 2003. Pulsatile Versus Oscillatory Shear Stress Regulates NADPH Oxidase Subunit Expression: Implication for Native LDL Oxidation. Circulation Research, 93(12), pp.1225-1232.

Iwata, A. et al., 2015. Selenide Targets to Reperfusing Tissue and Protects It From Injury*. Read Online: Critical Care Medicine | Society of Critical Care Medicine, 43(7).

Jacques S., "How tissue optics affect dosimetry of photodynamic therapy", Journal of Biomedical Optics 15(5), 051608, 2010.

Jekell, A. et al., 2004. Elevated circulating levels of thioredoxin and stress in chronic heart failure. The european Journal of Heart failure, 6, pp.883-890.

Jackson, W.F., 2016. Arteriolar oxygen reactivity: where is the sensor and what is the mechanism of action? Journal of Physiology, 594(18), pp.5055-5077.

S. Kalinina et al., "Correlative NAD(P)H-FLIM and oxygen sensing-PLIM for metabolic mapping," J. Biophotonics, vol. 9, no. 8, pp. 800-811, Aug. 2016.

Kalogeris T., Christopher P. Baines1,2,3, Maike Krenz1,2, and Ronald J. Korthuis Ischemia/Reperfusion, Compr Physiol. ; 7(1): 113-170, 2017.

T. I. Karu, L. V. Pyatibrat, and N. I. Afanasyeva, "Cellular effects of low power laser therapy can be mediated by nitric oxide," Lasers Surg. Med., vol. 36, no. 4, pp. 307-314, Apr. 2005.

Kennedy R. T., et al., « Metabolic oscillations in □-cells », Diabetes, 51(S1), 2002.

Y. H. Kim, D. H. Kim, H. Lim, D.-Y. Baek, H.-K. Shin, and J.-K. Kim, "The anti-inflammatory effects of methylsulfonylmethane on lipopolysaccharide-induced inflammatory responses in murine macrophages," Biol. Pharm. Bull., vol. 32, no. 4, pp. 651-656, Apr. 2009.

Kindo, M. et al., 2016. Left Ventricular Transmural Gradient in Mitochondrial Respiration Is Associated with Increased Sub-Endocardium Nitric Oxide and Reactive Oxygen Species Productions. Frontiers in physiology, 7(August), pp.1-8.

Kindo, M. et al., 2012. Pressure overload-induced mild cardiac hypertrophy reduces left ventricular transmural differences in mitochondrial respiratory chain activity and increases oxidative stress. Frontier in physiology, 3(August), pp.1-14.

Kondo K, Bhushan S, King AL, Prabhu SD, Hamid T, Koenig S, et al. "H2S protects against pressure overload-induced heart failure via upregulation of endothelial nitric oxide synthase", Circulation, 127:1116-1127, 2013.

Köhn, C. et al., 2012. Hydrogen sulfide: Potent regulator of vascular tone and stimulator of angiogenesis. International Journal of Biomedical Science, 8(2), pp.81-86.

Kuganesan, M. et al., 2019. Selenium and hydrogen selenide: essential micronutrient and the fourth gasotransmitter? Intensive Care Medicine Experimental, 7(1). http://dx.doi.org/10.1186/s40635-019-0281-y.

Kumar, M., Pachori, R.B. & Acharya, U.R., 2017. Characterization of coronary artery disease using flexible analytic wavelet transform applied on ECG signals. Biomedical Signal Processing and Control, 31, pp.301-308.

Kvandal, P. et al., 2006. Low-frequency oscillations of the laser Doppler perfusion signal in human skin. Microvascular Research, 72(3), pp.120-127.

N. Lane, "Cell biology: power games," Nature, vol. 443, no. 7114, pp. 901-903, Oct. 2006.

Latham, F., 1951. THE OXYGEN PARADOX EXPERIMENTS ON THE EFFECTS OF OXYGEN IN HUMAN ANOXIA. The Lancet, 257(6646), pp.77-81

Li, S. & Yang, G., 2015. Hydrogen Sulfide Maintains Mitochondrial DNA Replication via Demethylation of TFAM. Antioxidants and Redox Signaling, 23(7), pp.630-642.

Li, Y.S.J., Haga, J.H. & Chien, S., 2005. Molecular basis of the effects of shear stress on vascular endothelial cells. Journal of Biomechanics, 38(10), pp.1949-1971.

Li, Y and Patrick J. Pagano, 2017. Microvascular NADPH oxidase in health and disease. Free Radic Biol Med., 109(1), pp.33-47.

Liebert, A. et al., 2017. A Role for Photobiomodulation in the Prevention of Myocardial Ischemic Reperfusion Injury: A Systematic Review and Potential Molecular Mechanisms. Scientific Reports, 7(February), pp.1-13. Available at: http://dx.doi.org/10.1038/srep42386.

N. L. Lohr, A. Keszler, P. Pratt, M. Bienengraber, D. C. Warltier, and N. Hogg, "Enhancement of nitric oxide release from nitrosyl hemoglobin and nitrosyl myoglobin by red/near infrared radiation: potential role in cardioprotection," J. Mol. Cell. Cardiol., vol. 47, no. 2, pp. 256-263, Aug. 2009.

Loutzenhiser, R., Bidani, A. & Chilton, L., 2002. Renal myogenic response: Kinetic attributes and physiological role. Circulation Research, 90(12), pp.1316-1324.

Martelli F., Del Bianco S., Ismaelli A., "Light propagation through biological tissue and other diffusive media: theory, solutions, and software", Bellingham: Society of Photo-Optical Instrumentation Engineers, 2009.

Irma Martišienė, Regina Mačianskienė, Voltage-Sensitive Fluorescence of Indocyanine Green in the Heart, Biophysical Journal, Volume 110, Issue 3, 2016, Pages 723-732, ISSN 0006-3495.

Moldogazieva, N.T. et al., 2018. ROS and RNS signalling: adaptive redox switches through oxidative/nitrosative protein modifications. Free Radical Research, 52(5), pp.507-543. Available at: https://doi.org/10.1080/10715762.2018.1457217.

Moss, N.G., Gentle, T.K. & Arendshorst, W.J., 2016. Modulation of the myogenic mechanism: Concordant effects of NO synthesis inhibition and O-2 dismutation on renal autoregulation in the time and frequency domains. American Journal of Physiology - Renal Physiology, 310(9), pp.F832-F845.

Nachiappan, Vasanthi; Muthukumar, Kannan, "Cadmium-induced oxidative stress in Saccharomyces cerevisiae". Indian Journal of Biochemistry and Biophysics. 47 (6), 2010.

Nagpure, B. V. & Bian, J.S., 2016. Interaction of Hydrogen Sulfide with Nitric Oxide in the Cardiovascular System. Oxidative Medicine and Cellular Longevity, 2016.

Nakamura, H., 2004. Thioredoxin as a Key Molecule in Redox Signaling. Antioxidants and Redox Signaling, 6(1), pp.15-17.

Nowak-Sliwinska P., J.-P. Ballini, G. Wagnières, and H. van den Bergh, "Processing of fluorescence angiograms for the quantification of vascular effects induced by anti-angiogenic agents in the CAM model," Microvasc. Res., vol. 79, no. 1, pp. 21-28, Jan. 2010.

P.T. Nowicki, S. Flavahan, H. Hassanain, S. Mitra, S. Holland, P.J. Goldschmidt-Clermont, N.A.F., 2001. Redox Signaling of the Arteriolar Myogenic Response. Circulation research, 89, pp.114-116.

Olson, K.R., 2012. A practical look at the chemistry and biology of hydrogen sulfide. Antioxidants and Redox Signaling, 17(1), pp.32-44.

Papagiannakis A, Niebel B, Wit E, Heinemann M, Autonomous Metabolic Oscillations Robustly Gate the Early and Late Cell Cycle. Molecular Cell, Volume, 65, Issue 2, 19 January 2017, Pages 285-295, https://doi.org/10.1016/j.molcel.2016.11.018.

Park, H.C. et al., 2017. Effect of LED phototherapy on blood lactate level in Taekwondo contest. Mechanisms of Photobiomodulation Therapy XII, 10048(February 2017), p.100480I.

Patidar, S. & Pachori, R.B., 2014. Classification of cardiac sound signals using constrained tunable-Q wavelet transform. Expert Systems with Applications, 41(16), pp.7161-7170. Available at: http://dx.doi.org/10.1016/j.eswa.2014.05.052.

Patidar, S., Pachori, R.B. & Rajendra Acharya, U., 2015. Automated diagnosis of coronary artery disease using tunable-Q wavelet transform applied on heart rate signals. Knowledge-Based Systems, 82, pp.1-10. Available at: http://dx.doi.org/10.1016/j.knosys.2015.02.011.

Pettersen, F.J. et al., 2016. Bioimpedance measurements of temporal changes in beating hearts. Biomedical Physics and Engineering Express, 2(6).

Petrenko, V. et al., 2020. In pancreatic islets from type 2 diabetes patients, the dampened circadian oscillators lead to reduced insulin and glucagon exocytosis. Proceedings of the National Academy of Sciences of the United States of America, 117(5), pp.2484-2495

Piper, H.M., 2000. The calcium paradox revisited: An artefact of great heuristic value. Cardiovascular Research, 45(1), pp.123-127

N. Ramanujam, R. Richards-Kortum, S. Thomsen, A. Mahadevan-Jansen, M. Follen, and B. Chance, "Low Temperature Fluorescence Imaging of Freeze-trapped Human Cervical Tissues," Opt. Express, vol. 8, no. 6, pp. 335-343, Mar. 2001.

V. Rapozzi, E. Della Pietra, S. Zorzet, M. Zacchigna, B. Bonavida, and L. E. Xodo, "Nitric oxide-mediated activity in anti-cancer photodynamic therapy," Nitric Oxide Biol. Chem., vol. 30, pp. 26-35, Apr. 2013.

Raddatz E, Thomas AC and al. Differential contribution of mitochondria, NADPH oxidases, and glycolysis to region-specific oxidant stress in the anoxic-reoxygenated embryonic heart. Am J Physiol Heart Circ Physiol 300: H820-H835, 2011.

K. J. Reeves, M. W. R. Reed, and N. J. Brown, "Is nitric oxide important in photodynamic therapy?," J. Photochem. Photobiol. B, vol. 95, no. 3, pp. 141-147, Jun. 2009.

Reglin, B. & Pries, A.R., 2014. Metabolic control of microvascular networks: Oxygen sensing and beyond. Journal of Vascular Research, 51(5), pp.376-392.

De Roever I, Bale G, Cooper RJ, Tachtsidis I." Functional NIRS Measurement of Cytochrome-C-Oxidase Demonstrates a More Brain-Specific Marker of Frontal Lobe Activation Compared to the Haemoglobins", Adv Exp Med Biol. 2017;977:141-147, 2017.

Sachar M. et al. "Protoporphyrin IX: the Good, the Bad, and the Ugly." The Journal of pharmacology and experimental therapeutics vol. 356,2 (2016): 267-75. doi:10.1124/jpet.115.228130.

Sedmera, D., Kucera, P. & Raddatz, E., 2002. Developmental changes in cardiac recovery from anoxia-reoxygenation. American Journal of Physiology - Regulatory Integrative and Comparative Physiology, 283(2 52-2), pp.379-388.

Shiogai, Y., Stefanovska, A. & McClintock, P.V.E., 2010. Nonlinear dynamics of cardiovascular ageing. Physics Reports, 488(2-3), pp.51-110.

Sies, H. & Jones, D.P., Reactive oxygen species (ROS) as pleiotropic physiological signalling agents. Nature Reviews Molecular Cell Biology. 2020 Available at: http://dx.doi.org/10.1038/s41580-020-0230-3.

M. C. Skala et al., "In vivo multiphoton microscopy of NADH and FAD redox states, fluorescence lifetimes, and cellular morphology in precancerous epithelia," Proc. Natl. Acad. Sci., vol. 104, no. 49, pp. 19494-19499, Dec. 2007.

M. Skala and N. Ramanujam, "Multiphoton Redox Ratio Imaging for Metabolic Monitoring in vivo," Methods Mol. Biol. Clifton NJ, vol. 594, pp. 155-162, 2010.

Smirnova, E. et al., 2013. Assessment of endothelial dysfunction in patients with impaired glucose tolerance during a cold pressor test. Diabetes and Vascular Disease Research, 10(6), pp.489-497.

Snijder P. M., A R Frenay, R A de Boer, A Pasch, J L Hillebrands, H G D Leuvenink and H van Goor, "Exogenous administration of thiosulfate, a donor of hydrogen sulfide, attenuates angiotensin II-induced hypertensive heart disease in rats", Br J Pharmacol. 2015 Mar; 172(6): 1494-1504.

Stefanovska, A., 2006. Coupled oscillators: Complex but not complicated cardiovascular and brain interactions. Annual International Conference of the IEEE Engineering in Medicine and Biology - Proceedings, pp.437-440

Szabo, C. et al., 2014. Regulation of mitochondrial bioenergetic function by hydrogen sulfide. Part I. Biochemical and physiological mechanisms. British Journal of Pharmacology, 171(8), pp.2099-2122.

Tamura, T., 2019. Current progress of photoplethysmography and SPO2 for health monitoring. Biomedical Engineering Letters, 9(1), pp.21-36. Available at: https://doi.org/10.1007/s13534-019-00097-w.

Tamura, T., 2018. Seamless Healthcare Monitoring, https://doi.org/10.1007/978-3-319-69362-0

Thomas DD. Breathing new life into nitric oxide signaling: A brief overview of the interplay between oxygen and nitric oxide. Redox Biol. 2015;5:225-233. doi:10.1016/j.redox.2015.05.002.

Ticcinelli, V. et al., 2017. Coherence and coupling functions reveal microvascular impairment in treated hypertension. Frontiers in Physiology, 8(OCT), pp.1-20.

EP 4 221 828 B1

Tretter, L., Patocs, A. & Chinopoulos, C., 2016. Succinate, an intermediate in metabolism, signal transduction, ROS, hypoxia, and tumorigenesis. Biochimica et Biophysica Acta - Bioenergetics, 1857(8), pp.1086-1101. Available at: http://dx.doi.org/10.1016/j.bbabio.2016.03.012.

Tsuruoka, N. et al., 2016. Lactate and glucose measurement in subepidermal tissue using minimally invasive microperfusion needle. Biomedical microdevices, (18:19), pp.2-9. http://dx.doi.org/10.1007/s10544-016-0049-z.

Tuchin V., "Tissue Optics and Photonics: Light-Tissue Interaction", J of Biomedical Photonics & Eng 1(2), pp 98 - 134, 2015.

Van Leeuwen, S.R., Baranoski, G.V.G. & Kimmel, B.W., 2017. Three-wavelength method for the optical differentiation of methemoglobin and sulfhemoglobin in oxygenated blood. Proceedings of the Annual International Conference of the IEEE Engineering in Medicine and Biology Society, EMBS, pp.4570-4573.

Arnoud H. M. van Vliet, Stefan Bereswill, and Johannes G. Kusters, "Ion Metabolism and Transport", in: Helicobacter pylori: Physiology and Genetics, Mobley HLT, Mendz GL, Hazell SL, editors, Washington (DC): ASM Press; 2001.

Wagnières G. et al., "In Vivo Fluorescence Spectroscopy and Imaging for Oncological Applications", Photochemistry and Photobiology, 68(5): 603-632, 1998.

A. Walsh, R. S. Cook, B. Rexer, C. L. Arteaga, and M. C. Skala, "Optical imaging of metabolism in HER2 overexpressing breast cancer cells," Biomed. Opt. Express, vol. 3, no. 1, pp. 75-85, Jan. 2012.

A. J. Walsh et al., "Optical metabolic imaging identifies glycolytic levels, subtypes, and early-treatment response in breast cancer," Cancer Res., vol. 73, no. 20, pp. 6164-6174, Oct. 2013.

G. Walther et al., "Metabolic syndrome individuals with and without type 2 diabetes mellitus present generalized vascular dysfunction: Cross-sectional study," Arterioscler. Thromb. Vasc. Biol., vol. 35, no. 4, pp. 1022-1029, 2015.

Wilson B., Patterson M., "The physics of photodynamic therapy", Phys. Med. Biol., (31)4, pp 327-360, 1986.

Wood K, Cortese-Krott M and al. Circulating Blood Endothelial Nitric Oxide Synthase Contributes to the Regulation of Systemic Blood Pressure and Nitrite Homeostasis. Arterioscler Thromb Vasc Biol. 2013;33:1861-1871.

Wu, M., Neilson, A., Swift, A.L., Moran, R., Tamagnine, J., Parslow, D., Armistead S., Lemire, K., Orrell, J., Teich, J., Chomicz, S., Ferrick, D.A., Multiparameter metabolic analysis reveals a close link between attenuated mitochondrial bioenergetic function and enhanced glycolysis dependency in human tumor cells. Am J Physiol Cell Physiol. 292, C125-C136, 2007.

Wu, D., Hu, Q. & Zhu, D., 2018. An update on hydrogen sulfide and nitric oxide interactions in the cardiovascular system. Oxidative Medicine and Cellular Longevity, 2018.

Yang, G., 2015. H2S epigenetic regulation of vascular cell functions. Cardiovascular Regenerative Medicine, pp.2-5.

Yu XH, Cui LB, Wu K, et al. Hydrogen sulfide as a potent cardiovascular protective agent. Clin Chim Acta. 2014;437:78-87. doi:10.1016/j.cca.2014.07.012.

Zhong, X. et al., 2018. Circadian Clock Regulation of Hepatic Lipid Metabolism by Modulation of m6A mRNA Methylation. Cell Reports, 25(7), p.1816-1828.e4.

Zhou, G. et al., 2016. Optimal ROS Signaling Is Critical for Nuclear Reprogramming. Cell Reports, 15(5), pp.919-925. Available at: http://dx.doi.org/10.1016/j.celrep.2016.03.084.

## Claims

1. A device for applying Photobiomodulation (PBM) on a biological object comprising a light source delivering light with an adequate temporal evolution of its optical power, said device also comprising a processing and/or a light control unit

33

configured to determine the adequate temporal evolution of the optical power to minimize the total treatment time on the basis of the biological object optical coefficients and the light delivery geometry on/in the biological object, **characterized by** the fact that said processing and/or light control unit is configured to control the light source to deliver light according to an illumination scheme protocol comprising at least a combined fluence rate and a time selected from the list comprising $3\pm2$ mW/cm$^2$ during $180\pm30$ s, $11\pm9$ mW/cm$^2$ during $80\pm25$ s, $16\pm10$ mW/cm$^2$ during $40\pm20$ s, $25\pm10$ mW/cm$^2$ during $15\pm10$ s and/or $10\pm9,7$ mW/cm$^2$ during $40\pm1$ s.

2. Device according to claim 1 comprising a glucose sensor and wherein said processing and/or light control unit is configured to adjust the light dose according to the level of glycemia measured by said glucose sensor.

3. Device according to anyone of the previous claims comprising a cardiac output sensor and wherein said processing and/or light control unit is configured to adjust the light dose according to the cardiac output measured by said cardiac output sensor.

4. Device according to anyone of the previous claims comprising a Krebs cycle enzymes kinetics measurement means and wherein said processing and/or light control unit is configured to adjust the light dose according to said enzyme activity measured by said Krebs cycle enzymes kinetics measurement means.

5. Device according to anyone of the previous claims comprising a unit for administering to said biological object at least one exogenous stimulus.

6. Device according to claim 5, wherein the at least one exogenous stimulus is an oxygen delivery.

7. Device according to claim 5, wherein the at least one exogenous stimulus is a temperature change.

8. Device according to claim 5, wherein the at least one exogenous stimulus is a gasotransmitter donor.

9. Device according to anyone of the previous claims comprising a metabolic monitoring unit, configured to adjust the optical power, the delivery of light, the time, the fluence rate and/or irradiance based on at least one metabolic parameter reflecting a metabolic activity of the biological object measured by the metabolic monitoring unit.

10. Device according to claim 9, wherein the adjustment of the optical power, the delivery of light, the time, the fluence rate and/or the irradiance influences or modify an amplitude, a frequency of fluctuations and/or a change of the at least one metabolic parameter of the biological object.

11. Device according to claim 10, wherein the at least one metabolic parameter is selected from the list comprising: temperature of the biological object, autofluorescence of the biological object, redox ratio, hemoglobin saturation, hemoglobin derivative contents, pH and/or bicarbonate levels, reactive oxygen species concentration, hydrogen sulfide level, hydrogen selenide level, ion concentration, cytochrome level, vascular tone of the biological object, vasomotion, electrical bioimpedance measurements, marker level, glucose level, succinate level, lactate and lactate dehydrogenase level, thioredoxin level, oxidative stress and/or chloride ions level.

12. Device according to anyone of claims 8-11, wherein the processing and/or light control unit is configured to predict the optimal time to start the application of PBM based on the at least one metabolic parameter measured by the metabolic monitoring unit.

13. Device according to anyone of the previous claims, **characterized by** the fact that the processing and/or light control unit is configured to generate a combined or sequential light comprising at least one potent wavelength and at least one ineffective wavelength when used alone.

14. Device according to claim 13, wherein the potent wavelength is 689nm or 808nm and the ineffective wavelength is 730nm.

15. Device according to anyone of the previous claims, wherein the light is delivered sequentially to treat simultaneously different depths of the biological object and at different distances from a surface of the biological object.

16. Device according to anyone of the previous claims, wherein the light source is adapted to be inserted in a heart compartment, the pulmonary artery or the cava vein such that light is delivered directly in the blood.

**Patentansprüche**

1. Vorrichtung zum Anwenden von Photobiomodulation (PBM) auf ein biologisches Objekt, umfassend eine Lichtquelle, die Licht mit einer angemessene zeitlichen Entwicklung seiner optischen Leistung abgibt, wobei die Vorrichtung auch eine Verarbeitungs- und/oder eine Lichtsteuereinheit umfasst, die dazu ausgelegt ist, die angemessene zeitliche Entwicklung der optischen Leistung zu bestimmen, um die Gesamtbehandlungszeit auf Basis der optischen Koeffizienten des biologischen Objekts und der Lichtabgabegeometrie auf/in dem biologischen Objekt zu minimieren, **dadurch gekennzeichnet, dass** die Verarbeitungs- und/oder Lichtsteuereinheit dazu ausgelegt ist, die Lichtquelle zu steuern, um Licht gemäß einem Beleuchtungsschemaprotokoll abzugeben, das mindestens eine kombinierte Fluenzrate und eine Zeit ausgewählt aus der Liste umfasst, die $3\pm2$ mW/cm$^2$ während $180\pm30$ s, $11\pm9$ mW/cm$^2$ während $80\pm25$ s, $16\pm10$ mW/cm$^2$ während $40\pm20$ s, $25\pm10$ mW/cm$^2$ während $15\pm10$ s und/oder $10\pm9,7$ mW/cm$^2$ während $40\pm1$ s umfasst.

2. Vorrichtung nach Anspruch 1, umfassend einen Glucosesensor, und wobei die Verarbeitungs- und/oder Lichtsteuereinheit dazu ausgelegt ist, die Lichtdosis gemäß dem durch den Glucosesensor gemessenen Glykämieniveau anzupassen.

3. Vorrichtung nach einem der vorhergehenden Ansprüche, umfassend einen Herzzeitvolumensensor, und wobei die Verarbeitungs- und/oder Lichtsteuereinheit dazu ausgelegt ist, die Lichtdosis gemäß dem durch den Herzzeitvolumensensor gemessenen Herzzeitvolumen anzupassen.

4. Vorrichtung nach einem der vorhergehenden Ansprüche, umfassend ein Messmittel für Kinetik von Enzymen des Krebs-Zyklus, und wobei die Verarbeitungs- und/oder Lichtsteuereinheit ausgelegt ist, um die Lichtdosis gemäß der Enzymaktivität anzupassen, die durch das Messmittel für Kinetik von Enzymen des Krebs-Zyklus gemessen wird.

5. Vorrichtung nach einem der vorhergehenden Ansprüche, umfassend eine Einheit zum Verabreichen mindestens eines exogenen Stimulus an das biologische Objekt.

6. Vorrichtung nach Anspruch 5, wobei der mindestens eine exogene Stimulus eine Sauerstoffgabe ist.

7. Vorrichtung nach Anspruch 5, wobei der mindestens eine exogene Stimulus eine Temperaturänderung ist.

8. Vorrichtung nach Anspruch 5, wobei der mindestens eine exogene Stimulus ein Gasotransmitter-Donator ist.

9. Vorrichtung nach einem der vorhergehenden Ansprüche, umfassend eine Stoffwechselüberwachungseinheit, die dazu ausgelegt ist, die optische Leistung, die Lichtabgabe, die Zeit, die Fluenzrate und/oder die Bestrahlungsstärke basierend auf mindestens einem Stoffwechselparameter anzupassen, der eine Stoffwechselaktivität des biologischen Objekts widerspiegelt, die durch die Stoffwechselüberwachungseinheit gemessen wird.

10. Vorrichtung nach Anspruch 9, wobei die Anpassung der optischen Leistung, der Lichtabgabe, der Zeit, der Fluenzrate und/oder der Bestrahlungsstärke eine Amplitude, eine Frequenz von Schwankungen und/oder eine Änderung des mindestens einen Stoffwechselparameters des biologischen Objekts beeinflusst oder modifiziert.

11. Vorrichtung nach Anspruch 10, wobei der mindestens eine Stoffwechselparameter ausgewählt ist aus der Liste, die Folgendes umfasst: Temperatur des biologischen Objekts, Autofluoreszenz des biologischen Objekts, Redoxverhältnis, Hämoglobinsättigung, Hämoglobinderivatgehalte, pH-Wert und/oder Bicarbonatspiegel, Konzentration der reaktiven Sauerstoffspezies, Schwefelwasserstoffspiegel, Selenwasserstoffspiegel, Ionenkonzentration, Cytochromspiegel, Gefäßtonus des biologischen Objekts, Vasomotion, elektrische Bioimpedanzmessungen, Markerspiegel, Glukosespiegel, Succinatspiegel, Lactat- und Lactatdehydrogenasespiegel, Thioredoxinspiegel, oxidativer Stress und/oder Chloridionenspiegel.

12. Vorrichtung nach einem der Ansprüche 8-11, wobei die Verarbeitungs- und/oder Lichtsteuereinheit dazu ausgelegt ist, die optimale Zeit zum Starten der Anwendung von PBM basierend auf dem mindestens einen Stoffwechselparameter, der durch die Stoffwechselüberwachungseinheit gemessen wird, vorherzusagen.

13. Vorrichtung nach einem der vorhergehenden Ansprüche, **dadurch gekennzeichnet, dass** die Verarbeitungs- und/oder Lichtsteuereinheit dazu ausgelegt ist, ein kombiniertes oder sequentielles Licht zu erzeugen, das mindestens eine potente Wellenlänge und mindestens eine bei alleiniger Verwendung ineffektive Wellenlänge umfasst.

**14.** Vorrichtung nach Anspruch 13, wobei die potente Wellenlänge 689 nm oder 808 nm beträgt und die ineffektive Wellenlänge 730 nm beträgt.

**15.** Vorrichtung nach einem der vorhergehenden Ansprüche, wobei das Licht sequentiell abgegeben wird, um gleichzeitig unterschiedliche Tiefen des biologischen Objekts und in unterschiedlichen Abständen von einer Oberfläche des biologischen Objekts zu behandeln.

**16.** Vorrichtung nach einem der vorhergehenden Ansprüche, wobei die Lichtquelle dazu eingerichtet ist, in ein Herzkompartiment, die Pulmonalarterie oder die Vena Cava eingeführt zu werden, so dass Licht direkt in das Blut abgegeben wird.

**Revendications**

**1.** Dispositif destiné à appliquer une photobiomodulation (PBM) sur un objet biologique comprenant une source de lumière délivrant de la lumière avec une évolution temporelle adéquate de sa puissance optique, ledit dispositif comprenant également une unité de traitement et/ou de contrôle de la lumière conçue pour déterminer l'évolution temporelle adéquate de la puissance optique afin de minimiser le temps total de traitement sur la base de coefficients optiques de l'objet biologique et de la géométrie de délivrance de lumière sur/dans l'objet biologique, **caractérisé par le fait que** ladite unité de traitement et/ou de contrôle de la lumière est conçue pour contrôler la source de lumière afin qu'elle délivre de la lumière selon un protocole de schéma d'éclairage comprenant au moins une combinaison de débit de fluence et de temps choisie dans la liste comprenant $3 \pm 2$ mW/cm$^2$ pendant $180 \pm 30$ s, $11 \pm 9$ mW/cm$^2$ pendant $80 \pm 25$ s, $16 \pm 10$ mW/cm$^2$ pendant $40 \pm 20$ s, $25 \pm 10$ mW/cm$^2$ pendant $15 \pm 10$ s et/ou $10 \pm 9{,}7$ mW/cm$^2$ pendant $40 \pm 1$ s.

**2.** Dispositif selon la revendication 1 comprenant un capteur de glucose et dans lequel ladite unité de traitement et/ou de contrôle de la lumière est conçue pour régler la dose de lumière en fonction du niveau de glycémie mesuré par ledit capteur de glucose.

**3.** Dispositif selon l'une quelconque des revendications précédentes comprenant un capteur de débit cardiaque et dans lequel ladite unité de traitement et/ou de contrôle de la lumière est conçue pour régler la dose de lumière en fonction du débit cardiaque mesuré par ledit capteur de débit cardiaque.

**4.** Dispositif selon l'une quelconque des revendications précédentes comprenant un moyen de mesure de la cinétique des enzymes du cycle de Krebs et dans lequel ladite unité de traitement et/ou de contrôle de la lumière est conçue pour régler la dose de lumière en fonction de ladite activité enzymatique mesurée par ledit moyen de mesure de la cinétique des enzymes du cycle de Krebs.

**5.** Dispositif selon l'une quelconque des revendications précédentes comprenant une unité servant à administrer audit objet biologique au moins un stimulus exogène.

**6.** Dispositif selon la revendication 5, dans lequel l'au moins un stimulus exogène est une délivrance d'oxygène.

**7.** Dispositif selon la revendication 5, dans lequel l'au moins un stimulus exogène est un changement de température.

**8.** Dispositif selon la revendication 5, dans lequel l'au moins un stimulus exogène est un donneur de gazotransmetteur.

**9.** Dispositif selon l'une quelconque des revendications précédentes comprenant une unité de surveillance métabolique, conçue pour régler la puissance optique, la délivrance de lumière, le temps, le débit de fluence et/ou l'éclairement énergétique sur la base d'au moins un paramètre métabolique reflétant une activité métabolique de l'objet biologique mesurée par l'unité de surveillance métabolique.

**10.** Dispositif selon la revendication 9, dans lequel le réglage de la puissance optique, de la délivrance de lumière, du temps, du débit de fluence et/ou de l'éclairement énergétique influence ou modifie une amplitude, une fréquence de fluctuations et/ou une variation de l'au moins un paramètre métabolique de l'objet biologique.

**11.** Dispositif selon la revendication 10, dans lequel l'au moins un paramètre métabolique est choisi dans la liste comprenant : la température de l'objet biologique, l'autofluorescence de l'objet biologique, le rapport redox, la saturation de l'hémoglobine, la teneur en dérivés de l'hémoglobine, le pH et/ou les niveaux de bicarbonate, la concentration en espèces réactives de l'oxygène, le niveau de sulfure d'hydrogène, le niveau de séléniure d'hydrogène, la concentration ionique, le niveau de cytochromes, le tonus vasculaire de l'objet biologique, la vasomotion, les mesures de bioimpédance électrique, le niveau de marqueurs, le niveau de glucose, le niveau de succinate, le niveau de lactate et de lactate déshydrogénase, le niveau de thiorédoxine, le stress oxydatif et/ou le niveau d'ions chlorure.

12. Dispositif selon l'une quelconque des revendications 8 à 11, dans lequel l'unité de traitement et/ou de contrôle de la lumière est conçue pour prédire le moment optimal pour commencer l'application d'une PBM sur la base de l'au moins un paramètre métabolique mesuré par l'unité de surveillance métabolique.

13. Dispositif selon l'une quelconque des revendications précédentes, **caractérisé par le fait que** l'unité de traitement et/ou de contrôle de la lumière est conçue pour générer de la lumière combinée ou séquentielle comprenant au moins une longueur d'onde active et au moins une longueur d'onde inefficace lorsqu'elle est utilisée seule.

14. Dispositif selon la revendication 13, dans lequel la longueur d'onde active est de 689 nm ou 808 nm et la longueur d'onde inefficace est de 730 nm.

15. Dispositif selon l'une quelconque des revendications précédentes, dans lequel la lumière est délivrée en séquence pour traiter simultanément différentes profondeurs de l'objet biologique et à différentes distances d'une surface de l'objet biologique.

16. Dispositif selon l'une quelconque des revendications précédentes, dans lequel la source de lumière est adaptée pour être insérée dans un compartiment cardiaque, l'artère pulmonaire ou la veine cave, de sorte que la lumière est directement délivrée dans le sang.

(Model and Monte-Carlo simulation : $\mu_a = 0.27$ mm$^{-1}$, $\mu_s = 18.7$ mm$^{-1}$, $g = 0.81$, $k = 4.87$, $\mu_{eff}^{-1} = 0.57$ mm) (S. L. JACQUES)

$$\frac{F(z)}{E} = e^{-\mu_{eff} z}$$

Fig.1

Fig. 2

**Fig. 3**

Branching points

Fluorescence angiography image

Processed image

| Angiogenesis descriptors | Control - no PBM | PBM | STS | PBM + STS | More developing angiogenesis corresponds to: |
|---|---|---|---|---|---|
| Branching points/mm$^2$ | 2400±200 | 3400±400 | 2700±200 | 4100±300 | a high value |
| The mean area of the vessel network meshes ($10^2$mm$^2$) | 6.7±1.5 | 2.9±0.6 | 3.8±0.4 | 4±0.5 | a low value |
| Mean of the 3$^{rd}$ quartile of mesh area histogram ($10^2$mm$^2$) | 5±1 | 3.1±0.5 | 1.8±0.4 | 2.1±0.3 | a low value |

Angiogenesis descriptors showing the effects of PBM performed at egg development day (EDD) 6 and observed at EDD 12. PBM was performed at 670 nm with 15 mW/cm$^2$ during 180 s. Note: the three descriptors are stongly correlated.

Fig. 4a

**Fig 4b**

**Fig 4c**

**Fig4d**

$$\mu_a = 0.27 mm^{-1}, \mu_s = 18.7 mm^{-1}, \mu_{\text{eff}} = 1.76 mm^{-1}$$
$$g = 0.81, k = 4.87$$

F(z)/E

$$\frac{F(z)}{E} = k e^{-\mu_{eff} z}$$

M.-C.

Depth (mm)

**Fig. 5**

EP 4 221 828 B1

Fig. 6

44

EP 4 221 828 B1

Fig. 7

45

Fig. 8

Fig. 9

$$F' = 3mW.cm^{-2} \quad k = 4{,}87 \quad \mu_{eff} = 1{,}76 \, mm^{-1}$$

$$E(z) = \frac{F'}{k} \times e^{(\mu_{eff} \times z)}$$

**Fig. 10**

$$F' = 3mW.cm^{-2} \Delta F' = 1{,}6mW.cm^{-2} \, k = 4{,}87 \quad \mu_{eff} = 1{,}76 \, mm^{-1}$$

$$T = 180s$$

$$E(t) = \frac{F'}{k} \times e^{\left(\frac{\Delta F'}{F'} \times \frac{t}{T}\right)}$$

**Fig. 11**

**Fig.12**

**Fig. 13**

Fig. 14a

Fig14b

Fig. 15

Fig. 16

Fig. 17

Fig. 18

Fig 19a

Fig 19b

Fig19c

Fig20

Fig21

**Fig22**

Fig23

Fig24a

Fig24b

Fig 25a

Fig25b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007219604 A1

**Non-patent literature cited in the description**

- **AALKJÆR, C.** ; **BOEDTKJER, D.** ; **MATCHKOV, V.** Vasomotion - what is currently thought?. *Acta Physiologica*, 2011, vol. 202 (3), 253-269
- **ACHARYA, U.R. et al.** Application of higher-order spectra for the characterization of Coronary artery disease using electrocardiogram signals. *Biomedical Signal Processing and Control*, 2017, vol. 31, 31-43, http://dx.doi.org/10.1016/j.bspc.2016.07.003
- **B. ALBERTS** ; **A. JOHNSON** ; **J. LEWIS** ; **M. RAFF** ; **K. ROBERTS** ; **P. WALTER**. Molecular Biology of the Cell. Garland Science, 2002
- **ÁLVAREZ, R.C. et al.** Cardiac Hemodynamic Monitoring in the Subcutaneous Space: A pre-clinical proof-of-concept. *MeMeA 2018 - 2018 IEEE International Symposium on Medical Measurements and Applications, Proceedings*, 2018
- **F. ANTUNES** ; **A. BOVERIS** ; **E. CADENAS**. On the mechanism and biology of cytochrome oxidase inhibition by nitric oxide. *Proc. Natl. Acad. Sci. U. S. A.*, November 2004, vol. 101 (48), 16774-16779
- **BAILEY, S.M.** ; **UDOH, U.S.** ; **YOUNG, M.E.** Circadian regulation of metabolism. *Journal of Endocrinology*, 2014, vol. 222 (2)
- **K. A. BALL** ; **P. R. CASTELLO** ; **R. O. POYTON**. Low intensity light stimulates nitrite-dependent nitric oxide synthesis but not oxygen consumption by cytochrome c oxidase: Implications for phototherapy. *J. Photochem. Photobiol. B*, March 2011, vol. 102 (3), 182-191
- **S. BANERJEE** ; **D. K. BHATT**. Histochemical studies on the distribution of certain dehydrogenases in squamous cell carcinoma of cheek. *Indian J. Cancer*, March 1989, vol. 26 (1), 21-30
- **M. A. BEILKE** ; **C. COLLINS-LECH** ; **P. G. SOHNLE**. Effects of dimethyl sulfoxide on the oxidative function of human neutrophils. *J. Lab. Clin. Med.*, July 1987, vol. 110 (1), 91-96
- **I. BENTOV** ; **M. J. REED**. The effect of aging on the cutaneous microvasculature. *Microvasc. Res.*, 2015, vol. 100, 25-31
- **BHATTACHARYYA, A. et al.** A novel approach for automated detection of focal EEG signals using empirical wavelet transform. *Neural Computing and Applications*, 2018, vol. 29 (8), 47-57
- **BHOI, A.K.** ; **SHERPA, K.S.** ; **KHANDELWAL, B.** Arrhythmia and ischemia classification and clustering using QRS-ST-T (QT) analysis of electrocardiogram. *Cluster Computing*, 2017, vol. 21 (1), 1033-1044
- **BIGARELLA, C.L.** ; **LIANG, R.** ; **GHAFFARI, S.** Stem cells and the impact of ROS signaling. *Development (Cambridge)*, 2014, vol. 141 (22), 4206-4218
- **T. S. BLACKER** ; **M. R. DUCHEN**. Investigating mitochondrial redox state using NADH and NADPH autofluorescence. *Free Radic. Biol. Med.*, November 2016, vol. 100 (C), 53-65
- **BLACKSTONE, E.** ; **MORRISON, M.** ; **ROTH, M.B.** H2S induces a suspended animation-like state in mice. *Science*, 2005, vol. 308 (5721), 518
- **BLEYS, J.** ; **NAVAS-ACIEN, A.** ; **GUALLAR, E.** Serum selenium levels and all-cause, cancer, and cardiovascular mortality among US adults. *Archives of Internal Medicine*, 2008, vol. 168 (4), 404-410
- **BRANDES, R.P.** ; **WEISSMANN, N.** ; **SCHRÖDER, K.** Nox family NADPH oxidases in mechano-transduction: Mechanisms and consequences. *Antioxidants and Redox Signaling*, 2014, vol. 20 (6), 887-898
- **M. BUTAWAN** ; **R. L. BENJAMIN** ; **R. J. BLOOMER**. Methylsulfonylmethane: Applications and Safety of a Novel Dietary Supplement. *Nutrients*, March 2017, vol. 9 (3)
- **BOS, E.M. et al.** Hydrogen sulfide-induced hypometabolism prevents renal ischemia/reperfusion injury. *Journal of the American Society of Nephrology*, 2009, vol. 20 (9), 1901-1905
- **CARLSTRÖM, M.** ; **WILCOX, C.S.** ; **ARENDSHORST, W.J.** Renal autoregulation in health and disease. *Physiological Reviews*, 2015, vol. 95 (2), 405-511

- **B. CHANCE** ; **B. SCHOENER** ; **R. OSHINO** ; **F. ITSHAK** ; **Y. NAKASE**. Oxidation-reduction ratio studies of mitochondria in freeze-trapped samples. NADH and flavoprotein fluorescence signals. *J. Biol. Chem.*, June 1979, vol. 254 (11), 4764-4771

- **B. CHANCE**. Metabolic Heterogeneities in Rapidly Metabolizing Tissues. *J. Appl. Cardiol.*, 1989, vol. 4 (4), 207-221

- **CHOUCHANI E. T.** ; **VICTORIA R. PELL** ; **ANDREW M. JAMES** ; **LORRAINE M. WORK** ; **KOUROSH SAEB-PARSY** ; **CHRISTIAN FREZZA** ; **THOMAS KRIEG** ; **MICHAEL P. MURPHY**. A Unifying Mechanism for Mitochondrial Superoxide Production during Ischemia-Reperfusion Injury. *Cell Metabolism*, 09 February 2016, vol. 23

- **CHOUCHANI, E.T. et al.** Ischaemic accumulation of succinate controls reperfusion injury through mitochondrial ROS. *Nature*, 2014, vol. 515 (7527), 431-435

- **CHUNG H** ; **DAI T** ; **SHARMA S** ; **HUANG Y** ; **CARROLL J** ; **HAMBLIN M**. The Nuts and Bolts of Low-level Laser (Light) Therapy. *Ann Biomed Eng.*, 2012, vol. 40 (2), 516-533

- **C. E. COOPER** ; **G. C. BROWN**. The inhibition of mitochondrial cytochrome oxidase by the gases carbon monoxide, nitric oxide, hydrogen cyanide and hydrogen sulfide: chemical mechanism and physiological significance. *J. Bioenerg. Biomembr.*, October 2008, vol. 40 (5), 533-539

- **DASGUPTA, A.** Cardiac Markers. Clinical Chemistry. *Immunology and Laboratory Quality Control*, 2014, 127-144, https://doi.org/10.1016/B978-0-12-407821-5.00008-5

- **DEVASAGAYAM TP** ; **TILAK JC** ; **BOLOOR KK** ; **SANE KS** ; **GHASKADBI SS** ; **LELE RD**. Free radicals and antioxidants in human health: current status and future prospects. *The Journal of the Association of Physicians of India*, 2004, vol. 52, 794-804

- **DI GIALLEONARDO V.** ; **DAVID M. WILSON** ; **KAYVAN R. KESHARI**. The Potential of Metabolic Imaging. *Semin. Nucl. Med.*, 2016, vol. 46 (1), 28-39

- **DONALD JA**. Handbook of Hormones. Elsevier, 2016, 601

- **DUAN, J. et al.** Metabolic remodeling induced by mitokines in heart failure. *Aging (Albany NY)*, 15 September 2019, vol. 11 (17), 7307-7327

- **FERRARESI C.** ; **M. R. HAMBLIN** ; **N. A. PARIZOTTO**. Low-level laser (light) therapy (LLLT) on muscle tissue: performance, fatigue and repair benefited by the power of light. *Photonics Lasers Med.*, November 2012, vol. 1 (4), 267-286

- **FERRARI R.** ; **CRISTINA BALLA** ; **MICHELE MALAGÙ** ; **GABRIELE GUARDIGLI** ; **GIAMPAOLO MORCIANO** ; **MATTEO BERTINI** ; **SIMONE BISCAGLIA** ; **GIANLUCA CAMPO**. Reperfusion Damage - A Story of Success, Failure, and Hope. *Circ J*, 2017, vol. 81, 131-141

- **FU M** ; **ZHANG W** ; **WU L** ; **YANG G** ; **LI H** ; **WANG R**. Hydrogen sulfide (H2S) metabolism in mitochondria and its regulatory role in energy production. *Proc Natl Acad Sci U S A.*, 2012, vol. 109 (8), 2943-8

- **FUSS T. L.** ; **B.A.** ; **LEO L. CHENG**. Metabolic Imaging in Humans. *Top Magn Reson Imaging*, 2016, vol. 25 (5), 223-235

- **R. A. GATENBY** ; **R. J. GILLIES**. Why do cancers have high aerobic glycolysis?. *Nat. Rev. Cancer*, November 2004, vol. 4 (11), 891-899

- **GRIMOLIZZI, F.** ; **ARRANZ, L.** Multiple faces of succinate beyond metabolism in blood. *Haematologica*, 2018, vol. 103 (10), 1586-1592

- **GRIENDLING, K.K. et al.** Measurement of Reactive Oxygen Species, Reactive Nitrogen Species, and Redox-Dependent Signaling in the Cardiovascular System: A Scientific Statement from the American Heart Association. *Circulation journal*, 2016, vol. 119 (5), 39-65

- **HAMBLIN M.** ; **M. VICTOR PIRES DE SOUSA** ; **T. AGRAWAL**. Handbook of Low-Level Laser Therapy. Pan Stanford Publishing Pte. Ltd., 2017

- **HAMBLIN M.** ; **C. FERRARESI** ; **Y.-Y. HUANG** ; **L. F. DE FREITAS** ; **J. CARROLL**. Low-level light therapy: photobiomodulation. 2018

- **HAYYAN M** ; **HASHIM MA** ; **ALNASHEF IM**. Superoxide Ion: Generation and Chemical Implications. *Chemical Reviews*, March 2016, vol. 116 (5), 3029-85

- **HELLMANN, M.** ; **ROUSTIT, M.** ; **CRACOWSKI, J.L.** Skin microvascular endothelial function as a biomarker in cardiovascular diseases?. *Pharmacological Reports*, 2015, vol. 67 (4), 803-810

- **HUANG, YING-YING** ; **CARROLL JD, H.M.** Biphasic dose response. *Dose response*, 2009, 358-383, http:// dx.doi.org/10.2203/dose-response.09-027. Hamblin

- **HWANG, J. et al.** Pulsatile Versus Oscillatory Shear Stress Regulates NADPH Oxidase Subunit Expression: Implication for Native LDL Oxidation. *Circulation Research*, 2003, vol. 93 (12), 1225-1232

- **IWATA, A. et al.** Selenide Targets to Reperfusing Tissue and Protects It From Injury*. *Read Online: Critical Care Medicine | Society of Critical Care Medicine*, 2015, vol. 43 (7)

- **JACQUES S**. How tissue optics affect dosimetry of photodynamic therapy. *Journal of Biomedical Optics*, 2010, vol. 15 (5), 051608

- **JEKELL, A. et al.** Elevated circulating levels of thioredoxin and stress in chronic heart failure. *The european Journal of Heart failure*, 2004, vol. 6, 883-890

- **JACKSON, W.F.** Arteriolar oxygen reactivity: where is the sensor and what is the mechanism of action?. *Journal of Physiology*, 2016, vol. 594 (18), 5055-5077
- **S. KALININA et al.** Correlative NAD(P)H-FLIM and oxygen sensing-PLIM for metabolic mapping. *J. Biophotonics*, August 2016, vol. 9 (8), 800-811
- **KALOGERIS T.** ; **CHRISTOPHER P. BAINES** ; **MAIKE KRENZ** ; **RONALD J. KORTHUIS**. Ischemia/Reperfusion. *Compr Physiol.*, 2017, vol. 7 (1), 113-170
- **T. I. KARU** ; **L. V. PYATIBRAT** ; **N. I. AFANASYEVA**. Cellular effects of low power laser therapy can be mediated by nitric oxide. *Lasers Surg. Med.*, April 2005, vol. 36 (4), 307-314
- **KENNEDY R. T. et al.** Metabolic oscillations in □-cells. *Diabetes*, 2002, vol. 51 (S1)
- **Y. H. KIM** ; **D. H. KIM** ; **H. LIM** ; **D.-Y. BAEK** ; **H.-K. SHIN** ; **J.-K. KIM**. The anti-inflammatory effects of methylsulfonylmethane on lipopolysaccharide-induced inflammatory responses in murine macrophages. *Biol. Pharm. Bull.*, April 2009, vol. 32 (4), 651-656
- **KINDO, M. et al.** Left Ventricular Transmural Gradient in Mitochondrial Respiration Is Associated with Increased Sub-Endocardium Nitric Oxide and Reactive Oxygen Species Productions. *Frontiers in physiology*, August 2016, vol. 7, 1-8
- **KINDO, M. et al.** Pressure overload-induced mild cardiac hypertrophy reduces left ventricular transmural differences in mitochondrial respiratory chain activity and increases oxidative stress. *Frontier in physiology*, August 2012, vol. 3, 1-14
- **KONDO K** ; **BHUSHAN S** ; **KING AL** ; **PRABHU SD** ; **HAMID T** ; **KOENIG S et al.** H2S protects against pressure overload-induced heart failure via upregulation of endothelial nitric oxide synthase. *Circulation*, 2013, vol. 127, 1116-1127
- **KÖHN, C. et al.** Hydrogen sulfide: Potent regulator of vascular tone and stimulator of angiogenesis. *International Journal of Biomedical Science*, 2012, vol. 8 (2), 81-86
- **KUGANESAN, M. et al.** Selenium and hydrogen selenide: essential micronutrient and the fourth gasotransmitter?. *Intensive Care Medicine Experimental*, 2019, vol. 7 (1), http://dx.doi.org/10.1186/s40635-019-0281-y
- **KUMAR, M.** ; **PACHORI, R.B.** ; **ACHARYA, U.R.** Characterization of coronary artery disease using flexible analytic wavelet transform applied on ECG signals. *Biomedical Signal Processing and Control*, 2017, vol. 31, 301-308
- **KVANDAL, P. et al.** Low-frequency oscillations of the laser Doppler perfusion signal in human skin. *Microvascular Research*, 2006, vol. 72 (3), 120-127
- **N. LANE**. Cell biology: power games. *Nature*, October 2006, vol. 443 (7114), 901-903
- **LATHAM, F.** THE OXYGEN PARADOX EXPERIMENTS ON THE EFFECTS OF OXYGEN IN HUMAN ANOXIA. *The Lancet*, 1951, vol. 257 (6646), 77-81
- **LI, S.** ; **YANG, G.** Hydrogen Sulfide Maintains Mitochondrial DNA Replication via Demethylation of TFAM. *Antioxidants and Redox Signaling*, 2015, vol. 23 (7), 630-642
- **LI, Y.S.J.** ; **HAGA, J.H.** ; **CHIEN, S.** Molecular basis of the effects of shear stress on vascular endothelial cells. *Journal of Biomechanics*, 2005, vol. 38 (10), 1949-1971
- **LI, Y** ; **PATRICK J. PAGANO**. Microvascular NADPH oxidase in health and disease. *Free Radic Biol Med.*, 2017, vol. 109 (1), 33-47
- **LIEBERT, A. et al.** A Role for Photobiomodulation in the Prevention of Myocardial Ischemic Reperfusion Injury: A Systematic Review and Potential Molecular Mechanisms. *Scientific Reports*, February 2017, vol. 7, 1-13, http://dx.doi.org/10.1038/srep42386
- **N. L. LOHR** ; **A. KESZLER** ; **P. PRATT** ; **M. BIENENGRABER** ; **D. C. WARLTIER** ; **N. HOGG**. Enhancement of nitric oxide release from nitrosyl hemoglobin and nitrosyl myoglobin by red/near infrared radiation: potential role in cardioprotection. *J. Mol. Cell. Cardiol.*, August 2009, vol. 47 (2), 256-263
- **LOUTZENHISER, R.** ; **BIDANI, A.** ; **CHILTON, L.** Renal myogenic response: Kinetic attributes and physiological role. *Circulation Research*, 2002, vol. 90 (12), 1316-1324
- **MARTELLI F.** ; **DEL BIANCO S.** ; **ISMAELLI A.** Light propagation through biological tissue and other diffusive media: theory, solutions, and software. *Bellingham: Society of Photo-Optical Instrumentation Engineers*, 2009
- **MOLDOGAZIEVA, N.T. et al.** ROS and RNS signalling: adaptive redox switches through oxidative/nitrosative protein modifications. *Free Radical Research*, 2018, vol. 52 (5), 507-543, https://doi.org/10.1080/10715762.2018.1457217
- **MOSS, N.G.** ; **GENTLE, T.K.** ; **ARENDSHORST, W.J.** Modulation of the myogenic mechanism: Concordant effects of NO synthesis inhibition and O-2 dismutation on renal autoregulation in the time and frequency domains. *American Journal of Physiology - Renal Physiology*, 2016, vol. 310 (9), F832-F845
- **NACHIAPPAN, VASANTHI** ; **MUTHUKUMAR, KANNAN**. Cadmium-induced oxidative stress in Saccharomyces cerevisiae. *Indian Journal of Biochemistry and Biophysics*, 2010, vol. 47 (6)
- **NAGPURE, B. V.** ; **BIAN, J.S.** Interaction of Hydrogen Sulfide with Nitric Oxide in the Cardiovascular System. *Oxidative Medicine and Cellular Longevity*, 2016, vol. 2016
- **NAKAMURA, H.** Thioredoxin as a Key Molecule in Redox Signaling. *Antioxidants and Redox Signaling*, 2004, vol. 6 (1), 15-17

- **NOWAK-SLIWINSKA P.** ; **J.-P. BALLINI** ; **G. WAGNIÈRES** ; **H. VAN DEN BERGH**. Processing of fluorescence angiograms for the quantification of vascular effects induced by anti-angiogenic agents in the CAM model. *Microvasc. Res.*, January 2010, vol. 79 (1), 21-28

- **P.T. NOWICKI** ; **S. FLAVAHAN** ; **H. HASSANAIN** ; **S. MITRA** ; **S. HOLLAND** ; **P.J. GOLDSCHMIDT-CLERMONT, N.A.F.** Redox Signaling of the Arteriolar Myogenic Response. *Circulation research*, 2001, vol. 89, 114-116

- **OLSON, K.R.** A practical look at the chemistry and biology of hydrogen sulfide. *Antioxidants and Redox Signaling*, 2012, vol. 17 (1), 32-44

- **PAPAGIANNAKIS A** ; **NIEBEL B** ; **WIT E** ; **HEINEMANN M**. Autonomous Metabolic Oscillations Robustly Gate the Early and Late Cell Cycle. *Molecular Cell*, 19 January 2017, vol. 65 (2), 285-295, https://doi.org/10.1016/j.molcel.2016.11.018

- **PARK, H.C. et al.** Effect of LED phototherapy on blood lactate level in Taekwondo contest. *Mechanisms of Photobiomodulation Therapy*, February 2017, vol. XII (10048), 100480I

- **PATIDAR, S.** ; **PACHORI, R.B.** Classification of cardiac sound signals using constrained tunable-Q wavelet transform. *Expert Systems with Applications*, 2014, vol. 41 (16), 7161-7170, http://dx.doi.org/10.1016/j.eswa.2014.05.052

- **PATIDAR, S.** ; **PACHORI, R.B.** ; **RAJENDRA ACHARYA, U.** Automated diagnosis of coronary artery disease using tunable-Q wavelet transform applied on heart rate signals. *Knowledge-Based Systems*, 2015, vol. 82, 1-10, http://dx.doi.org/10.1016/j.knosys.2015.02.011

- **PETTERSEN, F.J. et al.** Bioimpedance measurements of temporal changes in beating hearts. *Biomedical Physics and Engineering Express*, 2016, vol. 2 (6)

- **PETRENKO, V. et al.** In pancreatic islets from type 2 diabetes patients, the dampened circadian oscillators lead to reduced insulin and glucagon exocytosis. *Proceedings of the National Academy of Sciences of the United States of America*, 2020, vol. 117 (5), 2484-2495

- **PIPER, H.M.** The calcium paradox revisited: An artefact of great heuristic value. *Cardiovascular Research*, 2000, vol. 45 (1), 123-127

- **N. RAMANUJAM** ; **R. RICHARDS-KORTUM** ; **S. THOMSEN** ; **A. MAHADEVAN-JANSEN** ; **M. FOLLEN** ; **B. CHANCE**. Low Temperature Fluorescence Imaging of Freeze-trapped Human Cervical Tissues. *Opt. Express*, March 2001, vol. 8 (6), 335-343

- **V. RAPOZZI** ; **E. DELLA PIETRA** ; **S. ZORZET** ; **M. ZACCHIGNA** ; **B. BONAVIDA** ; **L. E. XODO**. Nitric oxide-mediated activity in anti-cancer photodynamic therapy. *Nitric Oxide Biol. Chem.*, April 2013, vol. 30, 26-35

- **RADDATZ E** ; **THOMAS AC**. Differential contribution of mitochondria, NADPH oxidases, and glycolysis to region-specific oxidant stress in the anoxic-reoxygenated embryonic heart. *Am J Physiol Heart Circ Physiol*, 2011, vol. 300, H820-H835

- **K. J. REEVES** ; **M. W. R. REED** ; **N. J. BROWN**. Is nitric oxide important in photodynamic therapy?. *J. Photochem. Photobiol. B*, June 2009, vol. 95 (3), 141-147

- **REGLIN, B.** ; **PRIES, A.R.** Metabolic control of microvascular networks: Oxygen sensing and beyond. *Journal of Vascular Research*, 2014, vol. 51 (5), 376-392

- **DE ROEVER I** ; **BALE G** ; **COOPER RJ** ; **TACHTSIDIS I**. Functional NIRS Measurement of Cytochrome-C-Oxidase Demonstrates a More Brain-Specific Marker of Frontal Lobe Activation Compared to the Haemoglobins. *Adv Exp Med Biol.*, 2017, vol. 977, 141-147

- **SACHAR M. et al.** Protoporphyrin IX: the Good, the Bad, and the Ugly. *The Journal of pharmacology and experimental therapeutics*, 2016, vol. 356 (2), 267-75

- **SEDMERA, D.** ; **KUCERA, P.** ; **RADDATZ, E.** Developmental changes in cardiac recovery from anoxia-reoxygenation. *American Journal of Physiology - Regulatory Integrative and Comparative Physiology*, 2002, vol. 283 (2 52-2), 379-388

- **SHIOGAI, Y.** ; **STEFANOVSKA, A.** ; **MCCLINTOCK, P.V.E.** Nonlinear dynamics of cardiovascular ageing. *Physics Reports*, 2010, vol. 488 (2-3), 51-110

- **SIES, H.** ; **JONES, D.P.** Reactive oxygen species (ROS) as pleiotropic physiological signalling agents. *Nature Reviews Molecular Cell Biology*, 2020, http://dx.doi.org/10.1038/s41580-020-0230-3

- **M. C. SKALA et al.** In vivo multiphoton microscopy of NADH and FAD redox states, fluorescence lifetimes, and cellular morphology in precancerous epithelia. *Proc. Natl. Acad. Sci.*, December 2007, vol. 104 (49), 19494-19499

- **M. SKALA** ; **N. RAMANUJAM**. Multiphoton Redox Ratio Imaging for Metabolic Monitoring in vivo. *Methods Mol. Biol. Clifton NJ*, 2010, vol. 594, 155-162

- **SMIRNOVA, E. et al.** Assessment of endothelial dysfunction in patients with impaired glucose tolerance during a cold pressor test. *Diabetes and Vascular Disease Research*, 2013, vol. 10 (6), 489-497

- **SNIJDER P. M.** ; **A R FRENAY** ; **R A DE BOER** ; **A PASCH** ; **J L HILLEBRANDS** ; **H G D LEUVENINK** ; **H VAN GOOR**. Exogenous administration of thiosulfate, a donor of hydrogen sulfide, attenuates angiotensin II-induced hypertensive heart disease in rats. *Br J Pharmacol.*, March 2015, vol. 172 (6), 1494-1504

- **STEFANOVSKA, A.** Coupled oscillators: Complex but not complicated cardiovascular and brain interactions. *Annual International Conference of the IEEE Engineering in Medicine and Biology - Proceedings*, 2006, 437-440
- **SZABO, C. et al.** Regulation of mitochondrial bioenergetic function by hydrogen sulfide. *Part I. Biochemical and physiological mechanisms. British Journal of Pharmacology*, 2014, vol. 171 (8), 2099-2122
- **TAMURA, T.** Current progress of photoplethysmography and SPO2 for health monitoring. *Biomedical Engineering Letters*, 2019, vol. 9 (1), 21-36, https://doi.org/10.1007/s13534-019-00097-w
- **TAMURA, T.** *Seamless Healthcare Monitoring*, 2018, https://doi.org/10.1007/978-3-319-69362-0
- **THOMAS DD**. Breathing new life into nitric oxide signaling: A brief overview of the interplay between oxygen and nitric oxide. *Redox Biol.*, 2015, vol. 5, 225-233
- **TICCINELLI, V. et al.** Coherence and coupling functions reveal microvascular impairment in treated hypertension. *Frontiers in Physiology*, October 2017, vol. 8, 1-20
- **TRETTER, L.** ; **PATOCS, A.** ; **CHINOPOULOS, C.** Succinate, an intermediate in metabolism, signal transduction, ROS, hypoxia, and tumorigenesis. *Biochimica et Biophysica Acta - Bioenergetics*, 2016, vol. 1857 (8), 1086-1101, http://dx.doi.org/10.1016/j.bbabio.2016.03.012
- **TSURUOKA, N. et al.** Lactate and glucose measurement in subepidermal tissue using minimally invasive microperfusion needle. *Biomedical microdevices*, 2016, vol. 18:19, 2-9, http://dx.doi.org/10.1007/s10544-016-0049-z
- **TUCHIN V.** Tissue Optics and Photonics: Light-Tissue Interaction. *J of Biomedical Photonics & Eng*, 2015, vol. 1 (2), 98-134
- Three-wavelength method for the optical differentiation of methemoglobin and sulfhemoglobin in oxygenated blood. **VAN LEEUWEN, S.R.** ; **BARANOSKI, G.V.G.** ; **KIMMEL, B.W.** Proceedings of the Annual International Conference of the IEEE Engineering in Medicine and Biology Society. EMBS, 2017, 4570-4573
- Ion Metabolism and Transport. **ARNOUD H. M. VAN VLIET** ; **STEFAN BERESWILL** ; **JOHANNES G. KUSTERS**. Helicobacter pylori: Physiology and Genetics. ASM Press, 2001
- **WAGNIÈRES G. et al.** In Vivo Fluorescence Spectroscopy and Imaging for Oncological Applications. *Photochemistry and Photobiology*, 1998, vol. 68 (5), 603-632
- **A. WALSH** ; **R. S. COOK** ; **B. REXER** ; **C. L. ARTEAGA** ; **M. C. SKALA**. Optical imaging of metabolism in HER2 overexpressing breast cancer cells. *Biomed. Opt. Express*, January 2012, vol. 3 (1), 75-85
- **A. J. WALSH et al.** Optical metabolic imaging identifies glycolytic levels, subtypes, and early-treatment response in breast cancer. *Cancer Res.*, October 2013, vol. 73 (20), 6164-6174
- **G. WALTHER et al.** Metabolic syndrome individuals with and without type 2 diabetes mellitus present generalized vascular dysfunction: Cross-sectional study. *Arterioscler. Thromb. Vasc. Biol.*, 2015, vol. 35 (4), 1022-1029
- **WILSON B.** ; **PATTERSON M.** The physics of photodynamic therapy. *Phys. Med. Biol.*, 1986, vol. 4 (31), 327-360
- **WOOD K** ; **CORTESE-KROTT M**. Circulating Blood Endothelial Nitric Oxide Synthase Contributes to the Regulation of Systemic Blood Pressure and Nitrite Homeostasis. *Arterioscler Thromb Vasc Biol.*, 2013, vol. 33, 1861-1871
- **WU, M.** ; **NEILSON, A.** ; **SWIFT, A.L.** ; **MORAN, R.** ; **TAMAGNINE, J.** ; **PARSLOW, D.** ; **ARMISTEAD S.** ; **LEMIRE, K.** ; **ORRELL, J.** ; **TEICH, J.** Multiparameter metabolic analysis reveals a close link between attenuated mitochondrial bioenergetic function and enhanced glycolysis dependency in human tumor cells. *Am J Physiol Cell Physiol.*, 2007, vol. 292, C125-C136
- **WU, D.** ; **HU, Q.** ; **ZHU, D.** An update on hydrogen sulfide and nitric oxide interactions in the cardiovascular system. *Oxidative Medicine and Cellular Longevity*, 2018, vol. 2018
- **YANG, G.** H2S epigenetic regulation of vascular cell functions. *Cardiovascular Regenerative Medicine*, 2015, 2-5
- **YU XH** ; **CUI LB** ; **WU K et al.** Hydrogen sulfide as a potent cardiovascular protective agent. *Clin Chim Acta.*, 2014, vol. 437, 78-87
- **ZHONG, X. et al.** Circadian Clock Regulation of Hepatic Lipid Metabolism by Modulation of m6A mRNA Methylation. *Cell Reports*, 2018, vol. 25 (7), 1816-1828.e4
- **ZHOU, G. et al.** Optimal ROS Signaling Is Critical for Nuclear Reprogramming. *Cell Reports*, 2016, vol. 15 (5), 919-925, http://dx.doi.org/10.1016/j.celrep.2016.03.084